(19) Europäisches Patentamt
European Patent Office
Office européen des brevets

(11) EP 1 399 436 B1

(12) **EUROPEAN PATENT SPECIFICATION**

(45) Date of publication and mention of the grant of the patent:
**16.03.2005 Bulletin 2005/11**

(21) Application number: **02732974.7**

(22) Date of filing: **24.06.2002**

(51) Int Cl.[7]: **C07D 403/12**

(86) International application number:
**PCT/GB2002/002872**

(87) International publication number:
**WO 2003/000692 (03.01.2003 Gazette 2003/01)**

# (54) OXYTOCIN AGONISTS

OXYTOCIN AGONISTEN

AGONISTES DE L'OCYTOCINE

(84) Designated Contracting States:
**AT BE CH CY DE DK ES FI FR GB GR IE IT LI LU MC NL PT SE TR**
Designated Extension States:
**LT LV**

(30) Priority: **25.06.2001 GB 0115515**

(43) Date of publication of application:
**24.03.2004 Bulletin 2004/13**

(73) Proprietor: **Ferring BV**
**2132 JX Hoofddorp (NL)**

(72) Inventors:
- **PITT, Gary, Robert, William**
  **Tidworth, Hampshire SP9 7US (GB)**
- **ROE, Michael, Bryan Ferring Research Limited**
  **Southampton SO16 7NP (GB)**
- **ROOKER, David, Philip**
  **Ferring Research Limited**
  **Southampton SO16 7NP (GB)**

(74) Representative: **Poulsen, Niels Jakob**
**Ferring Pharmaceuticals A/S,**
**Kay Fiskers Plads 11**
**2300 Copenhagen S (DK)**

(56) References cited:
**EP-A- 0 469 984**
**WO-A-00/46224**
**WO-A-01/49682**
**WO-A-02/00626**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a series of non-peptide oxytocin agonists and to pharmaceutical compositions comprising such compounds. The compositions are useful for the treatment of certain physiological disorders, such as erectile dysfunction.

## BACKGROUND

*Neurophyseal hormones*

The neurophyseal hormones oxytocin (OT) and vasopressin (VP) are cyclic nonapeptides secreted by the posterior pituitary gland. The structure of oxytocin is shown below.

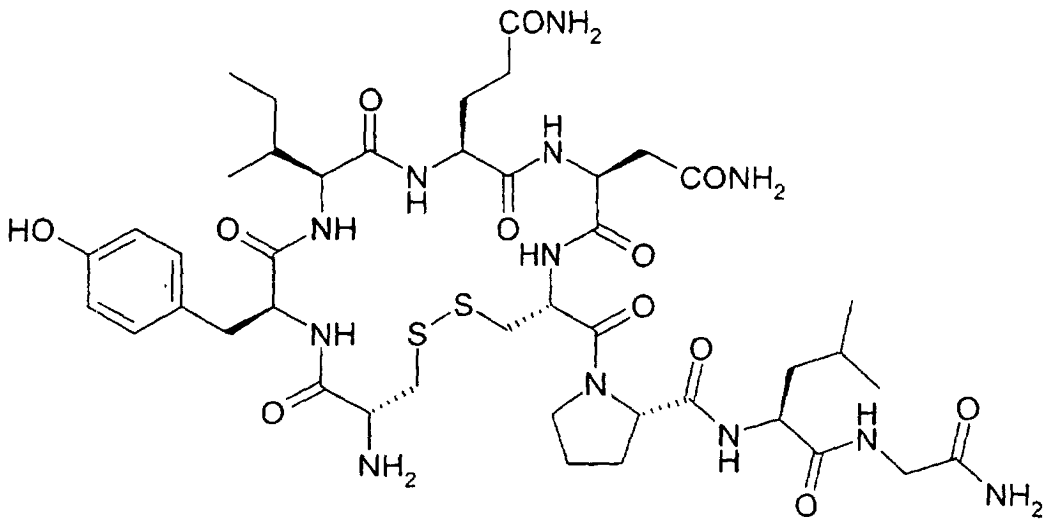

Oxytocin – *cyclo*$^{1,6}$-Cys-Tyr-Ile-Gln-Asn-Cys-Pro-Leu-Gly-$NH_2$

Vasopressin differs from oxytocin in that it has phenylalanine at position 3 in place of isoleucine and arginine at position 8 in place of leucine. Both hormones are synthesised *in vivo* as larger precursors, neurophysins, which are subject to post-translational processing to release the mature peptides. OT and VP act through a family of heptahelical receptors. Only one OT receptor has so far been well characterised, while three VP receptors are known. These are designated the $V_{1a}$, $V_{1b}$ and $V_2$ receptors.

The first target organs to be identified for OT were the uterus, where it is implicated in the onset and progress of labour, and mammary glands, where it is involved in the regulation of milk expression. Other organs also express OT receptors, and it is clear that OT has a range of physiological roles that have not been fully elaborated yet. In particular, it has been suggested that OT acting in the CNS is involved in the erectile response in males, and in the regulation of female sexual arousal. For example, OT is erectogenic when administered *i.c.v.* to male rats. It also has erectogenic activity when given *i.v.,* but the doses required are up to two orders of magnitude greater, which is consistent with a central mode of action.

Vasopressin acts on the blood vessels, where it is a potent vasoconstrictor, and on the kidneys, where it promotes water reuptake leading to an antidiuretic effect.

*Oxytocin agonists and antagonists*

A number of peptide analogues of OT are known in the literature. These include both agonists and antagonists. OT and its agonists are used, for example, to accelerate labour and to increase uterine muscle tone to control post-partum bleeding, and one antagonist, atosiban, has recently been registered as a treatment for pre-term labour. However, the peptidic nature of these compounds means that they are not likely to be bioavailable after oral dosing or to cross into the CNS. In order to get drugs that can be given orally and to be able to exploit the central effects of OT, attention has increasingly turned to non-peptides. As a result, there are many publications describing non-peptide OT antagonists in early-stage development. So far, however, there have been no reports of non-peptide OT agonists. This

is not unexpected, as it is generally held that it is easier to find a receptor antagonist than an agonist.

So there remains a need for non-peptide OT receptor agonists. Such compounds should preferably be selective for the OT receptor over the VP receptors. They could be expected to show therapeutic utility in male and female sexual dysfunction, particularly male erectile dysfunction, in promoting labour, in controlling post-partum bleeding, in increasing milk let-down as well as a number of other indications.

## SUMMARY OF THE INVENTION

We describe herein a series of potent and specific OT receptor agonists. In a first aspect, the present invention comprises novel compounds according to general formula **1**, and pharmaceutically acceptable salts thereof.

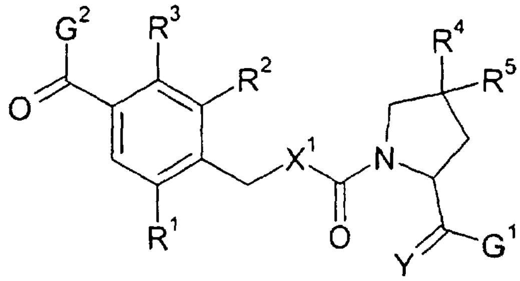

**1**

In general formula **1**, $G^1$ is a group according to general formula **2** or **3**.

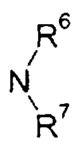

**2**

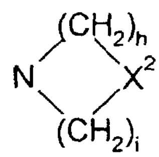

**3**

$G^2$ is a group according to general formula **4**, **5**, **6**, **7**, **8** or **9**.

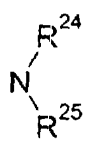

**4**

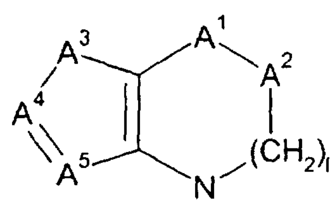

**5**

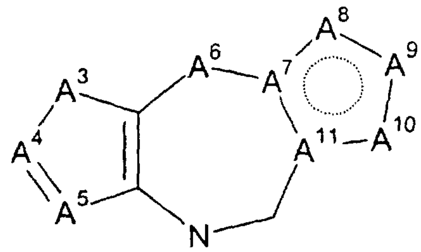

**6**

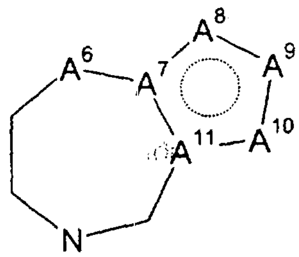
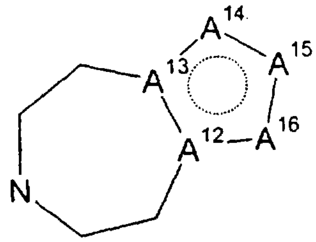
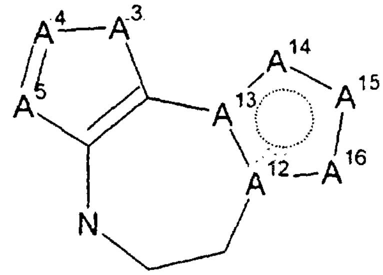

**7** **8** **9**

$A^1$ is $CH_2$, CH(OH), NH, N-alkyl, N-$(CH_2)_n$-$R^{27}$, O or S; $A^2$ is $CH_2$, CH(OH), C(=O) or NH; $A^3$ is S, NH, N-alkyl, -CH=CH- or -CH=N-; $A^4$ and $A^5$ are each CH or N; $A^6$ is $CH_2$, NH, N-alkyl or O; $A^7$ and $A^{11}$ are C or N; $A^8$ and $A^9$ are CH, N, NH, $N(CH_2)_mR^{26}$ or S; $A^{10}$ is -CH=CH-, CH, N, NH, N-$(CH_2)_m$-$R^{26}$ or S; $A^{12}$ and $A^{13}$ are N or C and $A^{14}$, $A^{15}$ and $A^{16}$ are NH, N-$CH_3$, S, N or CH, provided that not more than one of $A^8$, $A^9$ and $A^{10}$ is NH, N-$(CH_2)_m$-$R^{26}$ or S; that $A^7$ and $A^{11}$ are not both simultaneously N; that neither $A^7$ nor $A^{11}$ is N if one of $A^8$, $A^9$ and $A^{10}$ is NH, N-$(CH_2)_m$ -$R^{26}$ or S; that if $A^{10}$ is -CH=CH- then $A^8$ is N, $A^9$ is CH and both $A^7$ and $A^{11}$ are C; that if $A^{10}$ is not -CH=CH- then one of $A^8$, $A^9$ and $A^{10}$ is NH, N-$(CH_2)_m$-$R^{26}$ or S or one of $A^7$ and $A^{11}$ is N; that not more than one of $A^{14}$, $A^{15}$ and $A^{16}$ is NH, N-$CH_3$ or S; that $A^{12}$ and $A^{13}$ are not both simultaneously N; that if one of $A^{14}$, $A^{15}$ and $A^{16}$ is NH, N-$CH_3$ or S then $A^{12}$ and $A^{13}$ are both C; and that one of $A^{14}$, $A^{15}$ and $A^{16}$ is NH, N-$CH_3$ or S or one of $A^{12}$ and $A^{13}$ is N.

$X^1$ is O or NH; $X^2$ is $NR^{16}$, CH-$NR^{17}R^{18}$, CH-$CH_2NR^{17}R^{18}$, $N^+R^{19}R^{20}$, CH-$N^+R^{21}R^{22}R^{23}$ or CH-$CH_2N^+R^{21}R^{22}R^{23}$ and Y is O or S.

$R^1$, $R^2$ and $R^3$ are each H, alkyl, O-alkyl, F, Cl or Br; $R^4$ and $R^5$ are each H, O-alkyl, O-benzyl or F, or $R^4$ and $R^5$ together are =O, -$O(CH_2)_aO$- or -$S(CH_2)_aS$- and $R^6$ is a group according to one of general formulae **10** to **25**.

$$-(CH_2)_b-NR^8R^9 \qquad \textbf{10}$$

$$-(CH_2)_b-N^+R^{11}R^{12}R^{13} \qquad \textbf{11}$$

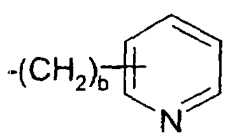
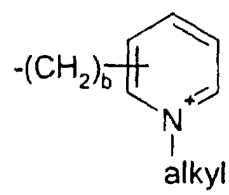

**12** **13**

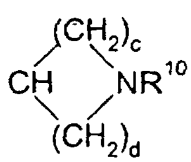
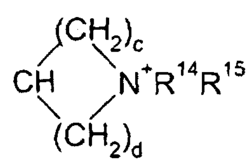

**14** **15**

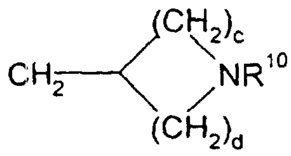

**16**

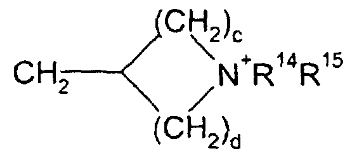

**17**

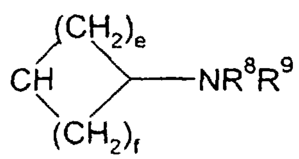

**18**

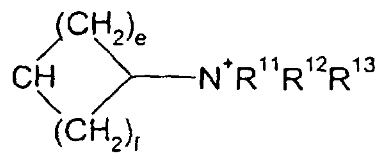

**19**

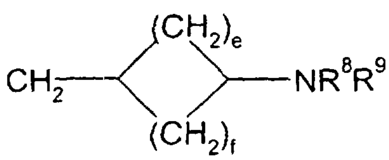

**20**

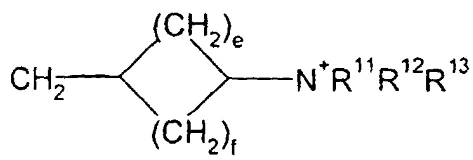

**21**

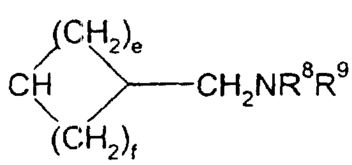

**22**

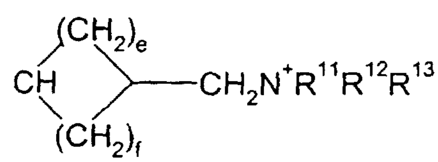

**23**

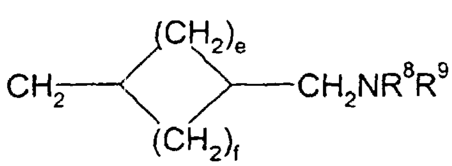

**24**

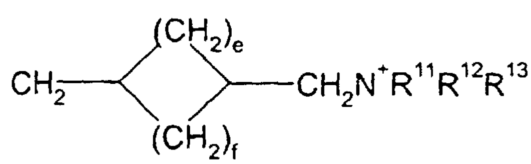

**25**

$R^7$ is H, alkyl or any group as defined above for $R^3$; $R^8$, $R^9$ and $R^{10}$ are independently H or alkyl, or $R^8$ and $R^9$ together may be $-(CH_2)_g-$; $R^{11}$, $R^{12}$, $R^{13}$, $R^{14}$ and $R^{15}$ are all alkyl, or $R^{11}$ and $R^{12}$ together or $R^{14}$ and $R^{15}$ together may be $-(CH_2)_g-$; $R^{16}$, $R^{17}$ and $R^{18}$ are independently H or alkyl, or $R^{17}$ and $R^{18}$ together may be $-(CH_2)_j-$; $R^{19}$, $R^{20}$, $R^{21}$, $R^{22}$ and $R^{23}$ are all alkyl, or $R^{19}$ and $R^{20}$ together or $R^{21}$ and $R^{22}$ together may be $-(CH_2)_j-$; $R^{24}$ and $R^{25}$ are independently alkyl, Ar or $-(CH_2)_k$-Ar; $R^{26}$ is H, alkyl, optionally substituted phenyl, pyridyl, thienyl, OH, O-alkyl, $NH_2$, NH-alkyl, $N(alkyl)_2$, $CO_2H$, $CO_2$-alkyl, $CONH_2$, CONH-alkyl, $CON(alkyl)_2$, CN or $CF_3$, and $R^{27}$ is OH, O-alkyl, O-CO-alkyl, $NH_2$, NH-alkyl or $N(alkyl)_2$.

Ar is thienyl or optionally substituted phenyl.

a is 2 or 3, b is 1, 2 or 3; c is 1 or 2, d is 1, 2 or 3; e is 1 or 2; f is 1, 2 or 3; g is 4, 5 or 6; h is 1, 2 or 3; i is 1, 2, 3 or 4; j is 4, 5 or 6; k is 1, 2 or 3; l is 1 or 2; m is 1, 2 or 3, and n is 2, 3 or 4.

In a second aspect, the present invention comprises pharmaceutical compositions of these novel compounds,

which compositions are useful for the treatment of, *inter alia,* male erectile dysfunction. In further aspects, the present invention comprises the use of such compositions in therapy and therapeutic methods using the compositions.

## DETAILED DESCRIPTION OF THE INVENTION

In a first aspect, the present invention comprises novel benzyl carbamates and ureas according to general formula **1**.

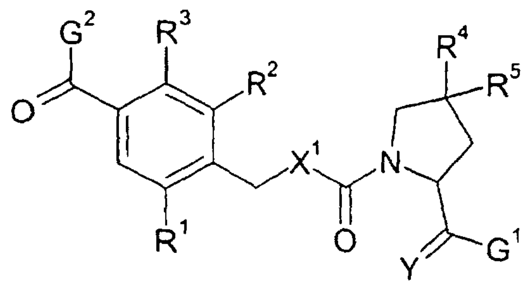

**1**

In this general formula the substituents $R^1$, $R^2$ and $R^3$ are independently selected from hydrogen (H), alkyl groups, alkoxy (O-alkyl) groups, and the halogens fluorine (F), chlorine (Cl) and bromine (Br). Preferably, at least one of $R^1$, $R^2$ and $R^3$ is H and at least one is not H. More preferably, one of $R^1$, $R^2$ and $R^3$ is an alkyl group or a halogen and the others are H. Most preferably, $R^1$ is methyl or Cl and $R^2$ and $R^3$ are both H.

The substituents $R^4$ and $R^5$ may be independently selected from H, O-alkyl, O-benzyl and F. Alternatively, $R^4$ and $R^5$ together may be =O so as to form (together with the carbon atom to which they are attached) the carbonyl group of a ketone. Finally, $R^4$ and $R^5$ together may be $-O(CH_2)_aO-$ or $-S(CH_2)_aS-$, where a is 2 or 3, so as to form (together with the carbon atom to which they are attached) a 1,3-dioxolane, a 1,3-dithiolane, a 1,3-dioxane or a 1,3-dithiane. Preferably, both $R^4$ and $R^5$ are H or one is H and the other is O-alkyl. More preferably, both $R^4$ and $R^5$ are H or one is H and the other is methoxy. Furthermore, in embodiments where one of $R^4$ and $R^5$ is F, it is preferred that the other should also be F.

The linking group $X^1$ is selected from oxygen (O) and unsubstituted nitrogen (NH). Preferably, $X^1$ is NH.

Y is selected from O and sulphur (S).

The group $G^1$ is a mono- or disubstituted nitrogen atom, such that the $C(=Y)-G^1$ bond is an amide or thioamide bond. A requirement of $G^1$ is the presence of at least one basic nitrogen atom or a quaternary salt derived from such a basic nitrogen atom within the group. The basic nitrogen atom may be present as a primary, secondary or tertiary amino group or a pyridyl nitrogen. When $G^1$ includes more than one such basic nitrogen atom then these may be the same or different. Accordingly, $G^1$ is selected from an acyclic group according to general formula **2** and a cyclic group according to general formula **3**.

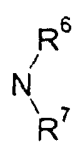

**2**

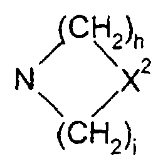

**3**

In general formula **2**, $R^6$ and $R^7$ may be the same or different. $R^6$ is selected from a group according to one of general formulae **10** to **25**.

$$-(CH_2)_b-NR^8R^9 \qquad \textbf{10}$$

$$-(CH_2)_b-N^+R^{11}R^{12}R^{13} \qquad \textbf{11}$$

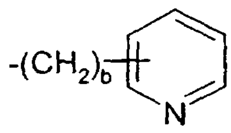

12

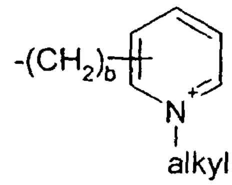

13

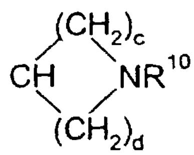

14

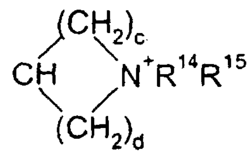

15

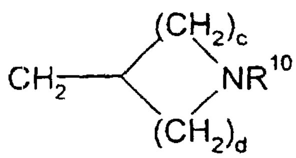

16

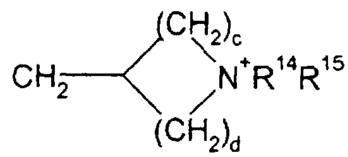

17

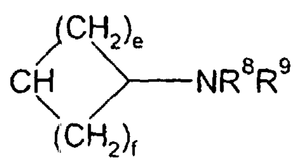

18

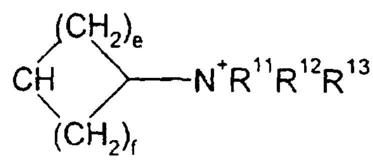

19

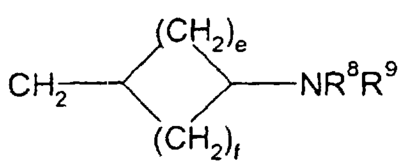

20

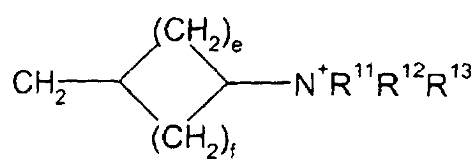

21

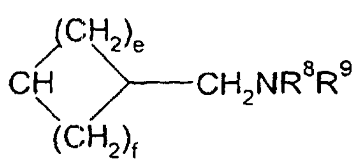

22

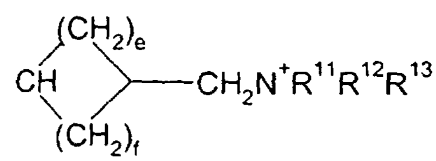

23

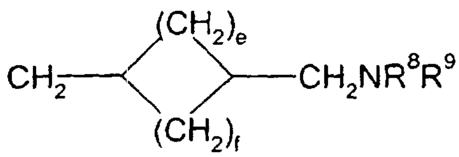

**24**

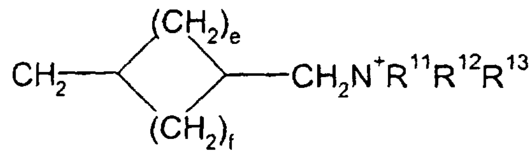

**25**

in the above it will be noted that, for each of the groups that includes a basic nitrogen atom (i.e. for general formulae **10**, **12**, **14**, **16**, **18**, **20**, **22** and **24**) there is a corresponding quaternary derivative (i.e. **11**, **13**, **15**, **17**, **19**, **21**, **23** and **25** respectively). For the polymethylene spacer groups b is 1, 2 or 3; c is 1 or 2, d is 1, 2 or 3; e is 1 or 2 and f is 1, 2 or 3. $R^8$, $R^9$ and $R^{10}$ are independently selected from H and alkyl. Alternatively $R^8$ and $R^9$ together may be a polymethylene chain $-(CH_2)_g-$, where g is 4, 5 or 6, so as to form, together with the nitrogen atom to which they are attached, a pyrrolidine, piperidine or perhydroazepine ring. $R^{11}$, $R^{12}$, $R^{13}$, $R^{14}$ and $R^{15}$ are all alkyl, or $R^{11}$ and $R^{12}$ together or $R^{14}$ and $R^{15}$ together may be $-(CH_2)_g-$, again allowing for pyrrolidine, piperidine and perhydroazepine rings.

For the groups according to general formulae **12** and **13**, the attachment to the pyridine ring may be at the 2-, 3- or 4-position.

$R^7$ may be any of the foregoing groups described for $R^6$, or it may be a non-basic group selected from H and alkyl.

In the group according to general formula **3**, h is 1, 2 or 3 and i is 1, 2, 3 or 4. $X^2$ is selected from the basic groups $NR^{16}$, $CH\text{-}NR^{17}R^{18}$ and $CH\text{-}CH_2NR^{17}R^{18}$ and the corresponding quaternary groups $N^+R^{19}R^{20}$, $CH\text{-}N^+R^{21}R^{22}R^{23}$ and $CH\text{-}CH_2N^+R^{21}R^{22}R^{23}$. $R^{16}$, $R^{17}$ and $R^{18}$ are independently selected from H and alkyl, or $R^{17}$ and $R^{18}$ together may be $-(CH_2)_j-$, where j is 4, 5 or 6, such that $R^{17}$, $R^{18}$ and the nitrogen atom to which they are attached may constitute a pyrrolidine, piperidine or perhydroazepine ring. $R^{19}$, $R^{20}$, $R^{21}$, $R^{22}$ and $R^{23}$ are all alkyl, or $R^{19}$ and $R^{20}$ together or $R^{21}$ and $R^{22}$ together may be $-(CH_2)_j-$.

The group $G^2$ is a disubstituted nitrogen such that the $C(=O)\text{-}G^2$ bond is an amide bond. $G^2$ is selected from an acyclic group according to general formula **4**, a fused bicyclic group according to general formulae **5**, **7** and **8**, and a fused tricyclic group according to general formulae **6** and **9**.

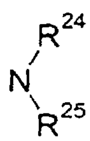

**4**

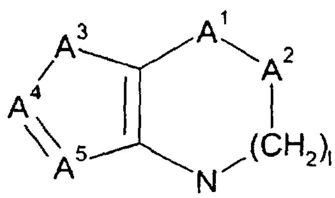

**5**

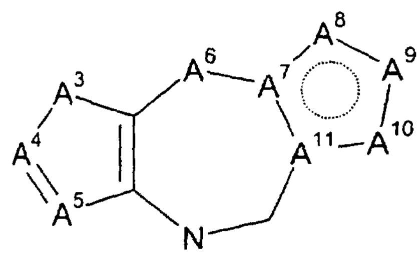

**6**

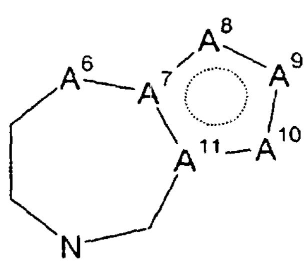

**7**

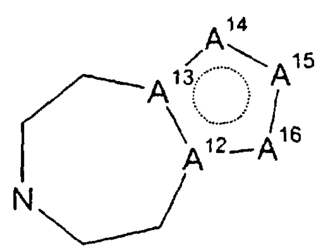

**8**

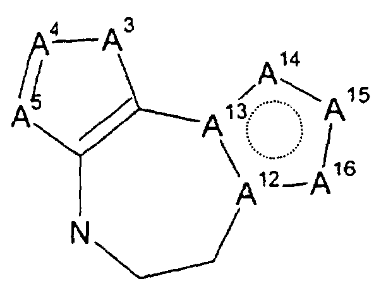

**9**

In general formula **4**, $R^{24}$ and $R^{25}$ are independently selected from alkyl, Ar and $-(CH_2)_k\text{-}Ar$, where k is 1, 2 or 3 and Ar is selected from thienyl and optionally substituted phenyl. Suitable substituents for the phenyl group are alkyl groups, OH, alkoxy groups, halogens, $NH_2$, NH-alkyl and $N(alkyl)_2$. The phenyl group may be substituted with up

to three such substituents which may be the same or different.

In general formula **5**, $A^1$ is selected from $CH_2$, CH(OH), NH, N-alkyl, N-$(CH_2)_n$-$R^{27}$ O and S, where n is 2, 3 or 4 and $R^{27}$ is selected from OH, alkoxy groups, acyloxy (O-CO-alkyl) groups, $NH_2$, NH-alkyl and $N(alkyl)_2$ $A^2$ is selected from $CH_2$, CH(OH), C(=O) and NH, and l is 1 or more preferably 2. It is preferred that when $A^2$ is NH then $A^1$ is $CH_2$. It is also preferred that when $A^2$ is C(=O) then $A^1$ is NH or N-alkyl.

In general formulae **5**, **6** and **9**, $A^3$ is selected from S, NH, N-alkyl, -CH=CH- and -CH=N- and $A^4$ and $A^5$ are each selected from CH and N. In a preferred embodiment, $A^3$ is S and $A^4$ and $A^5$ are both CH, so as to form a thiophene ring. In another preferred embodiment, $A^3$ is -CH=CH- and $A^4$ and $A^5$ are both CH, so as to form a benzene ring. In another preferred embodiment, $A^3$ is -CH=N- and $A^4$ and $A^5$ are both CH, so as to form a pyridine ring. In another preferred embodiment, $A^3$ is -CH=CH-, $A^4$ is CH and $A^5$ is N, again so as to form a pyridine ring.

In general formulae **6** and **7**, $A^6$ is selected from $CH_2$, NH, N-alkyl and O, $A^7$ and $A^{11}$ are selected from C and N, $A^8$ and $A^9$ are selected from CH, N, NH, N-$(CH_2)_m$-$R^{26}$ and S and $A^{10}$ is selected from -CH=CH-, CH, N, NH, N-$(CH_2)_m$-$R^{26}$ and S, where m is 1, 2 or 3 and $R^{26}$ is selected from H, alkyl, optionally substituted phenyl, pyridyl, thienyl, OH, O-alkyl, $NH_2$, NH-alkyl, $N(alkyl)_2$, $CO_2H$, $CO_2$-alkyl, $CONH_2$, CONH-alkyl, $CON(alkyl)_2$, CN and $CF_3$. Suitable substituents for the phenyl group are alkyl groups, OH, alkoxy groups, halogens, $NH_2$, NH-alkyl and $N(alkyl)_2$. The phenyl group may be substituted with up to three such substituents which may be the same or different.

The ring constituted by $A^7$, $A^8$, $A^9$, $A^{10}$ and $A^{11}$ is aromatic, and accordingly the groups must satisfy certain requirements. When $A^{10}$ is -CH=CH- the ring is a six-membered ring. As such, it can only comprise atoms of the type -C(R)= and -N=. Hence $A^7$ and $A^{11}$ must both be C and $A^8$ and $A^9$ must be either CH or N. We have found that suitable activity is only obtained when $A^8$ is N and $A^9$ is CH. When $A^{10}$ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when $A^{10}$ is not -CH=CH- then one (and only one) of $A^7$, $A^8$, $A^9$, $A^{10}$ and $A^{11}$ must be S or a trigonal nitrogen. Hence the selection of $A^7$, $A^8$, $A^9$, $A^{10}$ and $A^{11}$ is subject to the following restrictions.

1) If $A^{10}$ is not -CH=CH- then one of $A^8$, $A^9$ and $A^{10}$ is NH, N-$(CH_2)_m$-$R^{26}$ or S or one of $A^7$ and $A^{11}$ is N.
2) Not more than one of $A^8$, $A^9$ and $A^{10}$ may be NH, N-$(CH_2)_m$-$R^{26}$ or S.
3) $A^7$ and $A^{11}$ may not both simultaneously be N.
4) Neither $A^7$ nor $A^{11}$ may be N if one of $A^8$, $A^9$ and $A^{10}$ is NH, $N(CH_2)_mR^{26}$ or S.

In a preferred embodiment, $A^6$ is NH. In another preferred embodiment, $A^8$ is NH or N-$(CH_2)_m$-$R^{26}$. In a more preferred embodiment, $A^8$ is NH or N-$(CH_2)_m$-$R^{26}$, $A^9$ is N and $A^{10}$ is CH.

In general formulae **8** and **9**, $A^{12}$ and $A^{13}$ are selected from N and C and $A^{14}$, $A^{15}$ and $A^{16}$ are selected from NH, N-$CH_3$, S, N and CH. Again, these atoms constitute an aromatic five-membered ring and so there must be one, and only one, S or trigonal nitrogen. Hence the selection of $A^{12}$, $A^{13}$, $A^{14}$, $A^{15}$ and $A^{16}$ is subject to the following restrictions.

1) One of $A^{14}$ , $A^{15}$ and $A^{16}$ is NH, N-$CH_3$ or S or one of $A^{12}$ and $A^{13}$ is N.
2) Not more than one of $A^{14}$, $A^{15}$ and $A^{16}$ is NH, N-$CH_3$ or S.
3) $A^{12}$ and $A^{13}$ may not both simultaneously be N.
4) If one of $A^{14}$, $A^{15}$ and $A^{16}$ is NH, N-$CH_3$ or S then $A^{12}$ and $A^{13}$ are both C

As used herein, the term "alkyl" is intended to designate lower alkyl groups, i.e. saturated hydrocarbon groups of between one and six carbon atoms, including linear, branched and cyclic alkyl groups. Examples of "alkyl" include, but are not limited to; $C_1$ - methyl, $C_2$ - ethyl, $C_3$ - propyl, isopropyl, cyclopropyl, $C_4$ - n-butyl, sec-butyl, isobutyl, tert-butyl, cyclobutyl, cyclopropylmethyl, methylcyclopropyl, $C_5$ - n-pentyl, neopentyl, cyclopentyl, cyclopropylethyl, dimethylcyclopropyl, and $C_6$ - n-hexyl, cyclohexyl, bicyclo[3.1.0]hexyl.

Those compounds according to the present invention that contain a basic nitrogen atom are capable of forming addition salts with protic acids such as hydrochloric acid, sulphuric acid, phosphoric acid, acetic acid, trifluoroacetic acid, benzoic acid, maleic acid, citric acid, fumaric acid, methanesulphonic acid and the like. Those compounds that contain a quaternary nitrogen atom will exist as salts with an appropriate counterion such as chloride, bromide, iodide, sulphate, phosphate, acetate, trifluoroacetate, benzoate, maleate, citrate, fumarate, methanesulphonate and the like. The compounds of the present invention may also contain an acidic group, such as a carboxylic acid group at $R^{26}$. These compounds may exist as inner salts (zwitterions) or as salts such as sodium, potassium, magnesium, calcium or tetra-alkylammonium salts. To the extent that such salts are pharmaceutically acceptable, they are included within the scope of the present invention.

The compounds according to the present invention all have at least one stereogenic centre ("asymmetric

carbon atom") and so may exhibit optical isomerism. The scope of the present invention includes all epimers, enantiomers and diastereomers of compounds according to general formula **1**, including single isomers, mixtures and racemates.

Particularly preferred embodiments within the present invention are those compounds that combine two or more of the preferred features described above. One such particularly preferred embodiment is a thioamide according to general formula **26.**

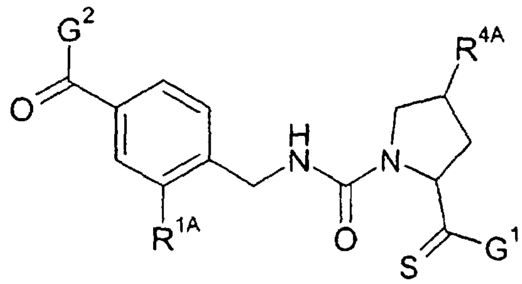

**26**

In general formula **26**, $R^{1A}$ is methyl or Cl and $R^{4A}$ is H or O-methyl. $G^1$ and $G^2$ are as previously defined.

Another particularly preferred embodiment is an amide according to general formula **27**.

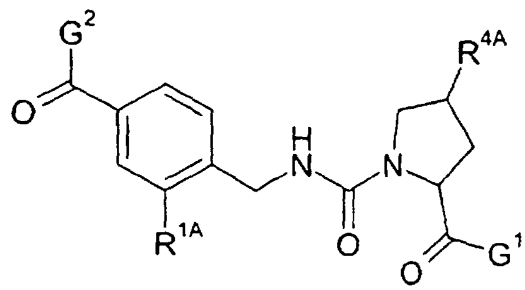

**27**

In general formula **27**, $R^{1A}$, $R^4A$ , $G^1$ and $G^2$ are as previously defined.

Another particularly preferred embodiment is a compound according to general formula **28**.

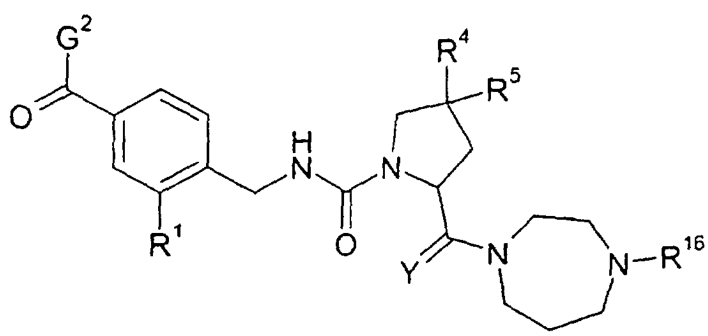

**28**

In general formula **28,** $R^1$, $R^4$, $R^5$, $R^{16}$, $G^2$ and Y are as previously defined.

Another particularly preferred embodiment is a compound according to general formula **29.**

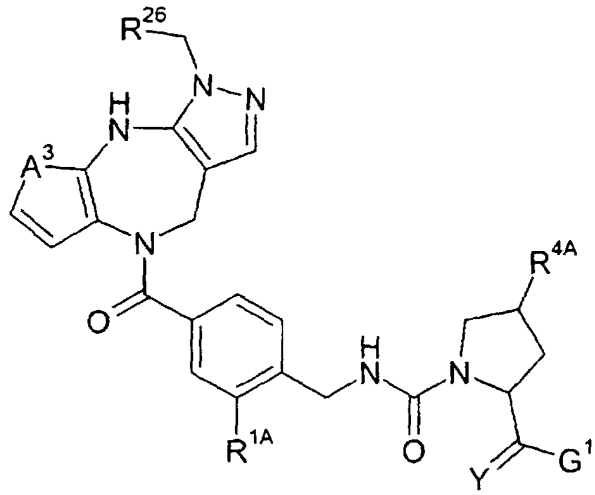

**29**

In general formula **29**, $R^{1A}$, $R^{4A}$, $R^{26}$, $A^3$, $G^1$ and Y are as previously defined.

A most preferred embodiment is a compound according to general formula **30**.

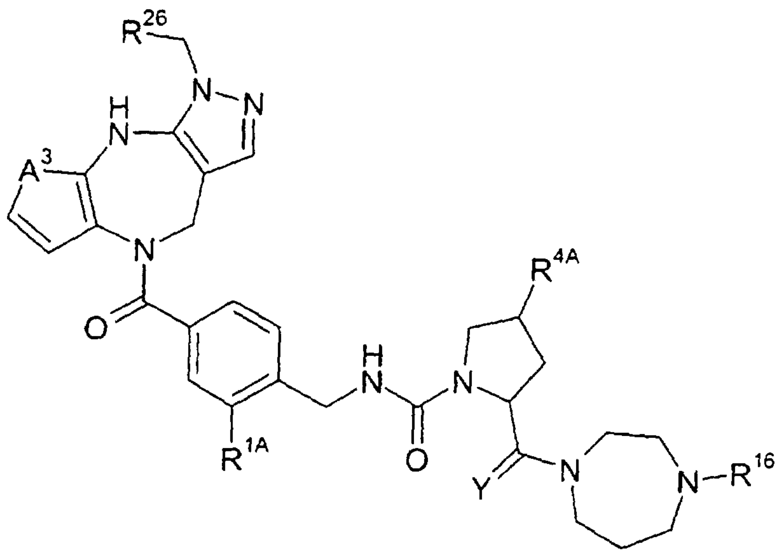

**30**

In general formula **30**, $R^{1A}$, $R^{4A}$, $R^{16}$, $R^{26}$, $A^3$ and Y are as previously defined.

Individual preferred compounds within the invention include:

4-methyl-1-(*N*-(2-methyl-4-(2,3,4,5-tetrahydro-1,5-benzodiazepin-4-on-1-ylcarbonyl)-benzylcarbamoyl)-L-thioprolyl)perhydro-1,4-diazepine,

4-methyl-1-(*N*-(2-methyl-4-(1-methyl-4,10-dihydropyrazolo[5,4-*b*][1,5]benzodiazepin-5-yl-carbonyl)benzylcarbamoyl)-L-thioprolyl)perhydro-1,4-diazepine,

4,4-dimethyl-1-(*N*-(2-methyl-4-(1-methyl-4,10-dihydropyrazolo[5,4-*b*][1,5]benzodiazepin-5-ylcarbonyl)benzylcarbamoyl)-L-thioprolyl)perhydro-1,4-diazepinium iodide,

4-methyl-1-(*N*-(2-methyl-4-(5,6,7,8-tetrahydrothieno[3,2-*b*]azepin-4-ylcarbonyl)benzyl-carbamoyl)-L-thioprolyl) perhydro-1,4-diazepine,

4-methyl-1-(*N*-(2-methyl-4-(5,6,7,8-tetrahydrothieno[3,2-*b*]azepin-4-ylcarbonyl)benzyloxy-carbonyl)-L-prolyl)perhydro-1,4-diazepine,

(4*R*)-*N*$^{\alpha}$-(2-chloro-4-(5,6,7,8-tetrahydrothieno[3,2-*b*]azepin-4-ylcarbonyl)benzyl-carbamoyl)-4-methoxy-L-proline-*N*-methyl-*N*-(2-picolyl)amide, and

1-((4*R*)-*N*$^{\alpha}$-(2-chloro-4-(5,6,7,8-tetrahydrothieno[3,2-*b*]azepin-4-ylcarbonyl)benzyl-carbamoyl)-4-methoxy-L-prolyl)-4-(1-pyrrolidinyl)piperidine

The compounds of the present invention can be prepared by standard chemical manipulations. In general, compounds according to general formula **1** can be considered to consist of four component parts:

- Component $C^1$ corresponding to $G^2$
- Component $C^2$ corresponding to the substituted benzoyl unit
- Component $C^3$ corresponding to the pyrrolidine unit
- Component $C^4$ corresponding to $G^1$.

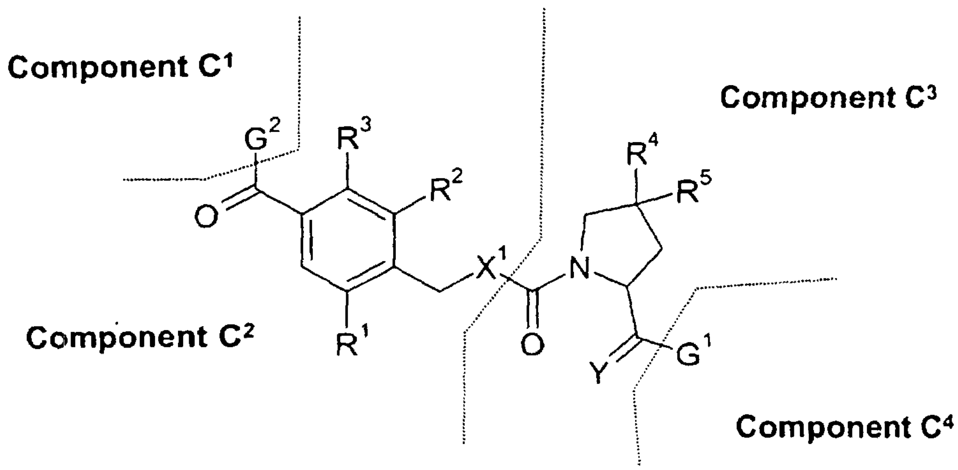


Intermediates corresponding to these components are prepared and then assembled to give the final product. These four components are:

(i) for $C^1$, a secondary amine

$$G^2-H$$

(ii) for $C^2$, a substituted benzoic acid

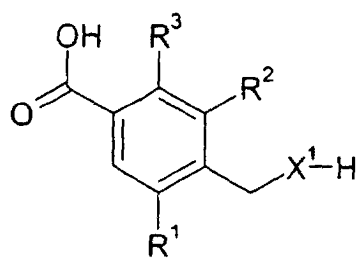

(iii) for $C^3$, a proline derivative

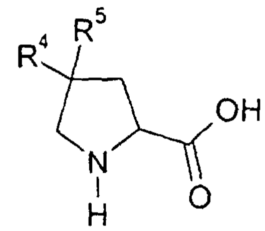

(iv) for $C^4$, a primary or secondary amine

$$H\text{-}G^1$$

It will be recognised that the substituted benzoic acid for $C^2$ and the proline derivative for $C^3$ both have two functional groups that will need temporary protection during the assembly of the final compound. The principles of functional group protection are well known in the art and are described in, for example, J.F.W. McOmie, "Protective Groups in Organic Chemistry", Plenum Press, 1973; T.W. Greene and P.G.M. Wuts, "Protective Groups in Organic Synthesis", 2nd edition, John Wiley, 1991; and P.J. Kocienski, "Protecting groups", Georg Thieme Verlag, 1994. The carboxylic acid group will usually be protected as an ester, such as the methyl, benzyl or tert-butyl ester. The secondary amine of the proline and the primary amine of the benzoic acid (when $X^1$ = NH) will usually be protected as a carbamate derivative such as the tert-butyl carbamate (BOC derivative), the benzyl carbamate (CBZ or more simply Z derivative) or the 9-fluorenylmethyl carbamate (Fmoc derivative). When $X^1$ = O the resulting alcohol function will usually be protected as an ester such as an acetate, or an ether such as a methoxymethyl, tetrahydropyranyl or trialkylsilyl ether. Other functional groups may require protection. For example, the group $G^1$ may include one or more primary or secondary amino groups which may need protection. In the following general description of the synthetic methodology it will be assumed that such protection is used when necessary.

*(i) Preparation of secondary amine for $C^1$*

Acyclic secondary amines corresponding to $HNR^{24}R^{25}$ are well known. Many are items of commerce. Those that are not may be prepared according to published methods or by simple modification of such methods. Some particularly useful methods are listed below.

a) Alkylation

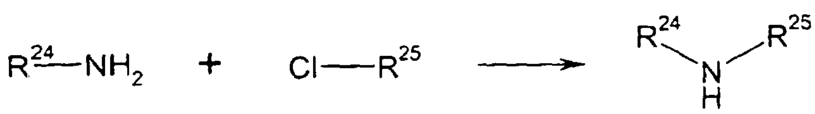

(This method is only applicable in cases where further alkylation can be avoided.)

b) Reductive amination

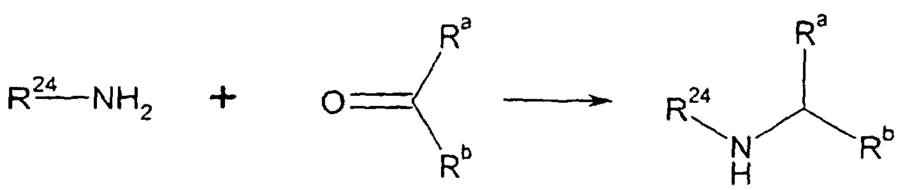

(where $R^aCHR^b$ corresponds to $R^{25}$)

c) Amide reduction

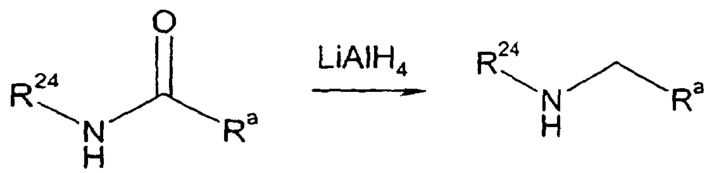


(where $R^aCH_2$ corresponds to $R^{25}$)

The starting amide can itself be prepared using well known methods.

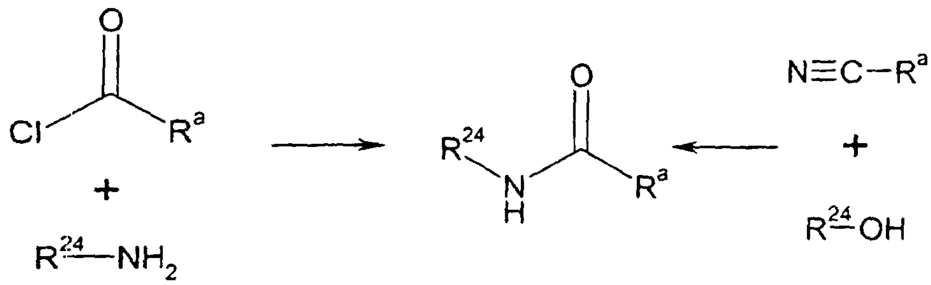

Secondary amines corresponding to $C^1$ where $G^2$ is a group according to general formulae **5 - 9** are generally not commercially available. They can be prepared according to published methods, or by obvious modifications of such methods. Particularly useful methods are described in: Aranapakam *et al.,* Bioorg. Med. Chem. Lett. 1993, 1733; Artico *et al.,* Farmaco. Ed. Sci. 24, 1969, 276; Artico *et al.,* Farmaco. Ed. Sci. 32., 1977, 339; Chakrabarti *et al.*, J. Med. Chem. 23, 1980, 878; Chakrabarti *et al.,* J. Med. Chem. 23, 1980, 884; Chakrabarti *et al.,* J. Med. Chem. 32, 1989, 2573; Chimirri *et al.,* Heterocycles 36, 1993, 601; Grunewald *et al.,* J. Med. Chem. 39, 1996, 3539; Klunder *et al.,* J. Med. Chem. 35, 1992, 1887; Liegeois *et al.,* J. Med. Chem. 37, 1994, 519; Olagbemiro *et al.,* J. Het. Chem. 19, 1982, 1501; Wright *et al.,* J. Med. Chem. 23, 1980, 462; Yamamoto *et al.,* Tet. Lett. 24, 1983, 4711; and International patent application, publication number WO99/06403.

*(ii) Preparation of substituted benzoic acid for $C^2$*

Substituted benzoic acids corresponding to $C^2$ are not generally items of commerce, but they can be prepared using published methods or obvious variations of such methods. The main challenge is generally the elaboration of the $-CH_2X^1H$ functionality at the 4-position. Some useful transformations are listed below.

a) Bromination/Substitution

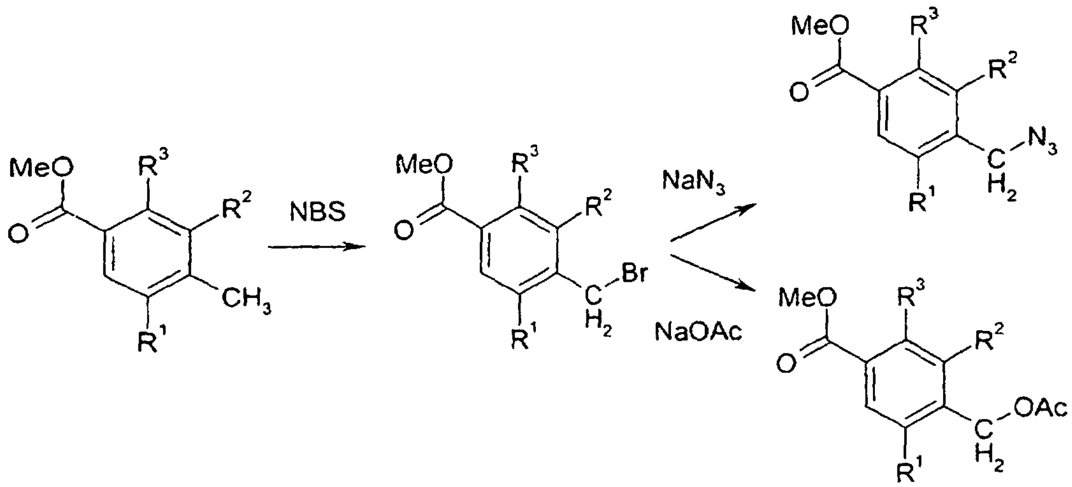


b) Sandmeyer reaction/reduction

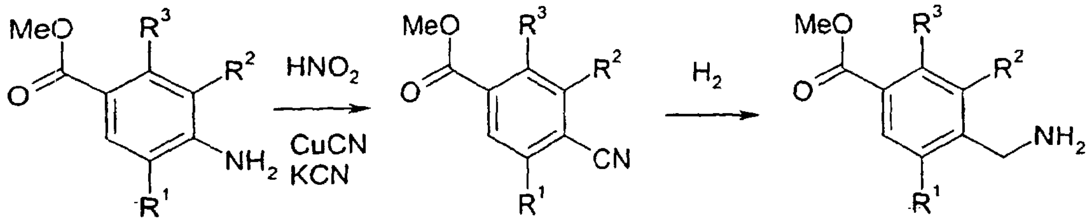


*(iii) Preparation of proline derivative for $C^3$*

Proline and hydroxyproline derivatives are items of commerce. Other proline derivatives corresponding to $C^3$ can be prepared according to the methods set out in Dugave *et al*., Tet. Lett. 39, 1998, 1169; Petrillo *et al.,* J. Med. Chem. 31, 1988. 1148; and Smith *et al*., J. Med. Chem. 31, 1988, 875.

*(iv) Preparation of primary or secondary amine for $C^4$*

As noted in (i) above, many amines are commercially available and others are readily accessible using well established chemistry. The quaternary ammonium salts can generally be prepared by treating a primary, secondary or tertiary amine with an excess of alkylating agent.

With the four components, suitably protected if necessary, in hand, the assembly of the final compound requires the formation of three bonds: between $C^1$ and $C^2$, between $C^2$ and $C^3$, and between $C^3$ and $C^4$. These bond-forming steps may be taken in any order. Thus, the following sequences can be proposed:

$$C^1 + C^2 \rightarrow C^1C^2 \rightarrow C^1C^2C^3 \rightarrow C^1C^2C^3C^4$$

$$C^3 + C^4 \rightarrow C^3C^4 \rightarrow C^2C^3C^4 \rightarrow C^1C^2C^3C^4$$

$$C^2 + C^3 \rightarrow C^2C^3 \rightarrow C^2C^3C^4 \rightarrow C^1C^2C^3C^4$$

$$C^2 + C^3 \rightarrow C^2C^3 \rightarrow C^1C^2C^3 \rightarrow C^1C^2C^3C^4$$

$$C^1 + C^2 \rightarrow C^1C^2;\ C^3 + C^4 \rightarrow C^3C^4;\ C^1C^2 + C^3C^4 \rightarrow C^1C^2C^3C^4$$

*(i) Formation of $C^1$-$C^2$bond*

The bond between $C^1$ and $C^2$ is a simple amide bond. The chemistry for making such bonds from a carboxylic acid and a secondary amine is well known in the art of organic synthesis, and particularly in the field of peptide synthesis. The carboxylic acid may be converted into a more reactive species such as an acid chloride (using, for example oxalyl chloride or thionyl chloride) or a mixed anhydride (using isobutyl chloroformate). This reactive species is then added to the secondary amine in a suitable solvent, generally an aprotic solvent such as dichloromethane or dimethylformamide, in the presence of a base such as triethylamine or 4-dimethylaminopyridine, and the reaction is allowed to proceed at a temperature between -20°C and the boiling point of the solvent. The choice of temperature and the time allowed for the reaction will depend on the reactivity of the two components.

Alternatively, the carboxylic acid and the secondary amine may be mixed in a suitable solvent as above, optionally in the presence of a base, and a condensing agent added. Suitable condensing agents include carbodiimides, such as dicyclohexylcarbodiimide (DCC) and N-ethyl-N'-dimethylaminopropylcarbodiimide (EDC, also WSCDI for water-soluble carbodiimide), phosphorus reagents such as (benzotriazol-1-yloxy)-tris(dimethylamino)phosphonium hexafluorophosphate (BOP), (benzotriazol-1-yloxy)-tripyrrolidinophosphonium hexafluorophosphate (PyBOP®) and bromotripyrrolidino-phosphonium hexafluorophosphate (PyBroP®), and ureas such as O-(benzotriazol-1-yl)-N,N,N',N'-tetramethyluronium hexafluorophosphate (HBTU).

*(ii)Formation of $C^2$-$C^3$ bond*

The bond between $C^2$ and $C^3$ is a carbamate (when $X^1$ = O) or a urea (when $X^1$ = NH). The first step in the formation of this bond is generally to react the proline derivative with phosgene or a phosgene equivalent such as trichloromethyl chloroformate, bis(trichloromethyl)carbonate or carbonyldiimidazole. Again, an aprotic solvent and a tertiary amine base will generally be used. The intermediate formed in this step is usually not isolated. The alcohol ($X^1$ = O) or amine ($X^1$ = NH) is added and the reaction is allowed to continue, directly forming the carbamate or urea. As an alternative, when $X^1$ = NH the reactive intermediate may be formed by the reaction of $C^2$ with the phosgene equivalent and the proline added in the second part of the synthesis.

*(iii) Formation* of *$C^3$-$C^4$ bond*

When Y = O, the bond between $C^3$ and $C^2$ is a simple amide bond, and it may be formed by the methods described above for the formation of the $C^1$-$C^2$ bond. When Y = S, the bond is a thioamide. This is generally prepared by first making the simple amide and then treating this with Lawesson's reagent (2,4-bis(4-methoxyphenyl)-1,3-dithia-2,4-diphosphetane-2,4-disulfide).

The compounds according to the present invention are useful in human and animal therapy. When so used, they will generally be formulated in an appropriate manner. Thus a second aspect of the present invention is a pharmaceutical formulation that includes a compound as described above as an active ingredient. A third aspect of the present invention is the use of a compound according to the first aspect in the manufacture of such a composition.

The composition according to the present invention may be presented in any form that is known in the art. For example, the formulation may be presented as a tablet, capsule, powder, suppository, cream, solution or suspension, or in a more complex form such as an adhesive patch. The formulation will generally include one or more excipients, such as diluents, bulking agents, binding agents, dispersants, solvents, preservatives, flavoring agents and the like. Where the formulation is presented as a tablet or capsule the excipients may optionally include one or more agents to control the release of the active species, such as a coating of a polymer that is insoluble at low pH but soluble at neutral or high pH. Such a coating (known as an "enteric coating") prevents the release of the active agent in the stomach but allows its release in the intestines. The formulation may also include one or more additional pharmacologically active species. Preferably the formulation includes no such additional active agents.

In further aspects, the present invention comprises the use of such compositions, and hence of the compounds of the invention, in human and animal therapy, and methods of treatment involving such use of the compositions and compounds. The compounds of the present invention are potent and selective oxytocin receptor agonists, and so the compositions are useful in the treatment of conditions for which inadequate oxytocin-like activity is implicated in the pathophysiology. Such conditions include, but are not limited to: sexual disorders such as male erectile dysfunction and ejaculatory disorders, female sexual dysfunction, cancer of the prostate, breast, ovary and bones, osteoporosis, benign prostatic hyperplasia, post-partum bleeding, and depression. The compositions may also be used to induce labour or delivery of the placenta, to decrease arterial blood pressure, to decrease exaggerated responses to stress

and to increase the nociceptive threshold.

In a preferred embodiment, the composition is used to treat male or female sexual dysfunction, and more preferably erectile dysfunction.

When used as therapeutic agents, the compositions of the present invention may be administered by any appropriate route that is known in the art. For example, they may be administered by the oral, buccal, sublingual, rectal, intravaginal, nasal, pulmonary or transdermal routes. Alternatively, they may be given by injection, including intravenous, subcutaneous and intramuscular injection. The amount given will be determined by the attending physician taking into consideration all appropriate factors. Generally a single dose will comprise between 0.1mg and 1000mg, preferably between 1mg and 250mg, of active compound. The dose may be given on a single occasion or repeatedly. When given repeatedly, it may be given at regular intervals, such as once, twice or three times daily, or on demand, according to the condition being treated.

For long-term treatment an alternative to repeated dosing may be the administration of a depot dose. For this method of administration the active agent is generally introduced into a matrix of biodegradable polymer, such as a copolymer of lactic and glycolic acids, and the formulation is given either *s.c.* or *i.m.* so as to form a deposit from which the active agent is released as the polymer degrades.

The foregoing description is further illustrated in the following examples, which are intended to demonstrate the application of the invention but not to limit the scope thereof.

**EXAMPLES**

The following abbreviations have been used:

| | |
|---|---|
| Bu | butyl - alkyl residues may be further denoted as n (normal, i.e. unbranched), i (iso) and t (tertiary) |
| DIEA | *N,N*-diisopropylethylamine |
| DMF | dimethylformamide |
| Et | ethyl |
| HOBt | 1-hydroxybenzotriazole |
| HPLC | high pressure liquid chromatography |
| hr | hour(s) |
| Me | methyl |
| MS | mass spectrum |
| NMR | nuclear magnetic resonance spectrum |
| OVA | ornithine vasotocin analogue |
| pet. ether | petroleum ether boiling in the range 60-80°C |
| Ph | phenyl |
| Pn | pentyl |
| Pr | propyl |
| RT | room temperature |
| THF | tetrahydrofuran |
| WSCD | water-soluble carbodiimide (*N*-ethyl-*N'*-(3-dimethylaminopropyl)carbodiimide hydrochloride |

Examples 1-10 describe the synthesis of intermediates. Compounds according to the present invention are described in Examples 11 to 105.

**Example 1**

**1-Benzyl-4,10-dihydropyrazolo[5,4-*b*][1,5]benzodiazepine**

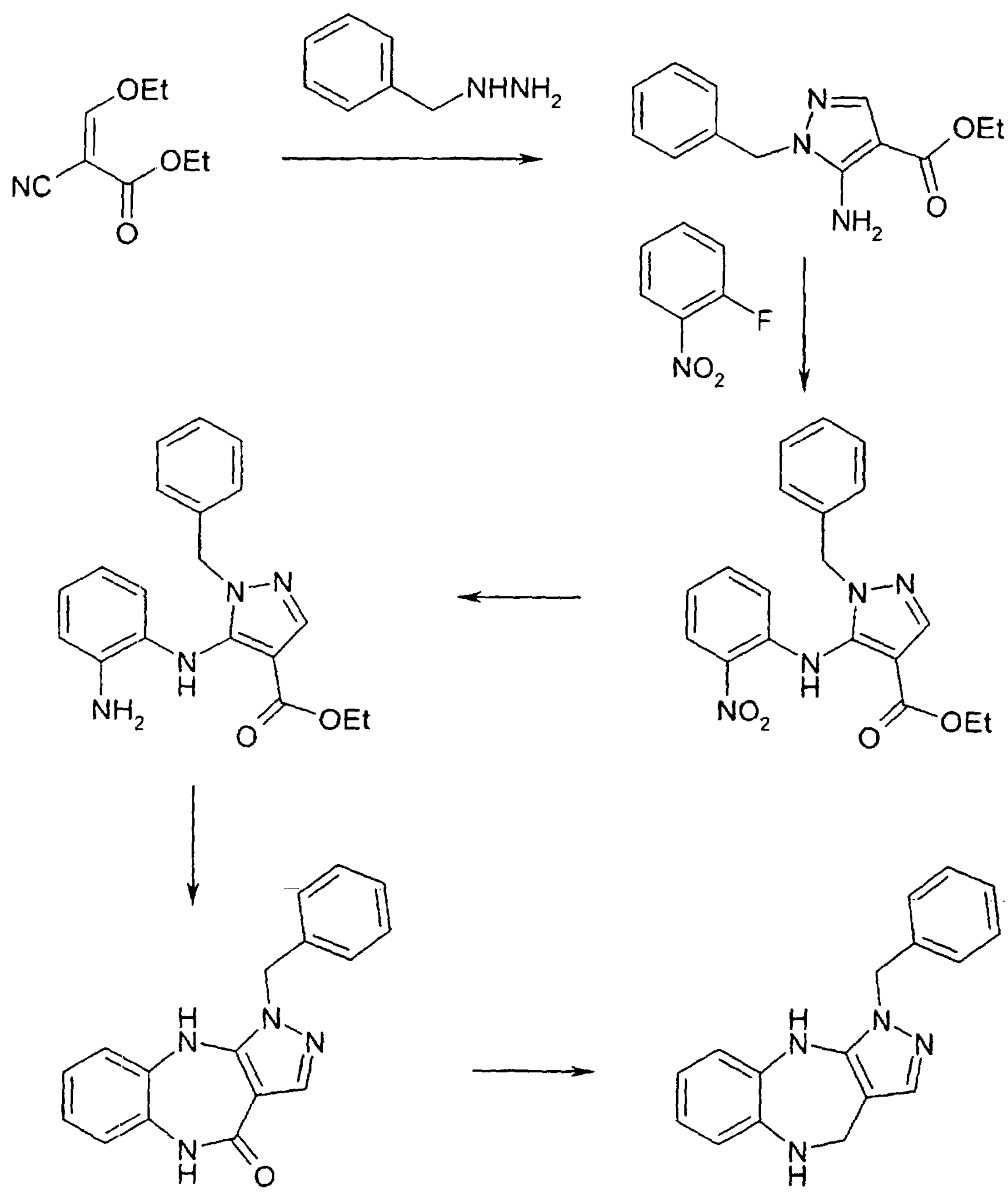

**1A: Ethyl 5-amino-1-benzylpyrazole-4-carboxylate**

Benzylhydrazine dihydrochloride (4.29g, 22mmol) was added to a solution of ethyl (ethoxymethylene)cyanoacetate (3.38g, 20mmol) and triethylamine (6.15ml, 44mmol, 2eq) in ethanol (40ml) and the mixture was heated at reflux for 18hr. The solvent was removed *in vacuo* and the residue was purified by flash chromatography on silica gel (eluant 60% pet. ether/40% ethyl acetate) to yield a pale yellow solid identified as ethyl 5-amino-1-benzylpyrazole-4-carboxylate (4.3g, 88%).

**1B: Ethyl 1-benzyl-5-(2'-nitrophenylamino)pyrazole-4-carboxylate**

Sodium hydride (60% dispersion in oil, 520mg, 13mmol) was added portionwise to a suspension of ethyl 5-amino-1-benzylpyrazole-4-carboxylate (2.2g, 9mmol) in anhydrous THF (30ml) at 0°C. The mixture was allowed to warm to room temperature and stirred for 2hr then 1-fluoro-2-nitrobenzene (1.26g, 9mmol) was added and the resultant deep purple suspension was stirred at RT for 18hr. 1M $KHSO_4$ was added to quench the reaction and the solvent was removed *in vacuo.* The residue was dissolved in ethyl acetate and the solution was washed with 0.3M $KHSO_4$, sat. $NaHCO_3$ and brine, dried over $Na_2SO_4$ and concentrated *in vacuo.* The residue was purified by flash chromatography on silica gel (eluant 75% pet. ether/25% ethyl acetate) to yield ethyl 1-benzyl-5-(2'-nitrophenylamino)pyrazole-4-carboxylate (2.5g, 76%).
**MS** $[M+H]^+$ 366.8

**1C: Ethyl 5-(2'-aminophenylamino)-1-benzylpyrazole-4-carboxylate**

Ethyl 1-benzyl-5-(2'-nitrophenylamino)pyrazole-4-carboxylate (2.5g, 6.8mmol) was dissolved in ethyl acetate/ethanol (1:1, 100ml) and hydrogenated over 10% Pd/C catalyst for 70 minutes. The mixture was filtered through Celite® filter agent and the filtrate was concentrated *in vacuo* to give a white solid identified as ethyl 5-(2'-aminophenylamino)-1-benzylpyrazole-4-carboxylate (1.5g, 86%).
**MS** $[M+H]^+$ 337.2

**1D: 1-Benzyl-4,10-dihydropyrazolo[5,4-*b*][1,5]benzodiazepin-4(5*H*)-one**

A solution of ethyl 5-(2'-arninophenylamino)-1-benzylpyrazole-4-carboxylate (1.75g, 5.2mmol) in acetic acid/2-propanol (1:9, 40ml) was heated at reflux for 3 days. The solvent was removed *in vacuo* and the residue was azeotroped with toluene to give an off-white solid that was purified by flash chromatography on silica gel (eluant 35% pet. ether/65% ethyl acetate) to yield a white solid identified as 1-benzyl-4,10-dihydro-pyrazolo[5,4-*b*][1,5]benzodiazepin-4(5*H*)-one (780mg, 52%).
**MS** $[M+H]^+$ 291.1

**1E: 1-Benzyl-4,10-dihydropyrazolo[5,4-*b*][1,5]benzodiazepine**

$LiAlH_4$ (365mg, 10mmol) was added portionwise to a suspension of 1-benzyl-4,10-dihydropyrazolo[5,4-*b*][1,5]benzodiazepin-4(5*H*)-one (780mg, 2.7mmol) in anhydrous THF (15ml) at 0°C over 10min. The resulting suspension was heated at reflux for 18hr, then allowed to cool to room temperature. A further portion of $LiAlH_4$ (90mg, 2.5mmol) was added and the mixture was heated at refluxed for 3hr. The mixture was cooled to 0°C, 35% ammonia solution (1ml) was added dropwise over 10min and the mixture was stirred at RT for 1hr. The resulting suspension was filtered through Celite® filter agent and the filtrate was concentrated *in vacuo* to give a white solid identified as 1-benzyl-4,10-dihydropyrazolo[5,4-*b*][1,5]benzodiazepine (450mg, 60%).
**MS** $[M+H]^+$ 276.9

**Example 2**

**1-Methyl-4,10-dihydropyrazolo[4,5-*c*]pyrido[2,3-*b*][1,4]diazepine**

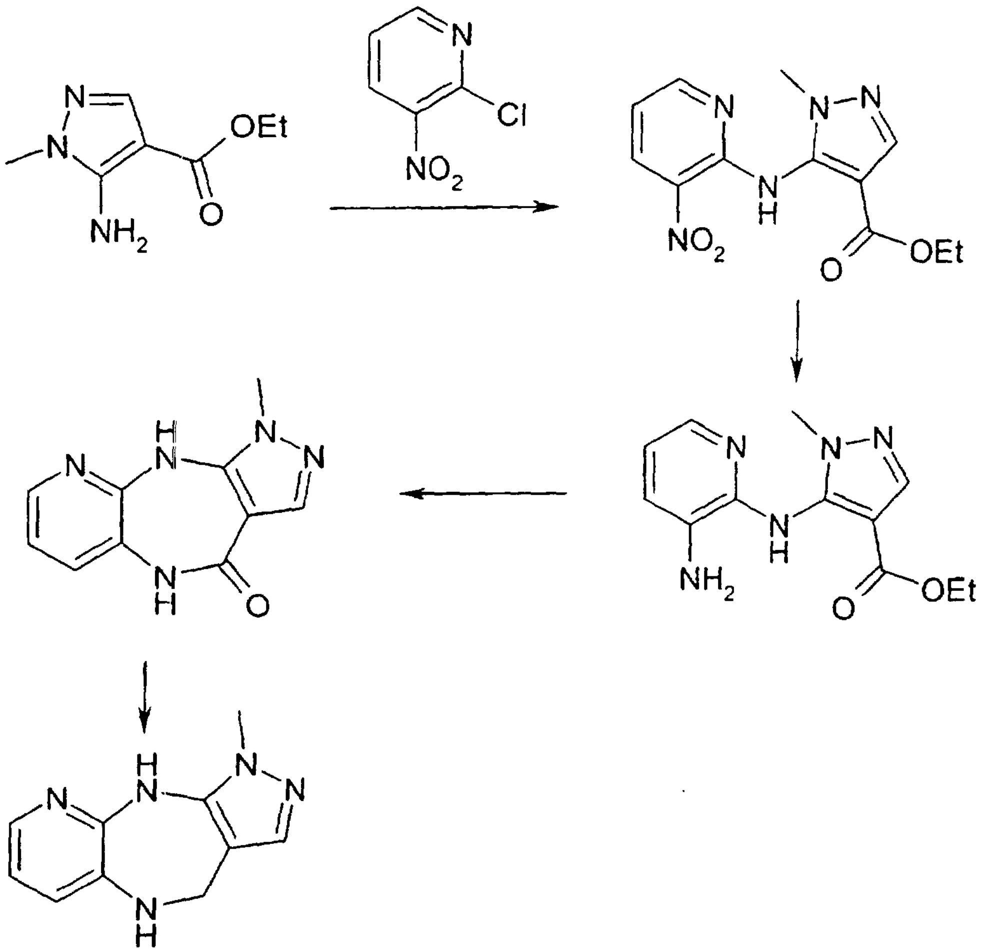

**2A: Ethyl 1-methyl-2-(3'-nitro-2'-pyridylamino)pyrazole-4-carboxylate**

Sodium hydride (60% dispersion in oil, 600mg, 15mmol) was added portionwise to a suspension of ethyl 5-amino-1-methylpyrazole-4-carboxylate (1.69g, 10mmol) in anhydrous THF (15ml) at 0°C. The mixture was stirred for 2hr at RT then 2-chloro-3-itropyridine (1.58g, 10mmol) was added and the resulting deep red suspension was stirred at RT for 18hr. 1M $KHSO_4$ was added to quench the reaction and the solvent was removed *in vacuo.* The residue was dissolved in ethyl acetate and the solution was washed with 0.3M $KHSO_4$, sat. $NaHCO_3$ and brine, dried over $Na_2SO_4$ and concentrated *in vacuo*. The residue was purified by flash chromatography on silica gel (eluant 30% pet. ether/70% ethyl acetate) to give ethyl 1-methyl-2-(3'-nitro-2'-pyridylamino)pyrazole-4-carboxylate (1.95g, 67%).
**MS** $[M+H]^+$ 292.0

**2B: Ethyl 2-(3'-amino-2'-pyridylamino)-1-methylpyrazole-4-carboxylate**

A solution of ethyl 1-methyl-2-(3'-nitro-2'-pyridylamino)pyrazole-4-carboxylate (1.95g, 6.7mmol) in ethanol (100ml) was hydrogenated over 10% Pd/C catalyst for 3hr. The reaction mixture was filtered through Celite® filter agent and the filtrate was concentrated *in vacuo* to give a white solid identified as ethyl 2-(3'-amino-2'-pyridylamino)-1-methyl-pyrazole-4-carboxylate (1.5g, 86%).

**2C: 1-Methyl-4,10-dihydropyrazolo[4,5-*c*]pyrido[2,3-*b*][1,4]diazepin-4(5*H*)-one**

A solution of ethyl 2-(3'-amino-2'-pyridylamino)-'1-methylpyrazole-4-carboxylate (1.5g, 5.75mmol) in acetic acid/2-propanol (1:9, 50ml) was heated at reflux for 3 days. The solvent was removed *in vacuo* and the residue was azeotroped with toluene. The residue was purified by recrystallization from ethanol and then flash chromatography on silica gel (eluant 95% chloroform/4% methanol/1% acetic acid) to give a white solid identified as 1-methyl-4,10-dihydropyrazolo[4,5-*c*]pyrido[2,3-*b*][1,4]diazepin-4(5*H*)-one (560mg, 45%).

**2D: 1-Methyl-4,10-dihydropyrazolo[4,5-*c*]pyrido[2,3-*b*][1,4]diazepine**

LiAlH4 (365mg, 10mmol) was added portionwise to a suspension of 1-methyl-4,10-dihydropyrazolo[4,5-*c*]pyrido[2,3-*b*][1,4]diazepin-4(5*H*)-one (560mg, 2.6mmol) in anhydrous THF (30ml) at 0°C over 10 minutes. The resulting suspension was heated at reflux for 18hr. The reaction was cooled to 0°C and 35% ammonia solution (1ml) was added dropwise over 10 minutes, then the mixture was stirred at RT for 1 hr. The resulting suspension was filtered through Celite® filter agent and the filtrate was concentrated *in vacuo* to give a white solid identified as 1-methyl-4,10-dihydropyrazolo[4,5-*c*]pyrido[2,3-*b*][1,4]diazepine (410mg, 78%).
**MS** $[M+H]^+$ 202.1.

**Example 3**

***tert*-Butyl *N*-methyl-*N*-(2-picolyl)carbamate**

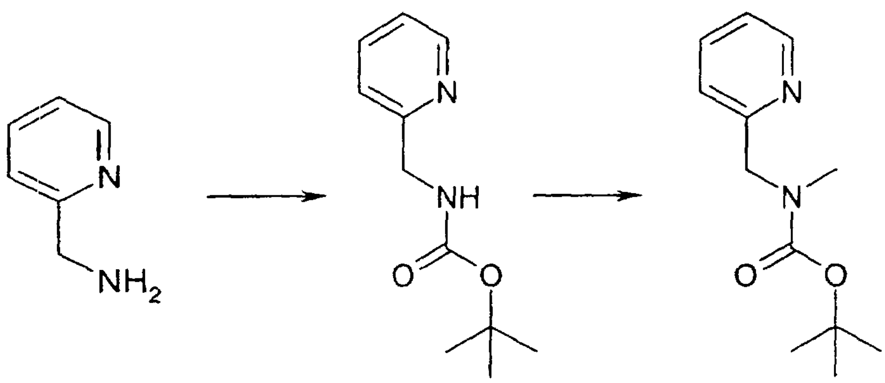

Di-*tert*-butyl dicarbonate (546mg, 2.5mmol) was added to an ice-cold solution of 2-(aminomethyl)pyridine (260mg, 2.4mmol) and triethylamine (340µl, 2.4mmol) in dichloromethane (10ml). The solution was stirred at RT for 2hr and the solvent was removed *in vacuo.* The residue was dissolved in anhydrous THF (10ml) and cooled to 0°C. Sodium hydride (60% dispersion in oil, 200mg, 3mmol) was added, the mixture was stirred for 30 minutes, and then iodomethane (186µl, 3mmol) was added. The resulting suspension was stirred at RT for 18hr. The reaction was quenched with water and the solvent was removed *in vacuo.* The residue was dissolved in ethyl acetate, washed with brine, then dried over $Na_2SO_4$. The solvent was removed *in vacuo* and the residue was purified by flash chromatography on silica gel (eluant 98% chloroform/2% methanol) to give a yellow gum identified as *tert*-butyl *N*-methyl-*N*-(2-picolyl) carbamate (100mg, 19%).

**Example 4**

***tert*-Butyl 4-aminomethyl-3-chlorobenzoate**

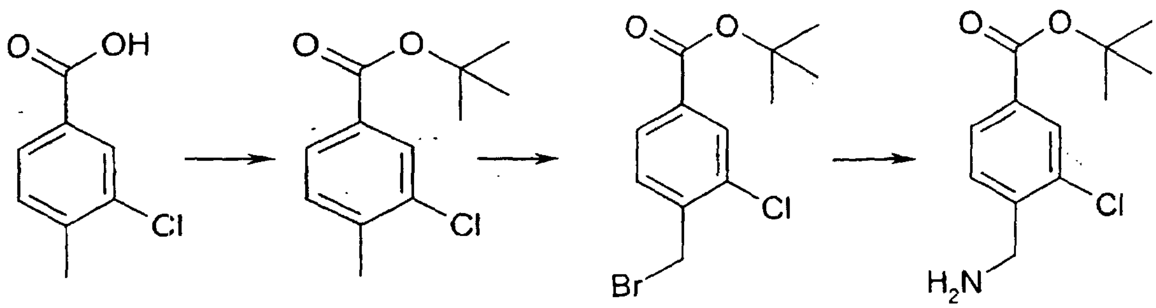

**4A: *tert-Butyl* 3-chloro-4-methylbenzoate**

Thionyl chloride (11ml, 150mmol) was added to a suspension of 3-chloro-4-methyl-benzoic acid (5.12g, 30mmol) in toluene (25ml) and the mixture was heated at reflux for 2hr. The solvent was removed *in vacuo* and the residue was azeotroped with toluene three times, then dissolved in anhydrous THF (40ml) and cooled to 0°C. Lithium *tert*-butoxide (2.4g, 30mmol) was added and the mixture was stirred at RT for 3 days. Water (5ml) was added and the solvent was removed *in vacuo.* The residue was dissolved in ethyl acetate. The solution was washed with 0.3M $KHSO_4$, sat. $NaHCO_3$ and brine, dried over $Na_2SO_4$ and concentrated *in vacuo* to give a pale yellow gum identified as *tert*-butyl 3-chloro-4-methylbenzoate (5.4g, 79%).

**4B: *tert*-Butyl 4-bromomethyl-3-chlorobenzoate**

*N*-Bromosuccinimide (4.27g, 24mmol) and 2,2'-azo-*bis*(2-methylpropionitrile) (394mg, 2.4mmol) were added to a solution of *tert*-butyl 3-chloro-4-methylbenzoate (5.4g, 23.8mmol) in carbon tetrachloride (75ml) and the mixture was heated at reflux for 18hr. The solvent was removed *in vacuo* and the residue was purified by flash chromatography on silica gel (eluant 95% pet.ether/5% ethyl acetate) to give a white solid identified as *tert*-butyl 4-bromomethyl-3-chlorobenzoate (5.7g, 78%).

**4C: *tert-Butyl* 4-aminomethyl-3-chlorobenzoate**

Ethanol (100ml) was saturated with ammonia, then *tert*-butyl 4-bromomethyl-3-chlorobenzoate (5.7g, 18.7mmol) was added and the mixture was stirred at RT for 2hr. The solvent was removed *in vacuo* and the residue was triturated with diethyl ether to give a white solid identified as *tert*-butyl 4-aminomethyl-3-chlorobenzoate (4.1g, 91%).

**Example 5**

**4-(*tert*-Butyloxycarbonylaminomethyl)-3-chlorobenzoic acid**

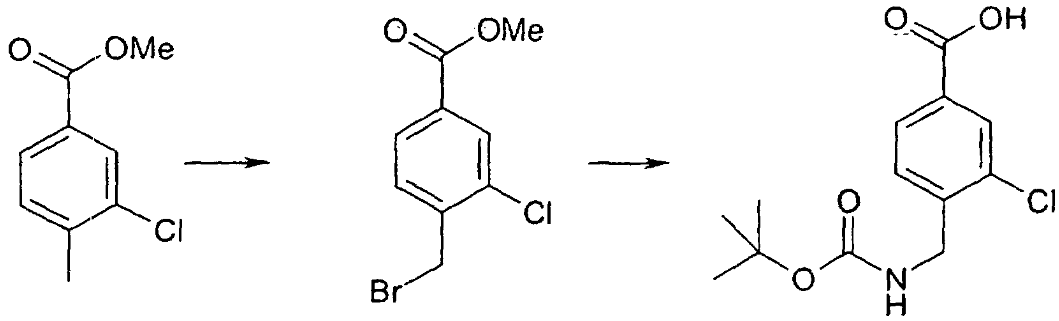

**5A. Methyl 4-bromomethyl-3-chlorobenzoate**

To a solution of methyl 3-chloro-4-methylbenzoate (5.0 g, 27.1 mmol) in carbon tetrachloride (50 ml) were added *N*-bromosuccinimide (5.8g, 32.0 mmol) and 2,2'-azo-*bis*(2-methylpropionitrile) (0.442g, 2.70mmol). The mixture was heated at reflux for 18hr, then allowed to cool to room temperature and concentrated *in vacuo.* The residue was purified by flash chromatography on silica (eluant pet. ether → 5% ethyl acetate/95% pet. ether) to give an oil identified as methyl 4-bromomethyl-3-chlorobenzoate (5.96g, 84%).

**5B. 4-(*tert*-Butyloxycarbonylaminomethyl)-3-chlorobenzoic acid**

To a saturated solution of ammonia in ethanol (170ml) was added methyl 4-bromomethyl-3-chlorobenzoate from Example 5A (5.5g, 20.9mmol). The mixture was stirred at room temperature for 1hr and then concentrated *in vacuo.* The residue was triturated with diethyl ether and the resultant white crystals were filtered off and washed with more diethyl ether. To a solution of this solid in water (100ml) were added solutions of di-*tert*-butyl dicarbonate (5.0g, 23.0mmol) in dioxan (100ml) and sodium hydroxide (1.86g, 46.0mmol) in water (100ml). The mixture was stirred at room temperature for 18hr and then concentrated *in vacuo.* The aqueous residue was acidified with citric acid and extracted with chloroform/2-propanol. The organic layer was washed with water, dried over $MgSO_4$, and concentrated *in vacuo* to give a white solid identified as 4-(*tert*-butyloxycarbonylaminomethyl)-3-chlorobenzoic acid (2.8g, 67%).

**Example 6**

**4-(*tert*-Butyloxycarbonylaminomethyl)-3-nitrobenzoic acid**

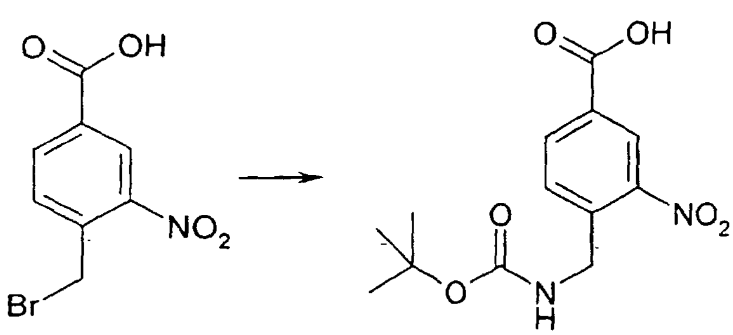

4-Bromomethyl-3-nitrobenzoic acid (4.75g, 18.2mmol) was reacted following the method of Example 5B to give a yellow solid identified as 4-(*tert*-butyloxycarbonylaminomethyl)-3-nitrobenzoic acid (2.6g, 49%).

**Example 7**

**4-Cyano-3-methylbenzoic acid**

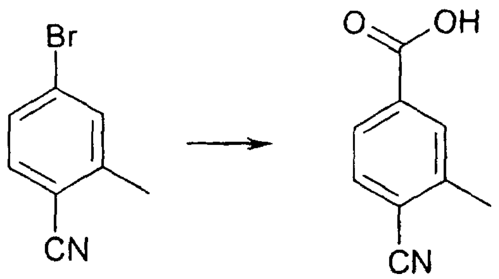

To a solution of 4-bromo-2-methylbenzonitrile (2.0g, 10.2mmol) in THF (100ml) at -78°C under a nitrogen atmosphere was added dropwise a 2.5M solution of *n*-butyl lithium (4.48ml, 11.2mmol). The mixture was stirred at -78°C for 1hr and then poured onto solid carbon dioxide (5g) in THF (50ml). The mixture was allowed to warm to roon temperature. Water was added (200ml) and the mixture was extracted with diethyl ether (3 times). The aqueous layer was acidified by addition of concentrated HCl and extracted with chloroform (3 times). The combined chloroform extracts were washed with water, dried over $MgSO_4$, and concentrated *in vacuo* to give a white solid identified as 4-cyano-3-methylbenzoic acid (1.2g, 73%).

**Example 8**

**4-Cyano-2-methylbenzoic acid**

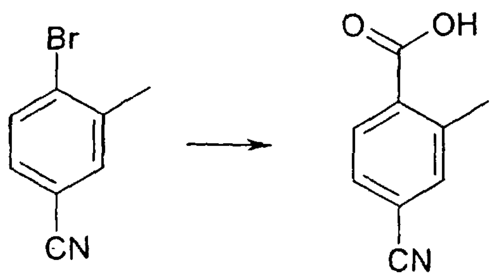

4-Bromo-3-methylbenzonitrile (2.0g, 10.2mmol) was reacted following the method of Example 7. The product was triturated with hexane to give a yellow solid identified as 4-cyano-2-methylbenzoic acid (0.96g, 59%).

**Example 9**

**4-(*tert*-Butyloxycarbonylaminomethyl)-2-fluorobenzoic acid**

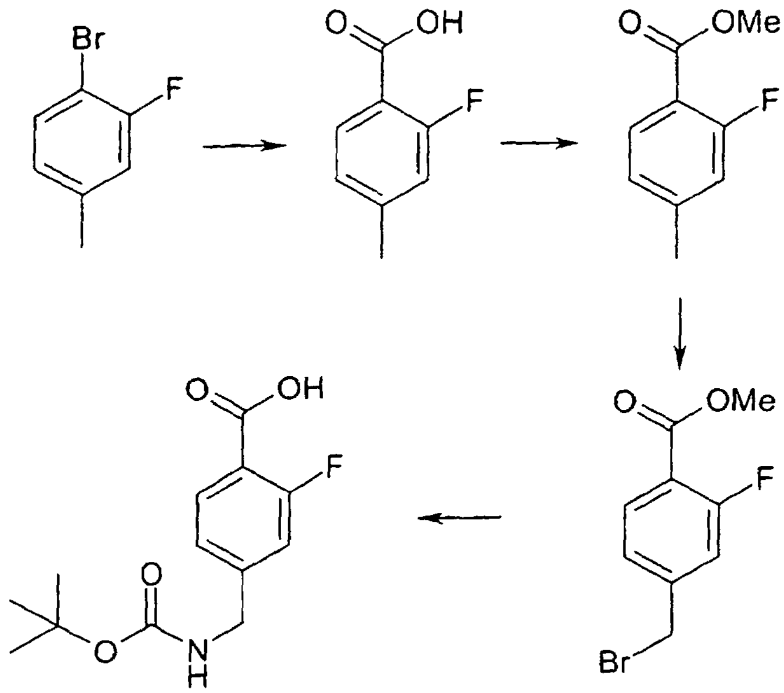

**9A. 2-Fluoro-4-methylbenzoic acid**

4-Bromo-3-fluorotoluene (8.33g, 44.07mmol) was reacted following the method of Example 7 to give a white solid identified as 2-fluoro-4-methylbenzoic acid (4.89g, 72%).

**9B. Methyl 2-fluoro-4-methylbenzoate**

To a solution of 2-fluoro-4-methylbenzoic acid (6.04g, 39.18mmol) in toluene (80ml) was added thionyl chloride (65ml, 89.11mmol). The mixture was heated at reflux for 2.5hr, cooled and concentrated *in vacuo.* The residue was dissolved in dichloromethane (50ml) and methanol (50ml) was added. The mixture was stirred at room temperature for 2.5hr and then concentrated *in vacuo.* The residue was dissolved in dichloromethane (100ml), washed with saturated sodium bicarbonate solution and brine, dried over $MgSO_4$, and concentrated *in vacuo* to give a tan solid identified as methyl 2-fluoro-4-methylbenzoate (5.07g, 77%).

**9C. Methyl 4-bromomethyl-2-fluorobenzoate**

Methyl 2-fluoro-4-methylbenzoate (5.07g, 30.16mmol) was reacted following the method of Example of 5A. The product was purified by flash chromatography on silica (eluant 20% ethyl acetate/ 80% pet. ether) to give an oil identified as methyl 4-bromomethyl-2-fluorobenzoate (5.9g, 80%).

**9D. 4-(*tert*-Butyloxycarbonylaminomethyl)-2-fluorobenzoic acid**

Methyl 4-bromomethyl-2-fluorobenzoate (5.9g, 24.13mmol) was reacted following the method of Example 5B. The product was recrystallised from dioxan/pet. ether to give white crystals identified as 4-(*tert*-butyloxycarbonylaminomethyl)-2-fluorobenzoic acid (2.46g, 38%).

**Example 10**

**4-Cyano-3,5-dimethylbenzoic acid**

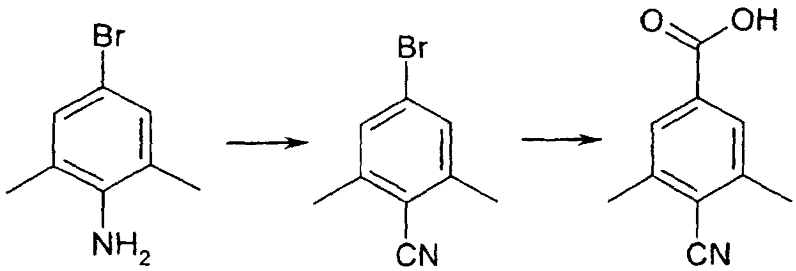

**10A. 4-Bromo-2,6-dimethylbenzonitrile**

4-Bromo-2,6-dimethylaniline (4.49g, 22.4mmol) was taken up in water (25ml) and concentrated hydrochloric acid (8.0ml) was added. The mixture was sonicated to form a fine suspension and then cooled to 0°C. A solution of sodium nitrite (1.67g, 24.2mmol) in water (5ml) was then added dropwise so as to maintain the temperature of the reaction between 0-5°C. The mixture was stirred at 0-5°C for 30 minutes and then neutralised by addition of solid sodium bicarbonate. The resulting solution was then added portionwise to a solution of copper cyanide (2.42g, 27.0mmol) and potassium cyanide (3.65g, 56.1 mmol) in water (25ml) at 70°C. The mixture was stirred at 70°C for 30 minutes, allowed to cool and then extracted with toluene (2 times). The combined extracts were washed with water and brine, dried over $MgSO_4$, and concentrated *in vacuo*. The residue was purified by flash chromatography on silica (eluant 5% ethyl acetate/ 95% pet. ether) to give an orange solid identified as 4-bromo-2,6-dimethylbenzonitrile (3.2g, 68%).

**10B. 4-Cyano-3,5-dimethylbenzoic acid**

4-Bromo-2,6-dimethylbenzonitrile (3.20g, 15.2mmol) was reacted following the method of Example 7 to give a tan solid identified as 4-cyano-3,5-dimethylbenzoic acid (1.5g, 56%).

**Example 11**

**4-Methyl-1-(*N*-(2-methyl-4-(2,3,4,5-tetrahydro-1,5-benzodiazepin-4-on-1-ylcarbonyl)benzylcarbamoyl)-L-thioprolyl)perhydro-1,4-diazepine**

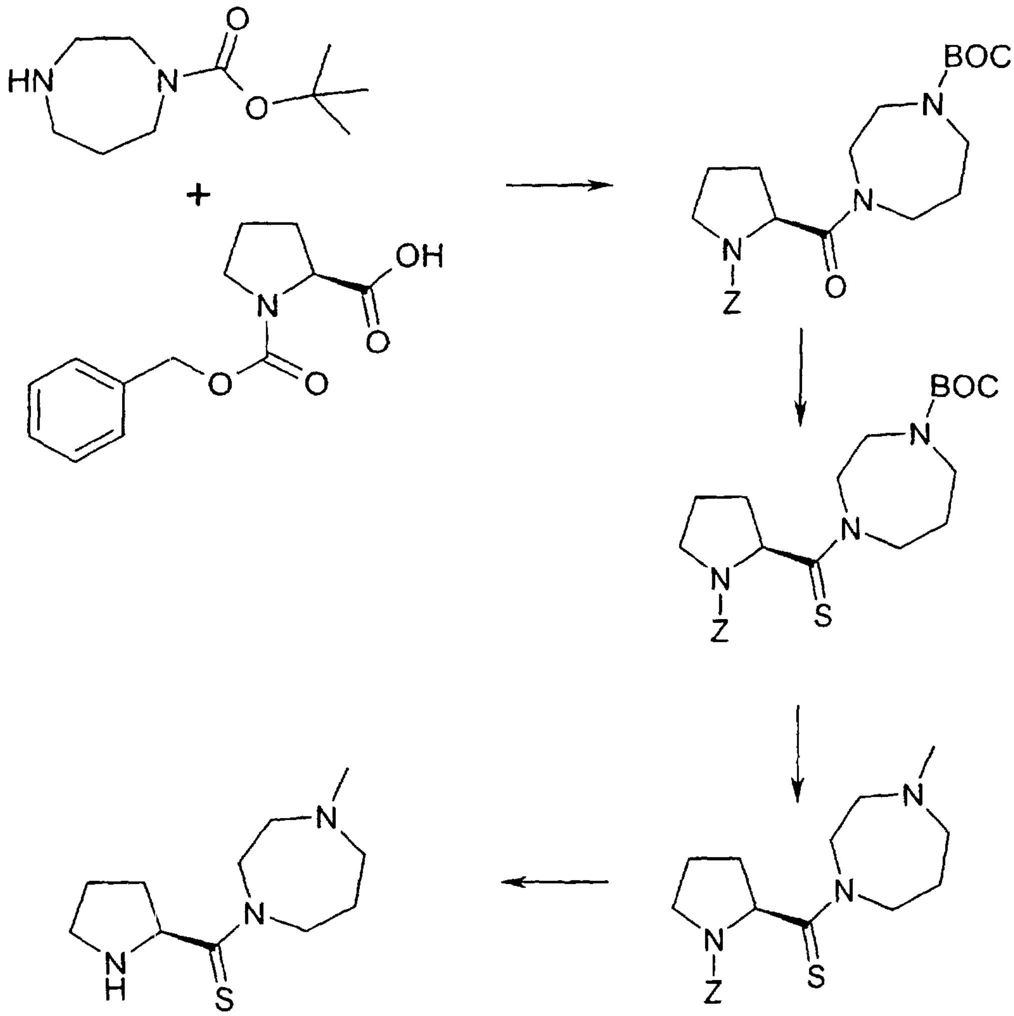

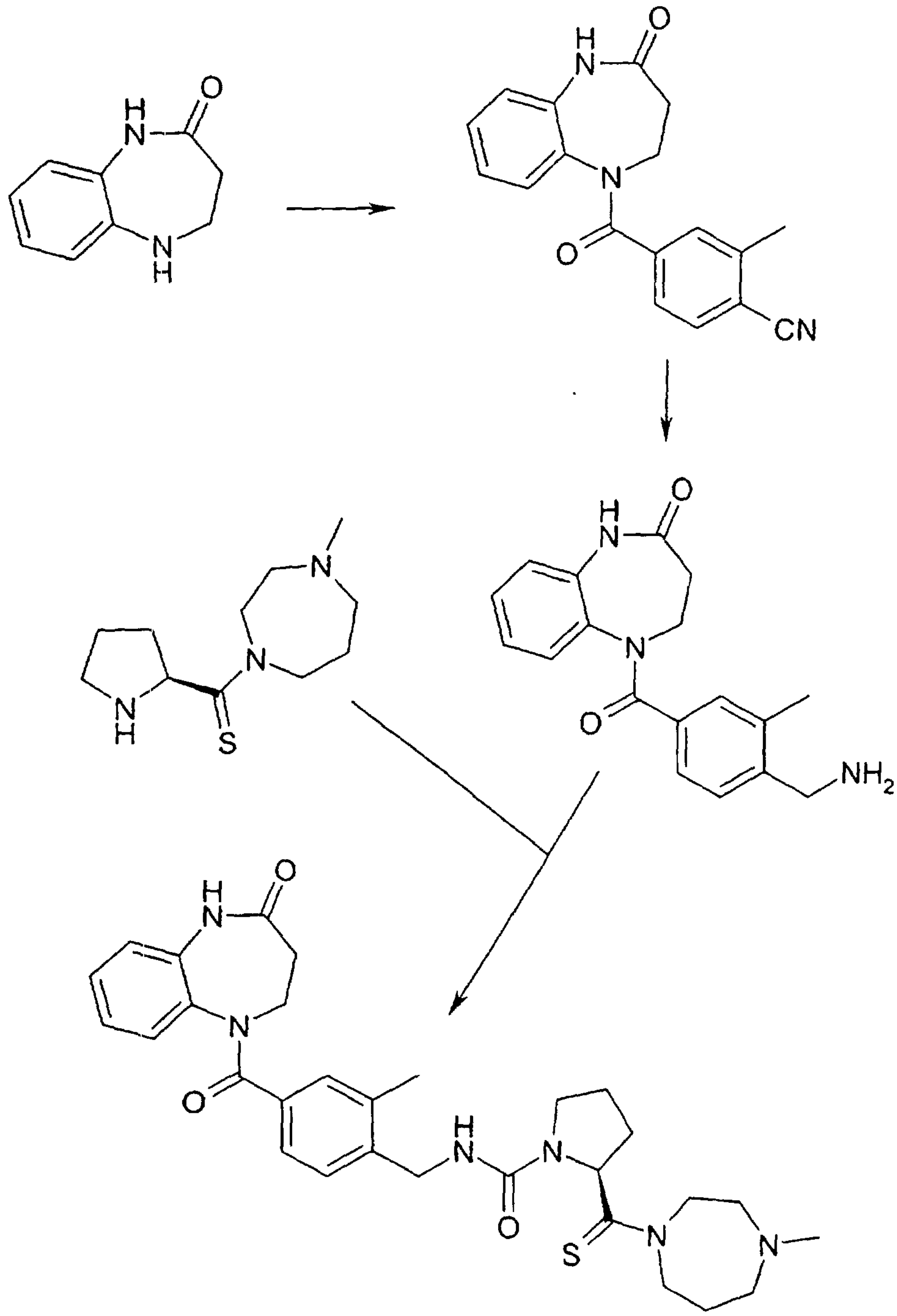

**11A: 1-(*N*-Benzyloxycarbonyl-L-prolyl)-4-(tert-butyloxycarbonyl)perhydro-1,4-diazepine**

HOBt (3.04g, 20mmol), WSCD (5.3g, 26mmol) and N-benzyloxycarbonyl-L-proline (4.7g, 18.8mmol) were added to a solution of *tert*-butyl homopiperazine-1-carboxytate (3.8g, 18.8mmol) and triethylamine (5.4ml, 37.6mmol) in dichloromethane (100ml) at 0°C. The resulting solution was stirred at RT for 18hr. The solvent was removed *in vacuo* and the residue was dissolved in ethyl acetate. The solution was washed with sat. $NaHCO_3$ and brine, dried over sodium sulphate and concentrated *in vacuo* to give a pale yellow gum identified as 1-(*N*-benzyloxycarbonyl-L-prolyl)-4-(*tert*-butyloxycarbonyl)perhydro-1,4-diazepine (8.1g, 100%).

**11B: 1-(*N*-Benzyloxycarbonyl-L-thioprolyl)-4-(*tert*-butyloxycarbonyl)perhydro-1,4-diazepine**

Lawesson's reagent (6.79g, 16.8mmol) was added to a solution of 1-(*N*-benzyloxycarbonyl-L-prolyl)-4-(*tert*-

butyloxycarbonyl)perhydro-1,4-diazepine (12g, 28mmol) in toluene (200ml) and stirred at 90°C for 1hr then 85°C for 18hr. The solvent was removed *in vacuo* and the residue was purified by flash chromatography on silica gel (eluant 98% chloroform/2% methanol) to give 1-(*N*-benzyloxycarbonyl-L-thioprolyl)-4-(*tert*-butyloxycarbonyl)perhydro-1,4-diazepine (8.4g, 67%).
**MS** $[M+H]^+$ 447.3

**11C: 1-(*N*-Benzyloxycarbonyl-L-thioprolyl)-4-methylperhydro-1,4-diazepine**

A solution of 1-(*N*-benzyloxycarbonyl-L-thioprolyl)-4-(*tert*-butyloxycarbonyl)perhydro-1,4-diazepine (8.4g, 18.1 mmol) in 4N HCl/dioxan (20ml) was stirred at RT for 35min. The solvent was removed *in vacuo* and the residue was azeotroped with toluene. The resulting gum was dissolved in acetic acid/methanol (1:9, 100ml) and stirred at 0°C. Formaldehyde (20% solution, 8.4ml, 56mmol) was added, the solution was stirred for 20min, then sodium cyanoborohydride (2.35g, 37mmol) was added and the solution was stirred at RT for 18hr. The solvent was removed *in vacuo* and the residue was purified by flash chromatography on silica gel (eluant 96% chloroform/3% methanol/1% triethylamine) to give a colourless gum identified as (6.56g, 97%).
**MS** $[M+H]^+$ 362.2

**11D: 4-Methyl-1-L-thioprolylperhydro-1,4-diazepine**

1-(*N*-Benzyloxycarbonyl-L-thioprolyl)-4-methylperhydro-1,4-diazepine (3g, 8.3mmol) was dissolved in ice-cold 30% HBr/acetic acid (20ml). The resulting brown solution was stirred at RT for 3hr, the solvent was removed *in vacuo* and the residue was azeotroped with toluene. Sat. $NaHCO_3$ (100ml) was added until the solution showed a pH>8. The solution was washed with diethyl ether (2 × 40ml) then evaporated *in vacuo* and azeotroped with toluene. The resulting solid was extracted with hot chloroform (3 × 100ml) and the solvent was removed *in vacuo* to yield a brown gum identified as 4-methyl-1-L-thioprolylperhydro-1,4-diazepine (1.72g, 91%).
**MS** $[M+H]^+$ 228.1

**11E: 1-(4-Cyano-3-methylbenzoyl)-2,3,4,5-tetrahydro-1,5-benzodiazepin-4-one**

Thionyl chloride (0.4ml, 6.0mmol) was added to a suspension of 4-cyano-3-methylbenzoic acid (322mg, 2.0mmol) in toluene (10ml) and the mixture was heated at reflux for 90min, then cooled to RT and concentrated *in vacuo.* The residue was azeotroped with toluene then dissolved in dichloromethane (5ml) and added to a suspension of 2,3,4,5-tetrahydro-1,5-benzodiazepin-2-one (324mg, 2.mmol) and triethylamine (280µl, 2.0mmol) in dichloromethane (3ml). The mixture was stirred overnight at RT then concentrated *in vacuo.* The residue was partitioned between dichloromethane and 0.3M $KHSO_4$. The organic phase was washed with sat. $NaHCO_3$ and brine, dried over $Na_2SO_4$ and concentrated *in vacuo* to give a red solid identified as 1-(4-cyano-3-methylbenzoyl)-2,3,4,5-tetrahydro-1,5-benzodiazepin-4-one (600mg, 98%).

**11F: 1-(4-Aminomethyl-3-methylbenzoyl)-2,3,4,5-tetrahydro-1,5-benzodiazepin-4-one**

Sodium borohydride (734mg, 19.3mmol) was added portionwise to an ice-cold suspension of 1-(4-cyano-3-methylbenzoyl)-2,3,4,5-tetrahydro-1,5-benzodiazepin-4-one (590mg, 1.93mmol) and $CoCl_2.6H_2O$ (920mg, 3.86mmol) in methanol (10ml). The mixture was stirred at RT for 1hr, then quenched with 1M HCl and concentrated *in vacuo.* The aqueous residue was diluted with 1M HCl (50ml) and filtered through Celite® filter agent. The filtrate was washed with diethyl ether (2 × 50ml) then basified with 4M NaOH and extracted with chloroform. (3 × 50ml). The combined organic phases were dried over $Na_2SO_4$ and concentrated *in vacuo* to give a pale yellow solid identified as 1-(4-aminomethyl-3-methylbenzoyl)-2,3,4,5-tetrahydro-1,5-benzodiazepin-4-one (300mg, 50%).

**11G: 4-Methyl-1-(*N*-(2-methyl-4-(2,3,4,5-tetrahydro-1,5-benzodiazepin-4-on-1-ylcarbonyl)benzylcarbamoyl)-L-thioprolyl)perhydro-1,4-diazepine**

1,1'-Carbonyldiimidazole (18mg, 0.11mmol) was added to a solution of 1-(4-aminomethyl-3-methylbenzoyl)-2,3,4,5-tetrahydro-1,5-benzodiazepin-4-one (31mg, 0.1mmol) and DIEA (54µl, 0.3mmol) in DMF (2ml) under nitrogen gas and the solution was stirred at RT for 1hr. A solution of 4-methyl-1-L-thioprolylperhydro-1,4-diazepine (22.7mg, 0.1mmol) and DIEA (54µl, 0.3mmol) in DMF (2ml) was added and the solution was stirred at RT for 18hr under nitrogen gas. The solvent was removed *in vacuo* and the residue was purified by flash chromatography on silica gel (eluant 97% chloroform/2% methanol/1% triethylamine) to give a white solid identified as 4-methyl-1-(*N*-(2-methyl-4-(2,3,4,5-tetrahydro-1,5-benzodiazepin-4-on-1-ylcarbonyl)benzylcarbamoyl)-L-thioprolyl)perhydro-1,4-diazepine

(17mg, 30%).
**MS** [M+H]+ 563.2

**Example 12**

**4-Methyl-1-(*N*-(2-methyl-4-(1-methyl-4,10-dihydropyrazolo[5,4-*b*][1,5]benzodiazepin-5-ylcarbonyl) benzylcarbamoyl)-L-thioprolyl)perhydro-1,4-diazepine**

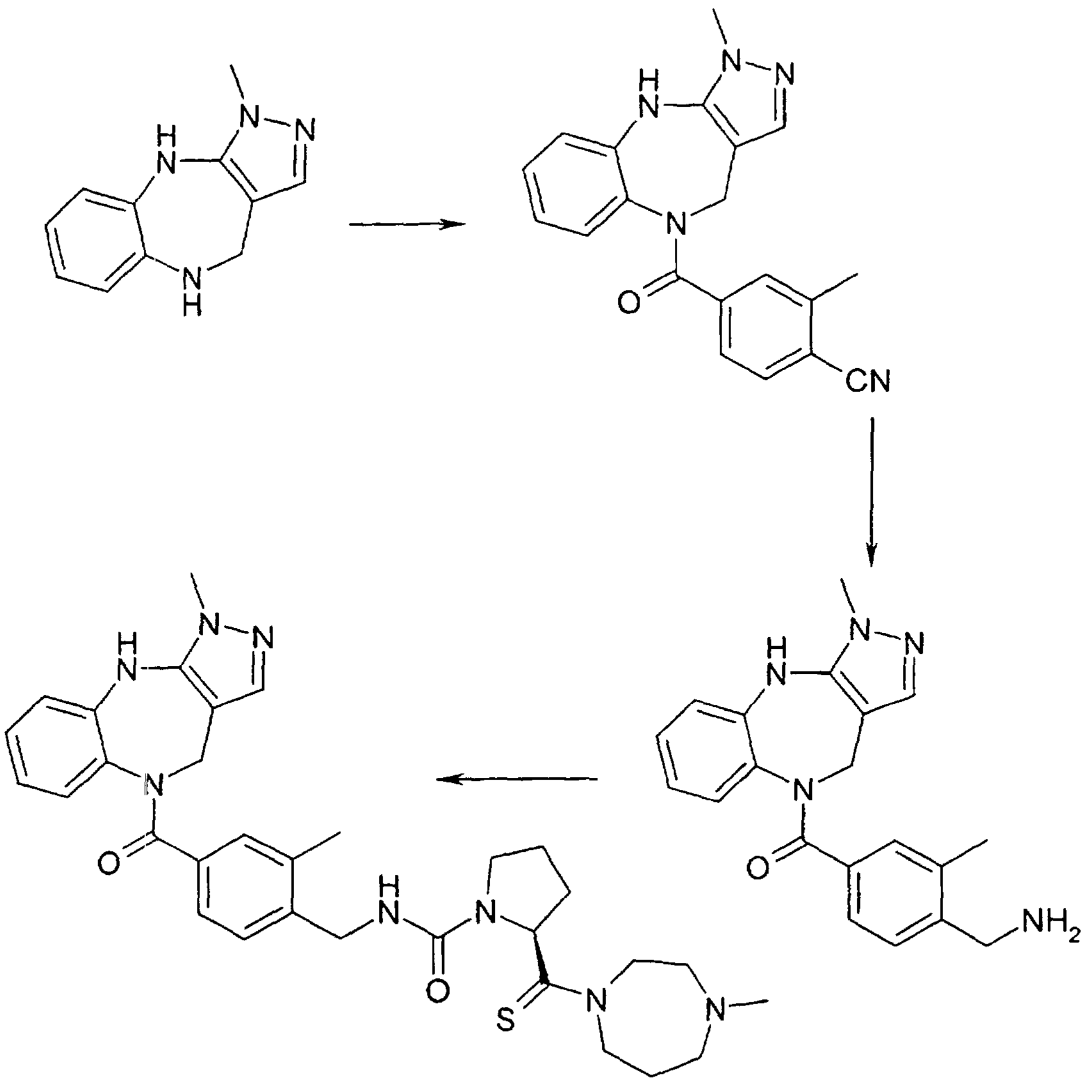

**12A: 5-(4-Cyano-3-methylbenzoyl)-1-methyl-4,10-dihydropyrazolo[5,4-*b*][1,5]-benzodiazepine**

Thionyl chloride (1.8ml, 27mmol) was added to a suspension of 4-cyano-3-methylbenzoic acid (1.29g, 8.0mmol) in toluene (25ml) and the mixture was heated at reflux for 2hr, then cooled to RT and concentrated *in vacuo.* The residue was azeotroped with toluene then dissolved in dichloromethane (10ml) and added to a suspension of 1-methyl-4,10-dihydropyrazolo[5,4-*b*][1,5]benzodiazepine (1.6g, 8mmol) and triethylamine (1.4ml, 10mmol) in dichloromethane (15ml). The mixture was stirred overnight at RT then concentrated *in vacuo.* The residue was partitioned between chloroform and 0.3M $KHSO_4$. The aqueous phase was extracted with chloroform/2-propanol (80:20). The combined organic phases were washed with sat. $NaHCO_3$ and brine, dried over $Na_2SO_4$ and concentrated *in vacuo.* The residue was purified by flash chromatography on silica gel (eluant 95%chloroform/5% methanol) to give a pale yellow solid identified as 5-(4-cyano-3-methylbenzoyl)-1-methyl-4,10-dihydropyrazoto[5,4-*b*][1,5]benzodiazepine

(2.4g, 87%).

**12B: 5-(4-Aminomethyl-3-methylbenzoyl)-1-methyl-4,10-dihydropyrazolo[5,4-*b*][1,5]-benzodiazepine**

Sodium borohydride (1.27g, 33.5 mmol) was added portionwise to an ice-cold suspension of 5-(4-cyano-3-methylbenzoyl)-1-methyl-4,10-dihydropyrazolo[5,4-*b*][1,5]benzodiazepine (1.15g, 3.35mmol) and $CoCl_2.6H_2O$ (1.59g, 6.7mmol) in methanol (35ml). The mixture was stirred at RT for 1hr, then quenched with 1M $KHSO_4$ and concentrated *in vacuo.* The aqueous residue was diluted with 1M $KHSO_4$ (40ml) and filtered through Celite® filter agent. The filtrate was washed with diethyl ether (2 × 50ml) then basified with 2M NaOH and extracted with chloroform. The organic phase was dried over $Na_2SO_4$ and concentrated *in vacuo* to give a pale brown solid identified as 5-(4-aminomethyl-3-methylbenzoyl)-1-methyl-4,10-dihydropyrazolo[5,4-*b*][1,5]benzodiazepine (745mg, 64%).

**12C: 4-Methyl-1-(*N*-(2-methyl-4-(1-methyl-4,10-dihydropyrazolo[5,4-*b*][1,5]-benzodiazepin-5-ylcarbonyl) benzylcarbamoyl)-L-thioprolyl)perhydro-1,4-diazepine**

1,1'-Carbonyldiimidazole (267mg, 1.65mmol) was added to a solution of 5-(4-aminomethyl-3-methylbenzoyl)-1-methyl-4,10-dihydropyrazolo[5,4-*b*][1,5]benzodiazepine (520mg, 1.5mmol) and DIEA (276µl, 1.5mmol) in DMF (10ml) under nitrogen gas and the solution was stirred at RT for 1hr. A solution of 4-methyl-1-L-thioprolylperhydro-1,4-diazepine (374mg, 1.65mmol) and DIEA (276µl, 1.5mmol) in DMF (10ml) was added and the resulting solution was-stirred-at RT for 18hr under nitrogen gas. The solvent was removed *in vacuo* and the residue was purified by flash chromatography on silica gel (eluant 96% chloroform/3% methanol/1% triethylamine then 92% chloroform/6% methanol/2% triethylamine) to yield a cream-coloured solid identified as 4-methyl-1-(*N*-(2-methyl-4-(1-methyl-4,10-dihydropyrazolo[5,4-*b*][1,5]benzodiazepin-5-ylcarbonyl)benzylcarbamoyl)-L-thioprolyl)perhydro-1,4-diazepine (568mg, 63%).
**NMR** (270MHz, $CDCl_3$) δ 1.91-2.77 (18H,m), 3.33-3.43 (2H,m), 3.62-3.64 (1H,m), 3.73 (3H,s), 3.75-3.95 (3H,m), 4.11-4.29 (2H,m), 4.55-4.57 (1H,m), 5.05-5.06 (1H,m). 5.85-5.91 (1H,m), 6.65-6.77 (3H,m), 6.94-6.98 (4H,m), 7.21-7.24 (1H,m) ppm.
**MS** $[M+H]^+$ 600.7

**Example 13**

**4,4-Dimethyl-1-(*N*-(2-methyl-4-(1-methyl-4,10-dihydropyrazolo[5,4-*b*][1,5]-benzodiazepin-5-ylcarbonyl) benzylcarbamoyl)-L-thioprolyl)perhydro-1,4-diazepinium iodide**

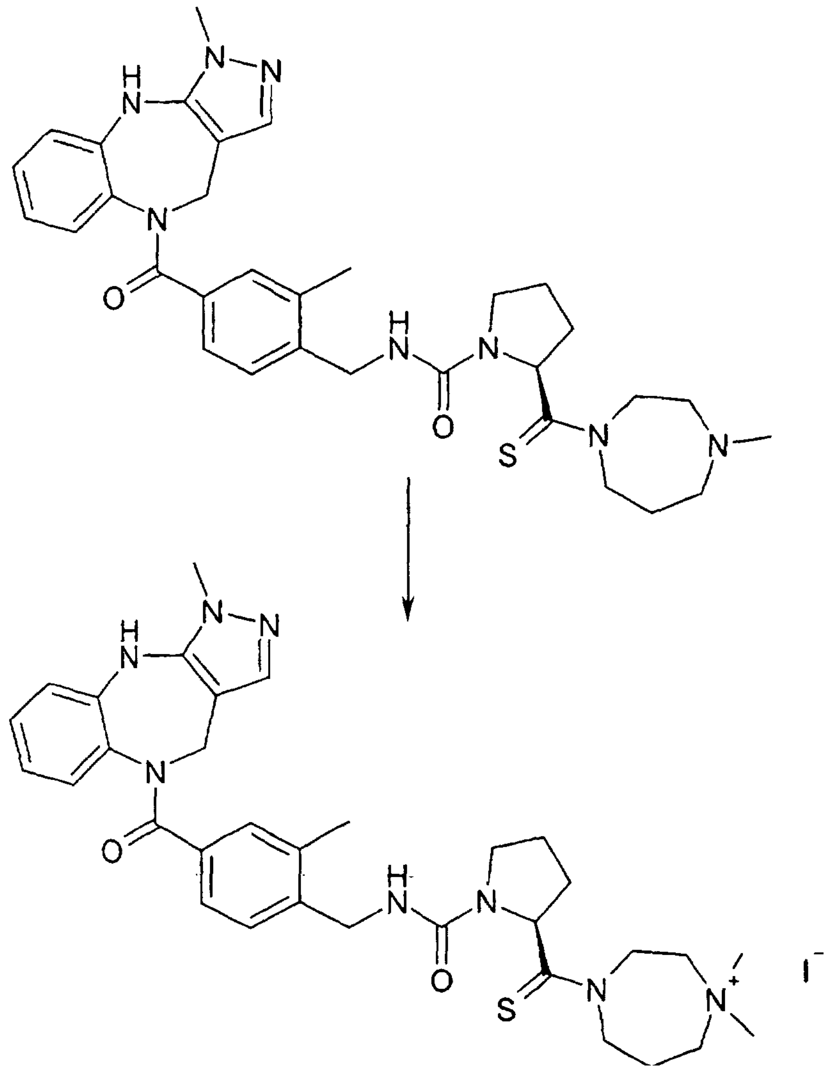

Iodomethane (13.9mg, 6.1μl, 0.098mmol) was added to a solution of 4-methyl-1-(*N*-(2-methyl-4-(1-methyl-4,10-dihydropyrazolo[5,4-*b*][1,5]benzodiazepin-5-ylcarbonyl)benzylcarbamoyl)-L-thioprolyl)perhydro-1,4-diazepine (30mg, 0.049mmol) in THF (10ml) and the mixture was stirred at RT for 4hr. The resulting solid was collected and dissolved in water (5ml). The solution was washed with ethyl acetate (2 × 10ml) and lyophilized to give a white solid identified as 4,4-dimethyl-1-(*N*-(2-methyl-4-(1-methyl-4,10-dihydropyrazolo[5,4-*b*][1,5]benzodiazepin-5-ylcarbonyl) benzylcarbamoyl)-L-thioprolyl)perhydro-1,4-diazepinium iodide (29mg, 79.6%).
**MS** $[M]^+$ 615.3

**Example 14**

**4-Methyl-1-(*N*-(2-methyl-4-(5,6,7,8-tetrahydrothieno[3,2-*b*]azepin-4-ylcarbonyl)-benzylcarbamoyl)-L-thioprolyl)perhydro-1,4-diazepine**

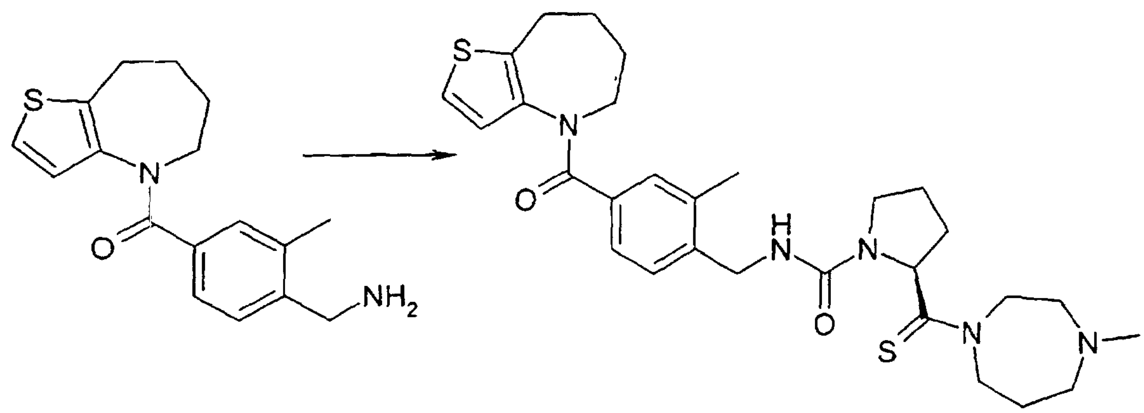

1,1'-Carbonyldiimidazole (20mg, 0.12 mmol) was added to a solution of 4-(4-aminomethyl-3-methylbenzoyl)-5,6,7,8-tetrahydrothieno[3,2-*b*]azepine (36mg, 0.1mmol) and DIEA (50µl, 0.3mmol) in DMF (10ml) under nitrogen gas and the solution was stirred at RT for 1hr. A solution of 4-methyl-1-L-thioprolylperhydro-1,4-diazepine (34mg, 0.15mmol) and DIEA (50µl, 0.3mmol) in DMF (10 ml) was added and the resulting solution was stirred at RT for 18hr under nitrogen gas. The solvent was removed *in vacuo* and the residue was purified by flash chromatography on silica gel (eluant 96% chloroform/3% methanol/1% triethylamine) and then by semi-preparative HPLC (Vydac $C_{18}$ column; 30% → 80% 0.1%TFA/acetonitrile in 0.1%TFA/water over 40min at 6 ml/min). Product-containing fractions were pooled and lyophilized to give a white solid identified as 4-methyl-1-(*N*-(2-methyl-4-(5,6,7,8-tetrahydrothieno[3,2-*b*]azepin-4-ylcarbonyl)benzylcarbamoyl)-L-thioprolyl)perhydro-1,4-diazepine trifluoroacetate (17mg, 25%).
**NMR** (270 MHz, $CD_3OD$) δ 1.79-2.32 (10H,m), 2.71-2.99 (5H,m), 3.40-3.91 (10H,m), 4.26-4.53 (3H,m), 4.86-5.10 (2H, m), 6.27-6.28 (1H,m), 6.79-6.81 (1H,m), 7.13-7.41 (3H,m) ppm.
**MS** $[M+H]^+$ 574.2

**Example 15**

**4-Methyl-1-(*N*-(2-methyl-4-(5,6,7,8-tetrahydrothieno[3,2-*b*]azepin-4-ylcarbonyl)-benzyloxycarbonyl)-L-prolyl) perhydro-1,4-diazepine**

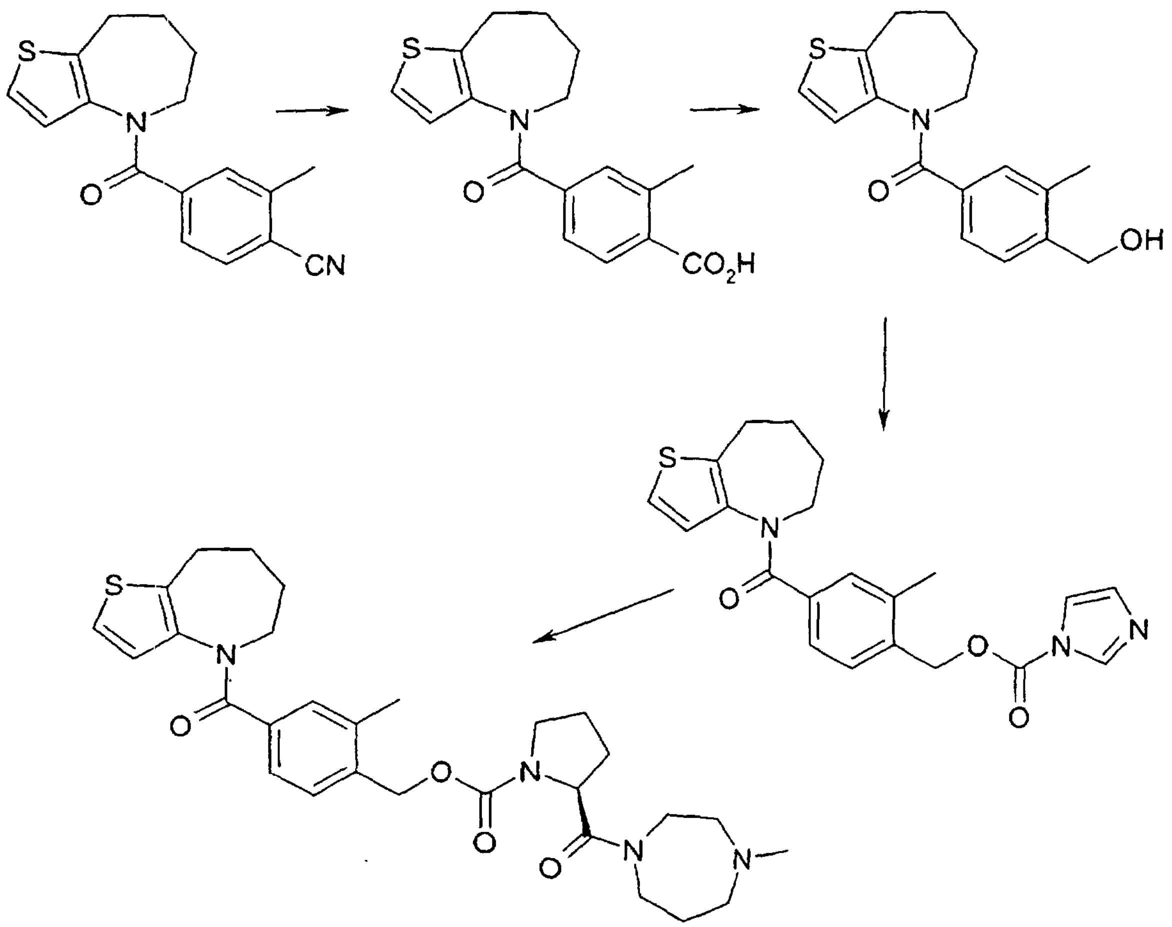

**15A: 4-(4-Carboxy-3-methylbenzoyl)-5,6,7,8-tetrahydrothieno[3,2-b]azepine**

A suspension of 4-(4-cyano-3-methylbenzoyl)-5,6,7,8-tetrahydrothieno[3,2-*b*]azepine (1g, 3.3mmol) in conc. sulphuric acid/water (1:1, 30ml) was heated at reflux for 5hr. The resulting solution was cooled to RT, diluted with water (20ml) and extracted with chloroform (3 × 20ml). The combined organic phases were extracted with sat. $NaHCO_3$ (2 × 20ml). The combined aqueous extracts were acidified with 1M $KHSO_4$ and extracted with chloroform (3 × 20ml). These chloroform extracts were combined, washed with brine, dried over $Na_2SO_4$ and concentrated *in vacuo* to give a pale brown solid identified as 4-(4-carboxy-3-methylbenzoyl)-5,6,7,8-tetrahydrothieno[3,2-*b*]azepine. (225mg, 23%).

**15B: 4-(4-hydroxymethyl-3-methylbenzoyl)-5,6,7,8-tetrahydrothieno[3,2-*b*]azepine**

Isobutyl chloroformate (250µl, 2mmol) was added to a solution of 4-(4-carboxy-3-methylbenzoyl)-5,6,7,8-tetrahydrothieno[3,2-*b*]azepine (470mg, 1.48mmol) and *N*-methylmorpholine (230µl, 2.1mmol) in THF (15ml) at 0°C and the mixture was stirred for 1hr. The resultant suspension was filtered and the filtrate was added to a solution of sodium borohydride (131mg, 3.45mmol) in water (15ml) at 0°C. The solution was stirred at RT for 2hr, then sat. $NH_4Cl$ (5ml) was added and the THF was removed *in vacuo.* The remaining solution was diluted with water and extracted with chloroform (3 × 20ml). The combined organic phases were washed with brine, dried over $Na_2SO_4$ and concentrated *in vacuo* to give a pale brown solid identified as 4-(4-hydroxymethyl-3-methylbenzoyl)-5,6,7,8-tetrahydrothieno[3,2-*b*] azepine (330mg, 74%).

**15C: 4-(4-(1-Imidazolecarbonyloxymethyl)-3-methylbenzoyl)-5,6,7,8-tetrahydrothieno[3,2-*b*]azepine**

1,1'-Carbonyldiimidazole (36mg, 0.22mmol) was added to a solution of 4-(4-hydroxymethyl-3-methylbenzoyl)-5,6,7,8-tetrahydrothieno[3,2-*b*]azepine (60mg, 0.17mmol) in DMF (2ml) under nitrogen gas and the solution was stirred at RT for 18hr. The solvent was removed *in vacuo* and the residue was purified by flash chromatography on silica gel (eluant 97% chloroform/3% methanol) to give a colourless gum identified as 4-(4-(1-imidazolecarbonyloxymethyl)-3-methylbenzoyl)-5,6,7,8-tetrahydrothieno[3,2-*b*]azepine (60mg, 45%).

**15D: 4-Methyl-1-(*N*-(2-methyl-4-(5,6,7,8-tetrahydrothieno[3,2-*b*]azepin-4-ylcarbonyl)-benzyloxycarbonyl)-L-prolyl)perhydro-1,4-diazepine**

A solution of 4-(4-(1-imidazolecarbonyloxymethyl)-3-methylbenzoyl)-5,6,7,8-tetrahydrothieno[3,2-*b*]azepine (27.24mg, 0.12mmol), 4-methyl-1-L-prolylperhydro-1,4-diazepine (47mg, 0.12mmol) and DIEA (45μl, 0.125mmol) in ethyl acetate (20ml) was heated at reflux for 48 Hrs. The solvent was removed *in vacuo* and the residue was purified by flash chromatography on silica gel (eluant 96% chloroform/3% methanol/1% triethylamine) then by semi-preparative HPLC (Vydac $C_{18}$ column; 25% → 65% 0.1%TFA/acetonitrile in 0.1%TFA/water over 40min at 6ml/min). Product-containing fractions were pooled and lyophilized to give a white solid identified as 4-methyl-1-(*N*-(2-methyl-4-(5,6,7,8-tetrahydrothieno[3,2-*b*]azepin-4-ylcarbonyl)benzyloxycarbonyl)-L-prolyl)perhydro-1,4-diazepine trifluoroacetate (11mg, 13%).
**NMR** (270 MHz, $CD_3OD$) δ 1.80-2.46 (15H,m), 2.92-2.98 (5H,m), 3.29-3.79 (11H,m), 4.07-4.15 (1H,m), 4.84-5.13 (3H, m), 6.21-6.25 (1H,m), 6.75-6.78 (1H,m), 7.02-7.21 (3H,m) ppm.
**MS** $[M+H]^+$ 585.3.

**Example 16**

**(4*R)*-*N*α-(2-Chloro-4-(5,6,7,8-tetrahydrothieno[3,2-*b*]azepin-4-ylcarbonyl)-benzylcarbamoyl)-4-methoxy-L-protine-*N*-methyl-*N*-(2-picolyl)amide**

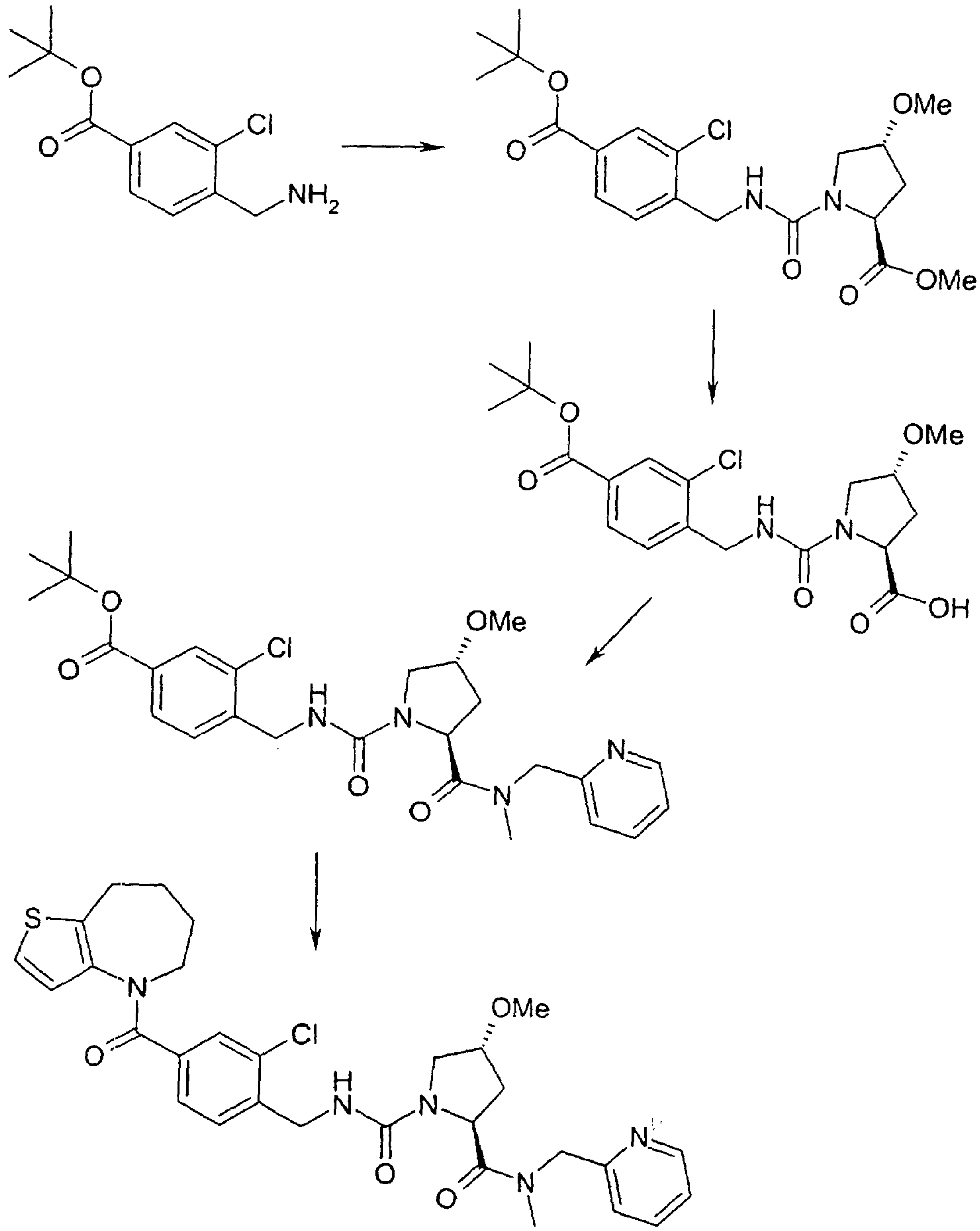

**16A: (4*R*)-*N*α-(4-(tert-Butytoxycarbonyl)-2-chlorobenzylcarbamoyl)-4-methoxy-L-proline methyl ester**

1,1'-Carbonyldiimidazole (800mg, 4.8mmol) was added to a solution of *tert*-butyl 4-(aminomethyl)-3-chlorobenzoate (967mg, 4mmol) and DIEA (1ml, 6mmol) in DMF (30ml) under nitrogen gas and the solution was stirred at RT for 1 hr. A solution of (4*R*)-4-methoxy-L-proline methyl ester (663mg, 4.17mmol) in DMF (10ml) was added and the solution was stirred at RT for 18hr. The solvent was removed *in vacuo* and the residue was dissolved in ethyl acetate. The solution was washed with 0.3M $KHSO_4$, sat. $NaHCO_3$ and brine, dried over $Na_2SO_4$, and concentrated *in vacuo.* The residue was purified by flash chromatography on silica gel (eluant 80% pet.ether/20% ethyl acetate) to give a white sold identified as (940mg, 55%).

**16B: (4R)-*N*$^{\alpha}$-(4-(tert-Butyloxycarbonyl)-2-chlorobenzylcarbamoyl)-4-methoxy-L-proline**

Lithium hydroxide (139mg, 3.3mmol) was added to a solution of (4*R*)-*N*$^{\alpha}$-(4-(*tert*-butyloxycarbonyl)-2-chlorobenzylcarbamoyl)-4-methoxy-L-proline methyl ester (930mg, 2.18mg) in dioxan/water (1:1, 30ml) and the mixture was stirred at RT for 1hr. The solvent was removed *in vacuo* and the residue was partitioned between ethyl acetate and 0.3M $KHSO_4$. The aqueous layer was extracted with ethyl acetate (2 × 30ml). The organic phases were combined and washed with brine, dried over $Na_2SO_4$ and concentrated *in vacuo* to give a white solid identified as (4*R*)-*N*$^{\alpha}$-(4-(*tert*-butyloxycarbonyl)-2-chlorobenzylcarbamoyl)-4-methoxy-L-proline (890mg, 99%).

**16C: (4*R)-N*$^{\alpha}$-(4-(*tert*-Butyloxycarbonyl)-2-chlorobenzylcarbamoyl)-4-methoxy-L-proline-*N*-methyl-N-(2-picolyl)amide**

*tert*-Butyl *N*-methyl-*N*-(2-picolyl)carbamate (100mg, 0.45mmol) was dissolved in 4N HCl/dioxan (10ml) and the solution was stirred at RT for 1hr. The solvent was removed *in vacuo* and the residue was azeotroped with toluene. The resulting gum was dissolved in dichloromethane (5ml) and triethylamine (140µl, 1mmol) was added to give a solution of *N*-methyl-2-picolylamine.

In a second flask, HOBt (107mg, 0.7mmol) and WSCD (106mg, 0.55mmol) were added to a solution of (4*R*) -*N*$^{\alpha}$-(4-(*tert*-butyloxycarbonyl)-2-chlorobenzylcarbamoyl)-4-methoxy-L-proline (185mg, 0.45mmol) in dichloromethane (10ml) at 0°C. The resulting solution was allowed to warm to RT and stirred for 30min. The solution of *N*-methyl-2-picolylamine was added to this solution and the reaction mixture was stirred at RT for 18hr. The solvent was removed *in vacuo* and the residue was dissolved in ethyl acetate. The solution was washed with 0.3M $KHSO_4$, sat. $NaHCO_3$ and brine, dried over $Na_2SO_4$ and concentrated *in vacuo.* The residue was purified by flash chromatography on silica gel (eluant 96% chloroform/4% methanol) to give a white gum identified as (4*R*)-N$^{\alpha}$-(4-(tert-butyloxycarbonyl)-2-chlorobenzylcarbamoyl)-4-methoxy-L-proline-*N*-methyl-*N*-(2-picolyl)amide (170mg, 73%).

**16D: (4*R)-N*$^{\alpha}$-(2-Chloro-4-(5,6,7,8-tetrahydrothieno[3,2-b]azepin-4-ylcarbonyl)-benzylcarbamoyl)-4-methoxy-L-proline-*N*-methyl-*N*-(2-picolyl)amide**

(4R)-*N*$^{\alpha}$-(4-(*tert*-Butyloxycarbonyl)-2-chlorobenzylcarbamoyl)-4-methoxy-L-proline-*N*-methyl-*N*-(2-picolyl) amide (60mg, 0.12mmol) was dissolved in 60% TFA/DCM (10ml) and the solution was stirred at RT for 90min. The solvent was removed *in vacuo,* and the residue was dissolved in dichloromethane (10ml) then cooled to 0°C. 5,6,7,8-Tetrahydrothieno[3,2-*b*]azepine (20mg, 0.12mmol), WCSD (48mg, 0.25mmol), 4-dimethylaminopyridine (15mg, 0.12mmol) and triethlyamine (56µl, 0.4mmol) were added and the solution was heated at reflux for 1 hr. The solvent was removed *in vacuo* and the residue was dissolved in ethyl acetate. The solution was washed with 0.3M $KHSO_4$, sat. $NaHCO_3$ and brine, dried over $Na_2SO_4$ and concentrated *in vacuo.* The residue was purified by flash chromatography on silica gel (eluant 97% chloroform/3% methanol) to give a white solid identified as (4*R*)-*N*$^{\alpha}$-(2-chloro-4-(5,6,7,8-tetrahydrothieno[3,2-*b*]azepin-4-ylcarbonyl)benzylcarbamoyl)-4-methoxy-L-proline-*N*-methyl-*N*-(2-picolyl) amide (32mg, 45%)

**NMR** (270MHz $CDCl_3$) δ 1.74-2.26 (8H,m), 2.86-2.89 (3H,m), 3.07-3.32 (4H,m), 3.44-4.97 (10H,m), 6.12-6.16 (1H,m), 6.58-6.65 (1H,m), 6.99-7.28 (4H,m), 7.53-7.70 (2H,m), 8.17-8.44 (1H,m) ppm.

**MS** $[M+H]^+$ 596.2

**Example 17**

**1-((4*R)-N*$^{\alpha}$-(2-Chloro-4-(5,6,7,8-tetrahydrothieno[3,2-*b*]azepin-4-ylcarbonyl)-benzylcarbamoyl)-4-methoxy-L-prolyl)-4-(1-pyrrolidinyl)piperidine**

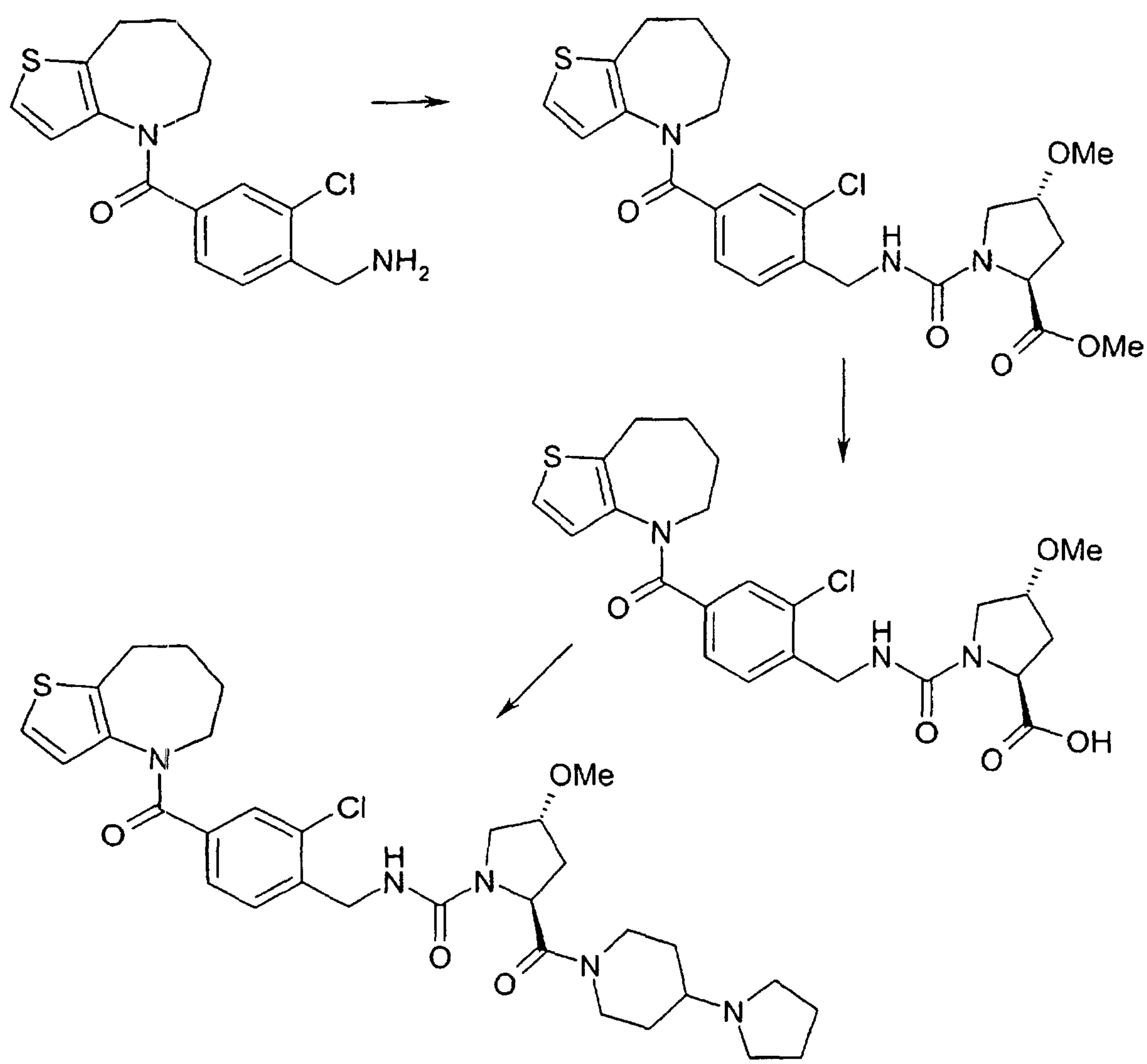

**17A: (4*R)-N*$^{\alpha}$-(2-Chloro-4-(5,6,7,8-tetrahydrothieno[3,2-*b*]azepin-4-ylcarbonyl)-benzylcarbamoyl)-4-methoxy-L-proline methyl ester**

1,1'-Carbonyldiimidazole (197.8mg, 1.22mmol) was added to a solution of 4-(4-aminomethyl-3-chlorobenzoyl)-5,6,7,8-tetrahydrothieno[3,2-*b*]azepine (400mg, 1.11mmol) and DIEA (306µl, 1.66mmol) in DMF (10ml) under nitrogen gas and the solution was stirred at RT for 1hr. A solution of (4R)-4-methoxy-L-proline methyl ester (176mg, 1.11mmol) in DMF (10ml) was added and the mixture was stirred at RT for 18hr under nitrogen gas. The solvent was removed *in vacuo* and the residue was purified by flash chromatography on silica gel (eluant 98% chloroform/1% methanol/1% acetic acid) to give a white solid identified as (4*R*)-*N*$^{\alpha}$(2-chloro-4-(5,6,7,8-tetrahydrothieno[3,2-*b*]azepin-4-ylcarbonyl)benzylcarbamoyl)-4-methoxy-L-proline methyl ester (550mg, 98%).

**17B: (4*R)-N*$^{\alpha}$-(2-Chloro-4-(5,6,7,8-tetrahydrothieno[3,2-*b*]azepin-4-ylcarbonyl)-benzylcarbamoyl)-4-methoxy-L-proline**

A solution of lithium hydroxide (68.5mg, 1.63mmol) and (4R)-*N*$^{\alpha}$-(2-chloro-4-(5,6,7,8-tetrahydrothieno[3,2-*b*]

azepin-4-ylcarbonyl)benzylcarbamoyl)-4-methoxy-L-proline methyl ester (555mg, 1.08 mmol) in THF/water (1:1, 40ml) was stirred at RT for 1hr. The dioxan was removed *in vacuo* and the aqueous residue was acidified with 1N HCl and extracted with ethyl acetate (3 × 50ml). The organic extracts were combined and washed with water, dried over $Na_2SO_4$ and concentrated *in vacuo* to give a white solid identified as (4R)-*N*α-(2-chloro-4-(5,6,7,8-tetrahydrothieno[3,2-*b*]azepin-4-ylcarbonyl)-benzylcarbamoyl)-4-methoxy-L-proline (490 mg, 92%).

**17C: 1-((4*R)-N*α-(2-Chloro-4-(5,6,7,8-tetrahydrothieno[3,2-*b*]azepin-4-ylcarbonyl)-benzylcarbamoyl)-4-methoxy-L-prolyl)-4-(1-pyrrolidinyl)piperidine**

HOBt (14.8mg, 0.097mmol), WSCD (22.68mg, 0.11mmol) and 4-(pyrrolidinyl)piperidine (13.7mg, 0.089mmol) were added to an ice-cold solution of (4R)-*N*α-(2-chloro-4-(5,6,7,8-tetrahydrothieno[3,2-*b*]azepin-4-ylcarbonyl)benzylcarbamoyl)-4-methoxy-L-proline (40mg, 0.081mmol) and triethylamine (18μl, 0.12mmol) in dichloromethane (10ml). The resulting solution was stirred at RT for 18hr. The solvent was removed *in vacuo* and the residue was dissolved in ethyl acetate. The solution was washed with sat. $NaHCO_3$ and brine, dried over $Na_2SO_4$ and concentrated *in vacuo.* The residue was purified by flash chromatography on silica gel (eluant 94% chloroform/4% methanol/2% triethylamine) to give a white solid identified as 1-((4*R*)-*N*α-(2-chloro-4-(5,6,7,8-tetrahydrothieno[3,2-*b*]azepin-4-ylcarbonyl)benzylcarbamoyl)-4-methoxy-L-prolyl)-4-(1-pyrrolidinyl)piperidine (40.9mg, 80%).
**NMR** (270MHz $CDCl_3$) δ 1.11-1.33(7H,m), 1.76-2.15 (12H,m), 2.60-2.73(3H,m), 2.88-2.92 (2H,m), 3.28 (3H,s), 3.29-3.32 (1H,m), 3.67-3.70 (1H,m), 3.95-4.23 (3H,m), 4.61-4.83 (2H,m), 4.76-4.78 (1H,m), 4.86-4.89 (1H,m), 6.16-6.17 (1H,m), 6.65-6.67 (1H,m), 7.02-7.05 (1H,m), 7.24-7.26 (3H,m) ppm.
**MS** $[M+H]^+$ 628.3

The following compounds are prepared by analogous methods.

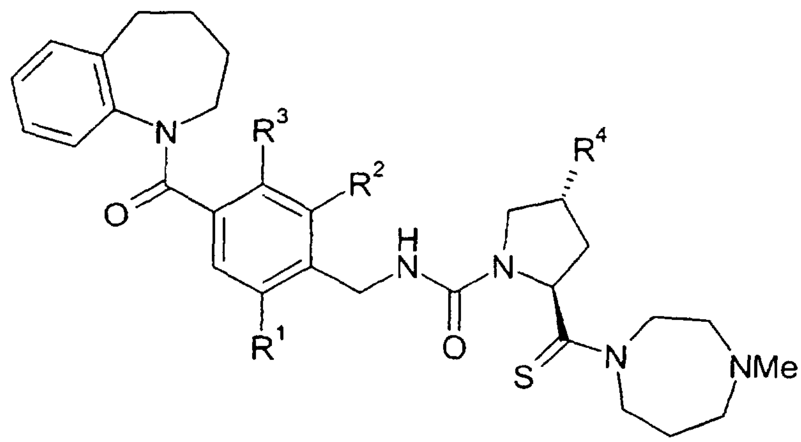

| Example No. | $R^1$ | $R^2$ | $R^3$ | $R^4$ | $[M+H]^+$ |
|---|---|---|---|---|---|
| **18** | H | H | Me | H | 548.3 |
| **19** | H | H | Me | OMe | 578.4 |
| **20** | Me | Me | H | H | 562.4 |
| **21** | Me | Me | H | OMe | 592.4 |
| **22** | Et | H | H | H | 562.3 |
| **23** | Et | H | H | OMe | 592.3 |
| **24** | Cl | H | H | H | 568.3 |
| **25** | Cl | H | H | OMe | 598.3 |
| **26** | H | H | H | H | 534.4 |
| **27** | H | H | H | OMe | 564.4 |

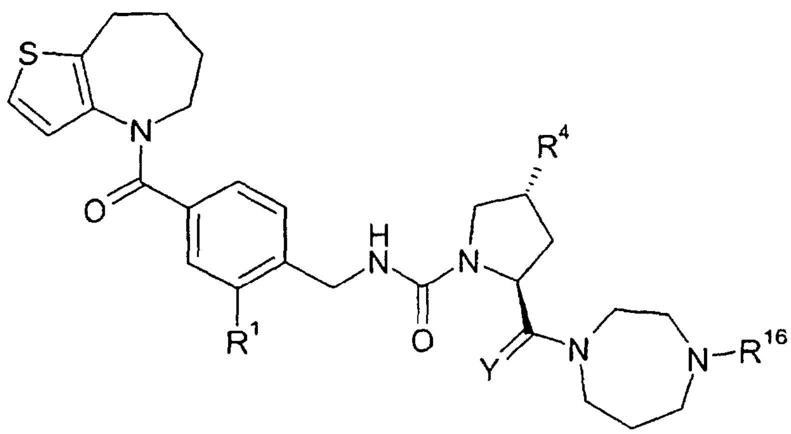

| Example No | $R^1$ | $R^4$ | $R^{16}$ | Y | $[M^+H]^+$ |
|---|---|---|---|---|---|
| **28** | Cl | OMe | Me | S | 604.2 |
| **29** | F | H | Me | S | 558.2 |
| **30** | Me | H | Me | S | 554.2 |
| **31** | Me | H | Pr | S | 582.3 |
| **32** | Me | H | iBu | S | 596.3 |
| **33** | Me | H | Et | S | 568.3 |
| **34** | Cl | H | Me | O | 558.3 |
| **35** | Cl | OMe | Me | O | 588.4 |

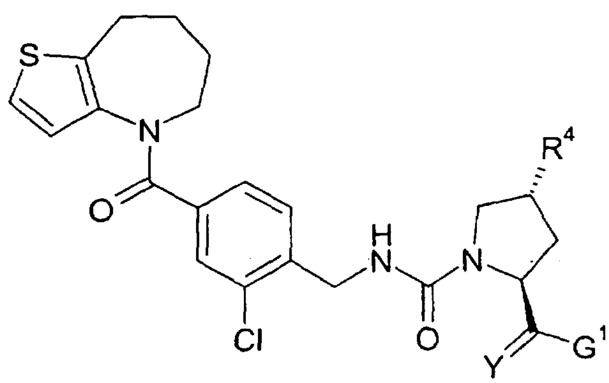

<table>
<tr><th>Example No</th><th>$R^4$</th><th>$G^1$</th><th>Y</th><th>$[M+H]^+$</th></tr>
<tr><td>36</td><td>H</td><td rowspan="2">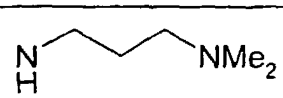</td><td>O</td><td>546.3</td></tr>
<tr><td>37</td><td>OMe</td><td>O</td><td>576.2</td></tr>
</table>

| Example No | $R^4$ | $G^1$ | Y | $[M+H]^+$ |
|---|---|---|---|---|
| 38 | H | 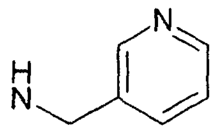 | O | 552.2 |
| 39 | OMe | | O | 582.2 |
| 40 | H | 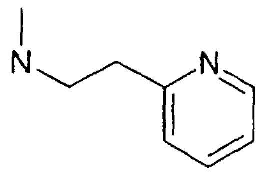 | O | 580.2 |
| 41 | OMe | | O | 610.3 |
| 42 | OMe | | S | 626.2 |
| 43 | OMe | 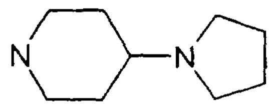 | S | 644.2 |
| 44 | H | | O | 598.2 |
| 45 | H | 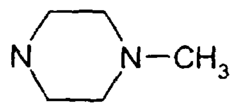 | O | 544.2 |
| 46 | OMe | | O | 574.2 |

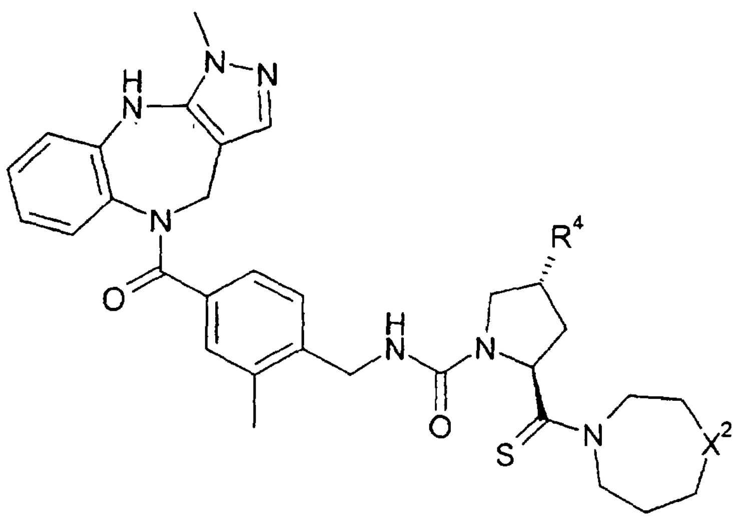

| Example No | $R^4$ | $X^2$ | $[M+H]^+$ |
|---|---|---|---|
| **47** | H | N-H | 587.3 |
| **48** | H | N-Pr | 629.2 |
| **49** | H | N-iBu | 643.3 |
| **37** | H | N-Et | 615.1 |
| **50** | OMe | N-Me | 631.3 |

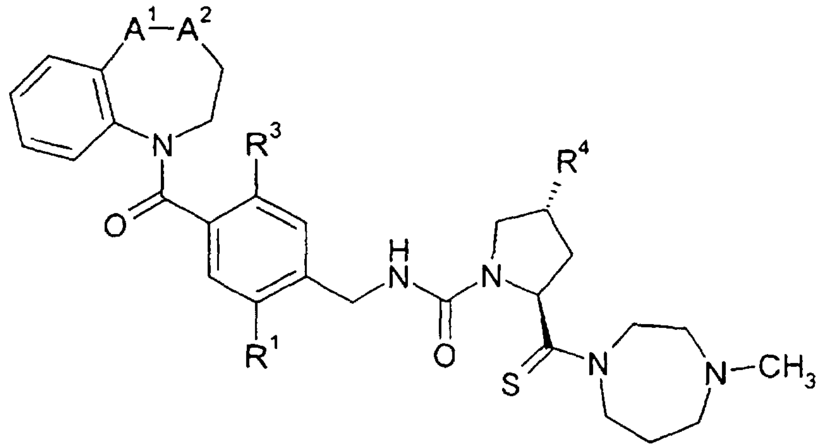

| Example No | $R^1$ | $R^3$ | $R^4$ | $A^1$ | $A^2$ | $[M^{+H]+}$ |
|---|---|---|---|---|---|---|
| **51** | H | Me | H | NH | $CH_2$ | 549.3 |
| **52** | H | Me | OMe | NH | $CH_2$ | 579.3 |
| **53** | Me | H | H | N-Et | $CH_2$ | 577.4 |
| **54** | Me | H | OMe | N-Et | $CH_2$ | 607.3 |
| **55** | H | Me | H | N-Me | $CH_2$ | 563.4 |
| **56** | H | Me | OMe | N-Me | $CH_2$ | 593.3 |
| **57** | Me | H | H | N-Me | $CH_2$ | 563.4 |
| **58** | Me | H | OMe | N-Me | $CH_2$ | 593.4 |
| **59** | Me | H | H | N-$(CH_2)_2$OH | $CH_2$ | 593.4 |
| **60** | Me | H | OMe | N-$(CH_2)_2$OH | $CH_2$ | 623.4 |
| **61** | Me | H | H | S | $CH_2$ | 566.3 |
| **62** | Me | H | OMe | S | $CH_2$ | 596.3 |
| **63** | H | H | H | O | $CH_2$ | 536.4 |
| **64** | H | H | OMe | O | $CH_2$ | 566.4 |
| **65** | Cl | H | H | O | $CH_2$ | 570.3 |
| **66** | Cl | H | OMe | O | $CH_2$ | 600.3 |
| **67** | Me | H | H | $CH_2$ | NH | 549.3 |

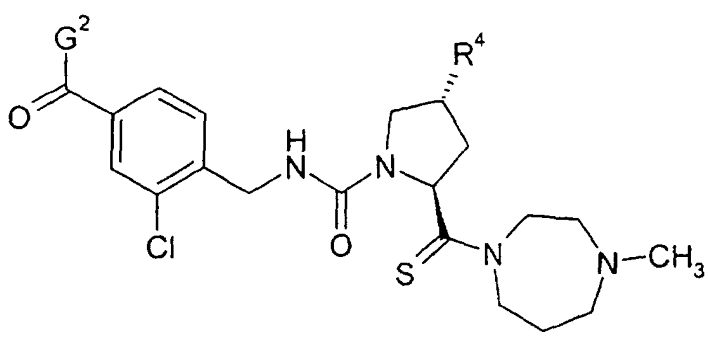

| Example No | $R^4$ | $G^2$ | $[M+H]^+$ |
|---|---|---|---|
| 68 | H | 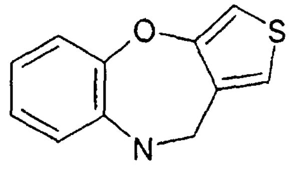 | 624.2 |
| 69 | OMe | | 654.3 |
| 70 | H | 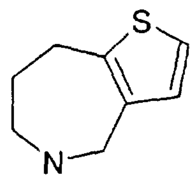 | 574.2 |
| 71 | OMe | | 604.2 |

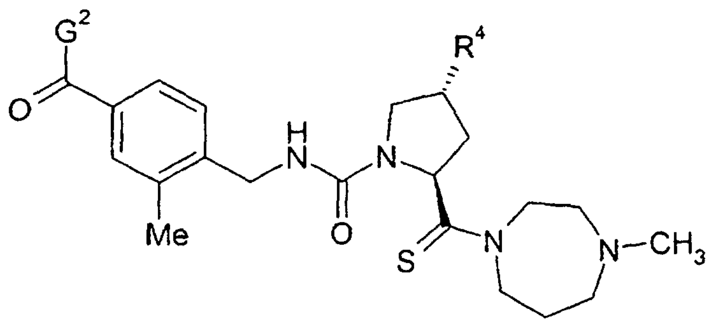

| Example No | $R^4$ | $G^2$ | $[M+H]^+$ |
|---|---|---|---|
| 72 | H | 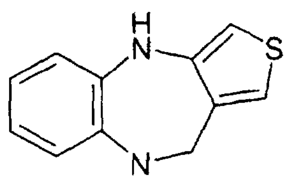 | 603.3 |
| 73 | OMe | | N/A |

| Example No | R4 | G2 | [M+H]+ |
|---|---|---|---|
| 74 | H | 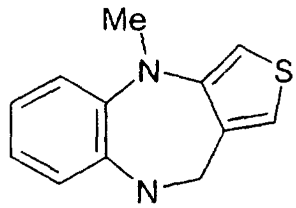 | 617.3 |
| 75 | OMe | | 647.3 |
| 76 | H | 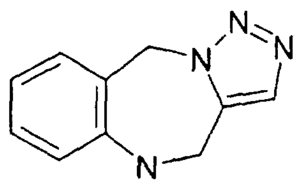 | 587.3 |
| 77 | OMe | | 617.3 |
| 78 | H | 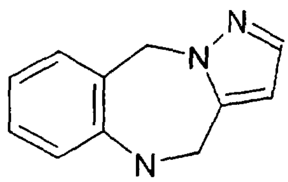 | 586.3 |
| 79 | H | 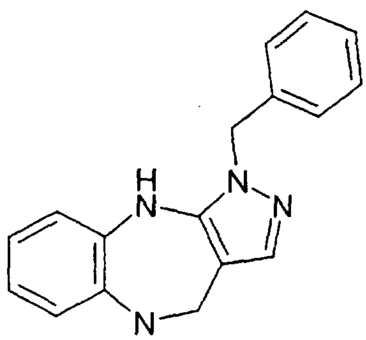 | 677.3 |
| 80 | H | 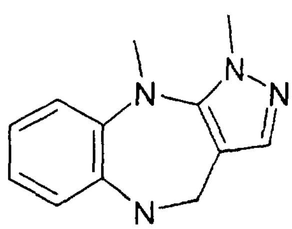 | 615.2 |
| 81 | H | 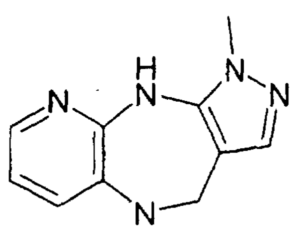 | 602.2 |
| 82 | H | | 598.4 |

| Example No | $R^4$ | $G^2$ | $[M+H]^+$ |
|---|---|---|---|
| 83 | OMe | 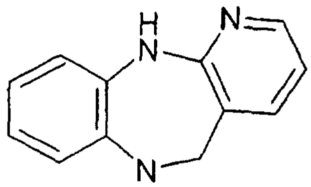 | 628.4 |
| 84 | H | 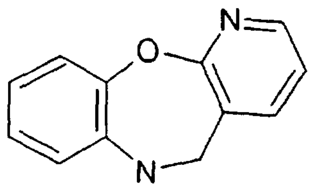 | 599.2 |
| 85 | H | 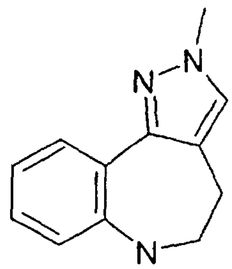 | 600.4 |
| 86 | OMe | | 630.4 |

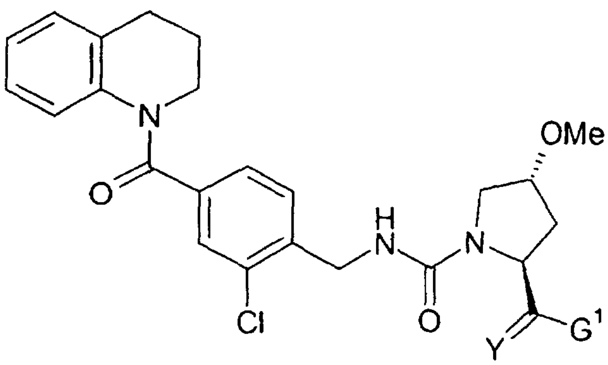

| Example No | Y | $G^1$ | $[M+H]^+$ |
|---|---|---|---|
| **87** | S | 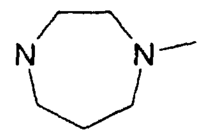 | |
| **88** | O | | 584.3 |
| **89** | S | 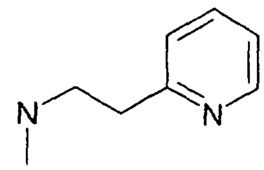 | 606.3 |

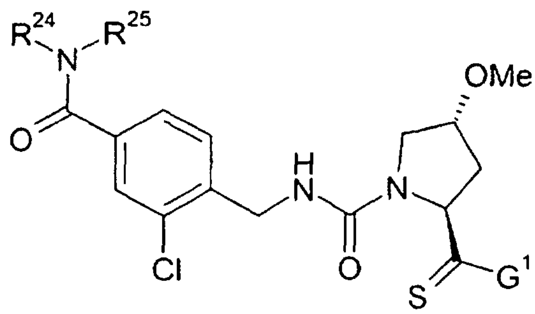

| Example No | $R^{24}$ | $R^{25}$ | Y | $G^1$ | $[M+H]^+$ |
|---|---|---|---|---|---|
| **90** | i-Bu | i-Bu | S | 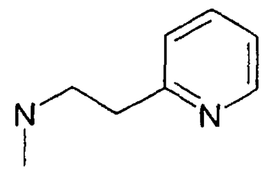 | 602.3 |
| **91** | i-Pr | 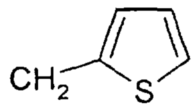 | S | | 628.3 |
| **92** | Me | i-Pr | S | | 524.4 |
| **93** | Et | i-Pr | O | 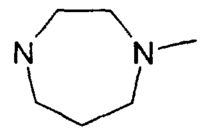 | 522.4 |
| **94** | Et | i-Pr | S | | |
| **95** | i-Pr | i-Pr | S | | 552.4 |
| **96** | i-Pr | 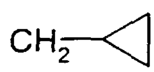 | S | 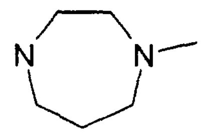 | 564.4 |
| **97** | i-Bu | i-Bu | S | | 580.2 |
| **98** | n-Pn | n-Pn | O | | |
| **99** | n-Pn | n-Pn | S | 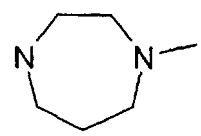 | |
| **100** | i-Pr | Ph | O | | 570.3 |

| Example No | $R^{24}$ | $R^{25}$ | Y | $G^1$ | $[M+H]^+$ |
|---|---|---|---|---|---|
| **101** | n-Bu | Ph | O | | |
| **102** | Et | 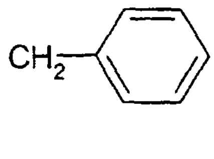 | S | 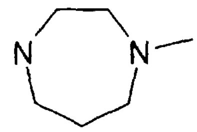 | |
| **103** | i-Pr | 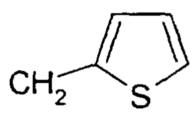 | O | | 590.3 |
| **104** | i-Pr | 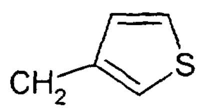 | S | 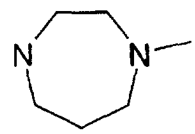 | |
| **105** | Ph | 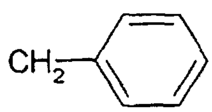 | O | | |

**Example 106**

***In vitro* Testing**

Compounds were assayed to determine their ability to inhibit the binding of [$^{125}$I]OVA to a cell membrane preparation of OT receptors (binding assay) and to mimic the cellular consequences of OT stimulation on intact cells (functional assay). In the binding assay the compounds of the invention generally demonstrate significant inhibition of radioligand binding at concentrations of 50μM or less. In the functional assay, the compounds of the invention cause significant cellular activation at concentrations of 30μM or less. Preferred compounds cause significant activation at concentrations of 300nM or less and can induce the same maximal effect as OT. The compounds are either significantly less active or completely devoid of activity in assays for vasopressin-like activity.

**Example 107**

***In vivo* Testing**

Representative compounds were tested for activity in the rat uterine contractility model, which is a recognised test for OT agonism. The compounds increased the strength and frequency of the uterine contractions at doses below 50mg/kg. Selected compounds were then given either *i.c.v.* or *i.v.* to male rats and the erectile response was determined.

**Example 108**

**Tablet for Oral Administration**

Tablets containing 100mg of the compound of Example 11 as the active agent are prepared from the following:

| | |
|---|---|
| Compound of Example 11 | 200.0g |
| Corn starch | 71.0g |

(continued)

| | |
|---|---|
| Hydroxypropylcellulose | 18.0g |
| Carboxymethylcellulose calcium | 13.0g |
| Magnesium stearate | 3.0g |
| Lactose | 195.0g |
| *Total* | *500.0g* |

The materials are blended and then pressed to give 2000 tablets of 250mg, each containing 100mg of the compound of Example 11.

The foregoing demonstrates that the compounds according to the present invention act as agonists at the oxytocin receptor and accordingly they may find utility as pharmaceutical agents for the treatment of conditions such as sexual disorders including male erectile dysfunction and ejaculatory disorders, female sexual dysfunction, cancer of the prostate, breast, ovary and bones, osteoporosis, benign prostatic hyperplasia, post-partum bleeding, and depression. The compounds may also be used to induce labour or delivery of the placenta, to decrease arterial blood pressure, to decrease exaggerated responses to stress and to increase the nociceptive threshold.

The scope of the present invention is further defined in the following claims.

## Claims

1. A compound according to general formula **1**, or a pharmaceutically acceptable salt thereof

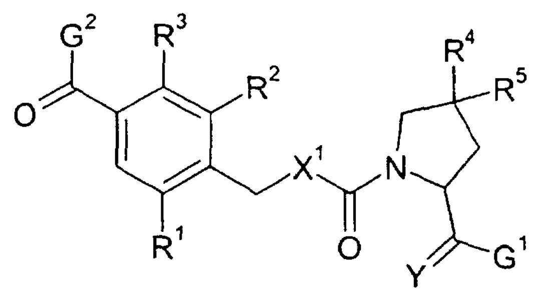

**1**

wherein:

$G^1$ is selected from a group according to general formula **2** and a group according to general formula **3**;

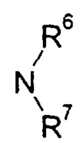

**2**

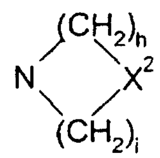

**3**

$G^2$ is selected from a group according to general formula **4**, a group according to general formula **5**, a group according to general formula **6**, a group according to general formula **7**, a group according to general formula **8** and a group according to general formula **9**;

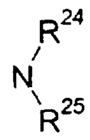
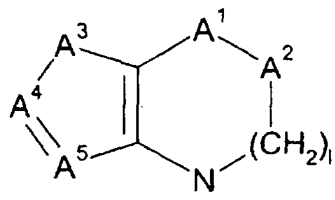
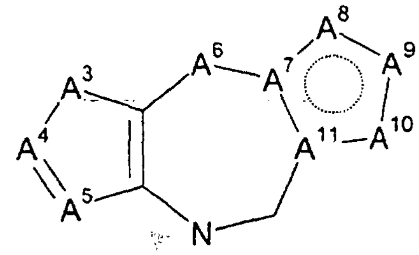

**4** **5** **6**

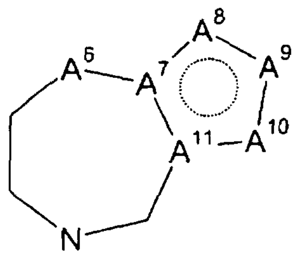
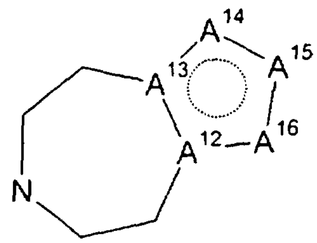
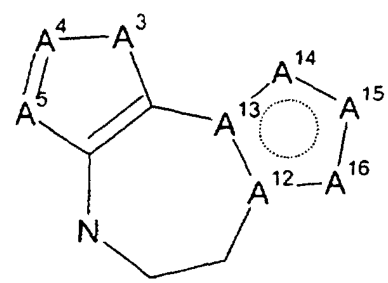

**7** **8** **9**

$A^1$ is selected from $CH_2$, CH(OH), NH, N-alkyl, N-$(CH_2)_n$-$R^{27}$, O and S;

$A^2$ is selected from $CH_2$, CH(OH), C(=O) and NH;

$A^3$ is selected from S, NH, N-alkyl, -CH=CH- and -CH=N-;

$A^4$ and $A^5$ are each selected from CH and N;

$A^6$ is selected from $CH_2$, NH, N-alkyl and O;

$A^7$ and $A^{11}$ are selected from C and N;

$A^8$ and $A^9$ are selected from CH, N, NH, $N(CH_2)_mR^{26}$ and S;

$A^{10}$ is selected from -CH=CH-, CH, N, NH, $N(CH_2)_mR^{26}$ and S;

$A^{12}$ and $A^{13}$ are selected from N and C;

$A^{14}$, $A^{15}$ and $A^{16}$ are selected from NH, N-$CH_3$, S, N and CH;

$X^1$ is selected from O and NH;

$X^2$ is selected from $NR^{16}$, CH-$NR^{17}R^{18}$, CH-$CH_2NR^{17}R^{18}$, $N^+R^{19}R^{20}$, CH-$N^+R^{21}R^{22}R^{23}$ and CH-$CH_2N^+R^{21}R^{22}R^{23}$;

Y is selected from O and S;

$R^1$, $R^2$ and $R^3$ are each selected from H, alkyl, O-alkyl, F, Cl and Br;

$R^4$ and $R^5$ are each selected from H, O-alkyl, O-benzyl and F, or $R^4$ and $R^5$ together are =O, -$O(CH_2)_aO$- or -$S(CH_2)_aS$-;

$R^6$ is selected from a group according to general formula **10,** a group according to general formula **11,** a group according to general formula **12,** a group according to general formula **13,** a group according to general formula **14,** a group according to general formula **15,** a group according to general formula **16,** a group according to general formula **17,** a group according to general formula **18,** a group according to general formula **19,** a group according to general formula **20,** a group according to general formula **21,** a group according to general formula **22,** a group according to general formula **23,** a group according to general formula **24** and a group according to general formula **25;**

$$-(CH_2)_b-NR^8R^9 \qquad \mathbf{10}$$

$$-(CH_2)_b-N^+R^{11}R^{12}R^{13} \qquad \mathbf{11}$$

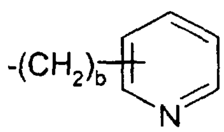

**12**

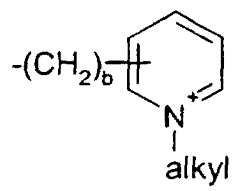

**13**

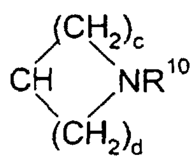

**14**

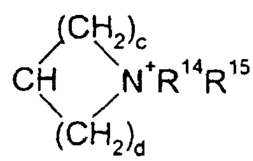

**15**

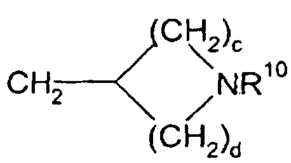

**16**

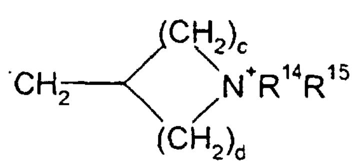

**17**

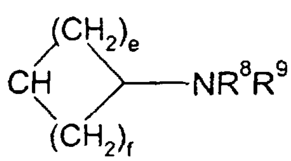

**18**

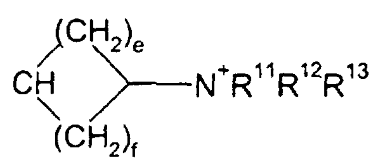

**19**

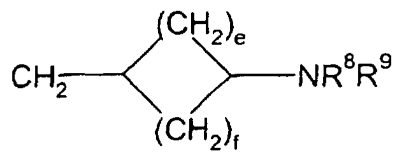

20

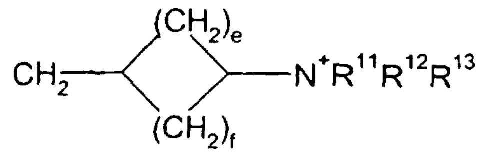

21

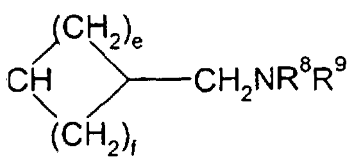

22

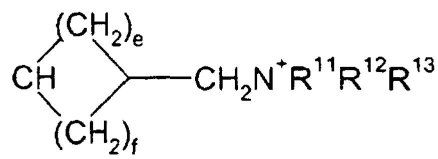

23

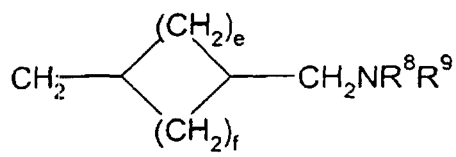

24

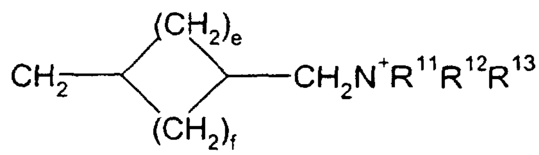

25

$R^7$ is selected from H, alkyl and any group as defined above for $R^6$;

$R^8$, $R^9$ and $R^{10}$ are independently selected from H and alkyl, or $R^8$ and $R^9$ together may be $-(CH_2)_g-$;

$R^{11}$, $R^{12}$, $R^{13}$, $R^{14}$ and $R^{15}$ are all alkyl, or $R^{11}$ and $R^{12}$ together or $R^{14}$ and $R^{15}$ together may be $-(CH_2)_g-$;

$R^{16}$, $R^{17}$ and $R^{18}$ are independently selected from H and alkyl, or $R^{17}$ and $R^{18}$ together may be $-(CH_2)_j-$;

$R^{19}$, $R^{20}$, $R^{21}$, $R^{22}$ and $R^{23}$ are all alkyl, or $R^{19}$ and $R^{20}$ together or $R^{21}$ and $R^{22}$ together may be $-(CH_2)_j-$;

$R^{24}$ and $R^{25}$ are independently selected from alkyl, Ar and $-(CH_2)_k$-Ar;

$R^{26}$ is selected from H, alkyl, optionally substituted phenyl, pyridyl, thienyl, OH, O-alkyl, $NH_2$, NH-alkyl, N (alkyl)$_2$, $CO_2H$ $CO_2$-alkyl, $CONH_2$, CONH-alkyl, CON(alkyl)$_2$, CN and $CF_3$;

$R^{27}$ is selected from OH, O-alkyl, O-CO-alkyl, $NH_2$, NH-alkyl and N(alkyl)$_2$;

Ar is selected from thienyl and optionally substituted phenyl;

a is 2 or 3, b is 1, 2 or 3; c is 1 or 2, d is 1, 2 or 3; e is 1 or 2; f is 1, 2 or 3; g is 4, 5 or 6; h is 1, 2 or 3; i is 1, 2, 3 or 4; j is 4, 5 or 6; k is 1, 2 or 3; l is 1 or 2; m is 1, 2 or 3; and n is 2, 3 or 4;

provided that:

not more than one of $A^8$, $A^9$ and $A^{10}$ is NH, $N(CH_2)_mR^{26}$ or S;

$A^7$ and $A^{11}$ are not both simultaneously N;

neither $A^7$ nor $A^{11}$ is N if one of $A^8$, $A^9$ and $A^{10}$ is NH, $N(CH_2)_mR^{26}$ or S;

if $A^{10}$ is -CH=CH- then $A^8$ is N, $A^9$ is CH and both $A^7$ and $A^{11}$ are C;

if $A^{10}$ is not -CH=CH- then one of $A^8$, $A^9$ and $A^{10}$ is NH, $N(CH_2)_mR^{26}$ or S or one of $A^7$ and $A^{11}$ is N;

not more than one of $A^{14}$, $A^{15}$ and $A^{16}$ is NH, $N\text{-}CH_3$ or S;

$A^{12}$ and $A^{13}$ are not both simultaneously N;

if one of $A^{14}$, $A^{15}$ and $A^{16}$ is NH, $N\text{-}CH_3$ or S then $A^{12}$ and $A^{13}$ are both C; and

one of $A^{14}$, $A^{15}$ and $A^{16}$ is NH, $N\text{-}CH_3$ or S or one of $A^{12}$ and $A^{13}$ is N.

**2.** A compound according to Claim 1 wherein at least one of $R^1$, $R^2$ and $R^3$ is H and one is not H.

**3.** A compound according to any preceding Claim wherein one of $R^1$, $R^2$ and $R^3$ is selected from alkyl, F, Cl and Br and the others are H.

**4.** A compound according to any preceding Claim wherein $R^1$ is methyl or Cl and $R^2$ and $R^3$ are H.

**5.** A compound according to any preceding Claim wherein one of $R^4$ and $R^5$ is H and the other is O-alkyl.

**6.** A compound according to any preceding Claim wherein one of $R^4$ and $R^5$ is H and the other is O-methyl.

**7.** A compound according to any of Claims 1 to 4 wherein $R^4$ and $R^5$ are both H.

**8.** A compound according to any of Claims 1 to 4 wherein $R^4$ and $R^5$ are both F

**9.** A compound according to any preceding Claim wherein $X^1$ is NH.

**10.** A compound according to any preceding Claim wherein $G^1$ is a group according to general formula **2**.

**11.** A compound according to any of Claims 1 to 9 wherein $G^1$ is a group according to general formula **3.**

**12.** A compound according to any preceding Claim wherein $G^2$ is a group according to general formula **4.**

**13.** A compound according to any of Claims 1 to 11 wherein $G^2$ is a group according to general formula **5**, **6, 7**, **8** or **9.**

**14.** A compound according to any of Claims 1 to 11 wherein $G^2$ is a group according to general formula **5,** $A^1$ is $CH_2$ and $A^2$ is NH.

**15.** A compound according to any of Claims 1 to 11 wherein $G^2$ is a group according to general formula **5,** $A^1$ is NH or N-alkyl and $A^2$ is C(=O).

**16.** A compound according to any of Claims 1 to 11 wherein $G^2$ is a group according to general formula **5**, **6** or **9**, $A^3$ is S and $A^4$ and $A^5$ are both CH.

**17.** A compound according to any of Claims 1 to 11 wherein $G^2$ is a group according to general formula **5**, **6** or **9**, $A^3$ is -CH=CH- and $A^4$ and $A^5$ are both CH.

**18.** A compound according to Claim 1 wherein $R^1$ is methyl or Cl, $R^2$ and $R^3$ are both H, $R^4$ is H or O-methyl, $R^5$ is H, $X^1$ is NH and Y is S.

**19.** A compound according to Claim 1 wherein $R^1$ is methyl or Cl, $R^2$ and $R^3$ are both H, $R^4$ is H or O-methyl, $R^5$ is H, $X^1$ is NH and Y is O.

**20.** A compound according to Claim 1 wherein $R^2$ and $R^3$ are both H, $X^1$ is NH and $G^1$ is

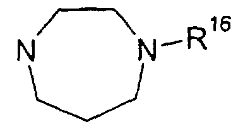

21. A compound according to Claim 1 wherein $R^1$ is methyl or Cl, $R^2$ and $R^3$ are both H, $R^4$ is H or O-methyl, $R^5$ is H, $X^1$ is NH and $G^2$ is

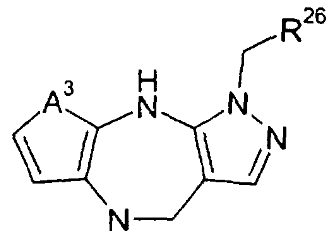

22. A compound according to Claim 1 wherein $R^1$ is methyl or Cl, $R^2$ and $R^3$ are both H, $R^4$ is H or O-methyl, $R^5$ is H, $X^1$ is NH, $G^1$ is

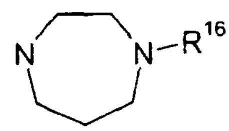

and $G^2$ is

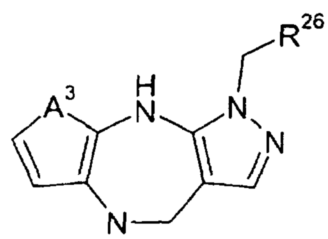

23. A compound according to Claim 1 selected from

4-methyl-1-(*N*-(2-methyl-4-(2,3,4,5-tetrahydro-1,5-benzodiazepin-4-on-1-yl-carbonyl)benzylcarbamoyl)-L-thioprolyl)perhydro-1,4-diazepine,

4-methyl-1-(*N*-(2-methyl-4-(1-methyl-4,10-dihydropyrazolo[5,4-*b*][1,5]-benzodiazepin-5-ylcarbonyl)benzylcarbamoyl)-L-thioprolyl)perhydro-1,4-diazepine,

4,4-dimethyl-1-(*N*-(2-methyl-4-(1-methyl-4,10-dihydropyrazolo[5,4-*b*][1,5]-benzodiazepin-5-ylcarbonyl)benzylcarbamoyl)-L-thioprolyl)perhydro-1,4-diazepinium iodide,

4-methyl-1-(*N*-(2-methyl-4-(5,6,7,8-tetrahydrothieno[3,2-*b*]azepin-4-ylcarbonyl)-benzylcarbamoyl)-L-thioprolyl)perhydro-1,4-diazepine,

4-methyl-1-(*N*-(2-methyl-4-(5,6,7,8-tetrahydrothieno[3,2-*b*]azepin-4-ylcarbonyl)-benzyloxycarbonyl)-L-prolyl)perhydro-1,4-diazepine,

(4*R*)-*N*$^{\alpha}$-(2-chloro-4-(5,6,7,8-tetrahydrothieno[3,2-*b*]azepin-4-ylcarbonyl)benzylcarbamoyl)-4-methoxy-L-proline-*N*-methyl-*N*-(2-picolyl)amide, and

1-((4*R*)-*N*$^{\alpha}$-(2-chloro-4-(5,6,7,8-tetrahydrothieno[3,2-*b*]azepin-4-ylcarbonyl)benzylcarbamoyl)-4-methoxy-L-prolyl)-4-(1-pyrroiidinyl)piperidine.

**24.** A pharmaceutical composition which comprises a compound according to any of Claims 1 to 23 as an active agent.

**25.** A pharmaceutical composition according to Claim 24 which is a tablet or capsule for oral administration.

**26.** A pharmaceutical composition according to Claim 24 or 25 which is for the treatment of male erectile dysfunction.

**27.** A use a compound according to any of Claims 1 to 23 of in the manufacture of a pharmaceutical composition.

**28.** A use according to Claim 27 wherein the pharmaceutical composition is to be used in the treatment of male erectile dysfunction.

**29.** The use of a compound according to any of Claims 1 to 23 in the manufacturing of a medicament for treating male or female sexual disorders.

**30.** One or more optical isomers of a compound according to any of claims 1 to 22.

**Patentansprüche**

**1.** Verbindung der allgemeinen Formel 1, oder ein pharmazeutisch annehmbares Salz davon

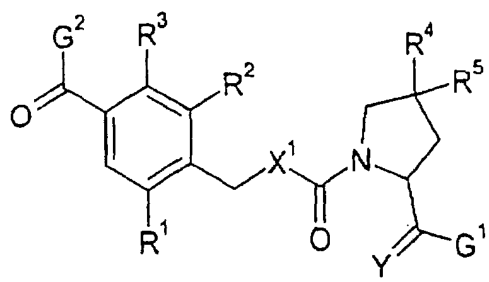

**1**

wobei

$G^1$ ausgewählt ist aus einer Gruppe der allgemeinen Formel 2 und einer Gruppe der allgemeinen Formel 3,

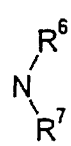

**2**

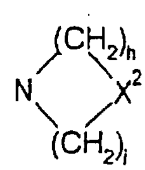

**3**

$G^2$ ausgewählt ist aus einer Gruppe der allgemeinen Formel 4, einer Gruppe der allgemeinen Formel 5, einer Gruppe der allgemeinen Formel 6, einer Gruppe der allgemeinen Formel 7, einer Gruppe der allgemeinen Formel 8 und einer Gruppe der allgemeinen Formel 9

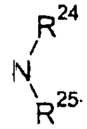

4

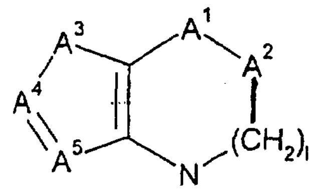

5

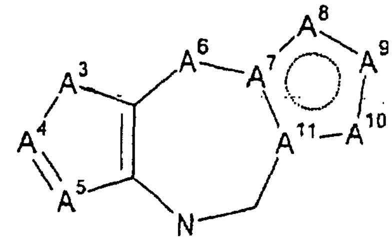

6

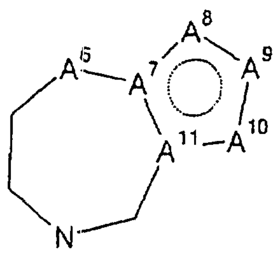

7

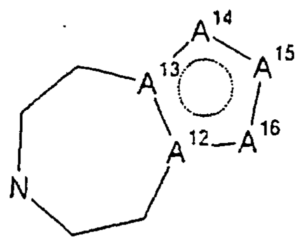

8

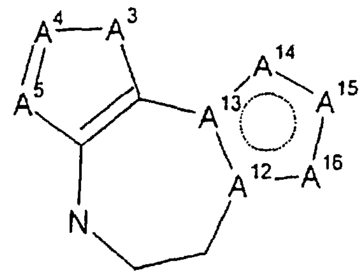

9

$A^1$ ausgewählt ist aus $CH_2$, CH(OH), NH, N-Alkyl, N-$(CH_2)_n$-$R^{27}$, O und S;
$A^2$ ausgewählt ist aus $CH_2$, CH(OH), C(=O) und NH;
$A^3$ ausgewählt ist aus S, NH, N-Alkyl, -CH=CH- und -CH=N-;
$A^4$ und $A^5$ jeweils ausgewählt sind aus CH und N;
$A^6$ ausgewählt ist aus $CH_2$, NH, N-Alkyl und O;
$A^7$ und $A^{11}$ ausgewählt sind aus C und N;
$A^8$ und $A^9$ ausgewählt sind aus CH, N, NH, $N(CH_2)_mR^{26}$ und S;
$A^{10}$ ausgewählt ist aus -CH=CH-, CH, N, NH, $N(CH_2)_mR^{26}$ und S;
$A^{12}$ und $A^{13}$ ausgewählt sind aus C und N;
$A^{14}$, $A^{15}$ und $A^{16}$ ausgewählt sind aus NH, N-$CH_3$, S, N und CH;
$X^1$ ausgewählt ist aus O und NH;
$X^2$ ausgewählt ist aus $NR^{16}$,CH-$NR^{17}R^{18}$, CH-$CH_2NR^{17}R^{18}$, $N^+R^{19}R^{20}$, CH-$N^+R^{21}R^{22}R^{23}$ und CH-$CH_2N^+R^{21}R^{22}R^{23}$;
Y ausgewählt ist aus O und S;
$R^1$, $R^2$ und $R^3$ jeweils ausgewählt sind aus H, Alkyl, O-Alkyl, F, Cl und Br;
$R^4$ und $R^5$ jeweils ausgewählt sind aus H, O-Alkyl, O-Benzyl und F oder $R^4$ und $R^5$ zusammen =O, -$O(CH_2)_aO$- oder -$S(CH_2)_aS$- sind;
$R^4$ ausgewählt ist aus einer Gruppe der allgemeinen Formel 10, einer Gruppe der allgemeinen Formel 11, einer Gruppe der allgemeinen Formel 12, einer Gruppe der allgemeinen Formel 13, einer Gruppe der allgemeinen Formel 14, einer Gruppe der allgemeinen Formel 15, einer Gruppe der allgemeinen Formel 16, einer Gruppe der allgemeinen Formel 17, einer Gruppe der allgemeinen Formel 18, einer Gruppe der allgemeinen Formel 19, einer Gruppe der allgemeinen Formel 20, einer Gruppe der allgemeinen Formel 21, einer Gruppe der allgemeinen Formel 22, einer Gruppe der allgemeinen Formel 23, einer Gruppe der allgemeinen Formel 24 und einer Gruppe der allgemeinen Formel 25;

$$-(CH_2)_b-NR^8R^9 \qquad \mathbf{10}$$

$$-(CH_2)_b-N^+R^{11}R^{12}R^{13} \qquad \mathbf{11}$$

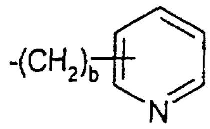

12

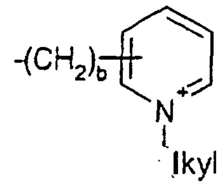

13

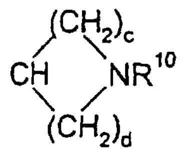

14

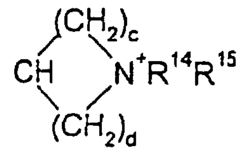

15

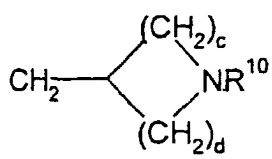

16

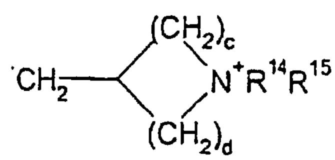

17

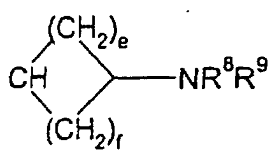

18

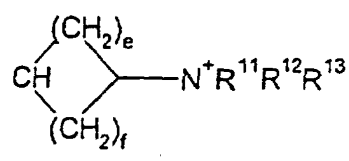

19

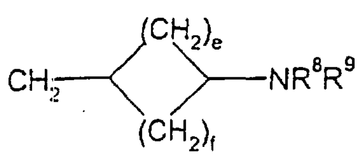

20

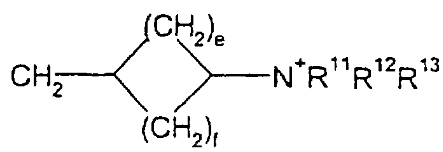

21

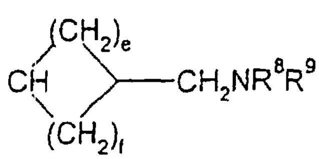

22

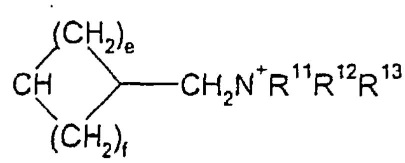

23

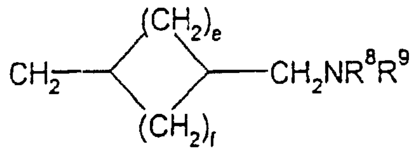

**24**

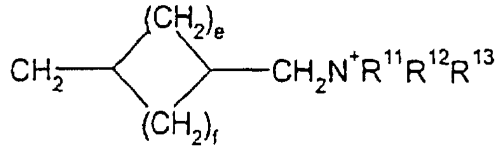

**25**

$R^7$ ausgewählt ist aus H, Alkyl, und irgendeiner Gruppe, wie für $R^6$ definiert;
$R^8$, $R^9$ und $R^{10}$ unabhängig ausgewählt sind aus H und Alkyl oder $R^8$ und $R^9$ zusammen $-(CH_2)_g-$ sein können;
$R^{11}$, $R^{12}$, $R^{13}$, $R^{14}$ und $R^{15}$ alle Alkyl sind oder $R^{11}$ und $R^{12}$ zusammen oder $R^{14}$ und $R^{15}$ zusammen $-(CH_2)_g-$ sein können;
$R^{16}$, $R^{17}$ und $R^{18}$ unabhängig ausgewählt sind aus H und Alkyl oder $R^{17}$ und $R^{18}$ zusammen $-(CH_2)_j-$ sein können;
$R^{19}$, $R^{20}$ , $R^{21}$ , $R^{22}$ und $R^{23}$ alle Alkyl sind oder $R^{19}$ und $R^{20}$ zusammen oder $R^{21}$ und $R^{22}$ zusammen $-(CH_2)_j-$ sein können;
$R^{24}$ und $R^{25}$ unabhängig ausgewählt sind aus Alkyl, Ar und $-(CH_2)_k$-Ar;
$R^{26}$ ausgewählt ist aus H, Alkyl, gegebenenfalls substituiertem Phenyl, Pyridyl Thienyl, OH, O-Alkyl, $NH_2$, NH-Alkyl, $N(Alkyl)_2$, $CO_2H$, $CO_2$-Alkyl, $CONH_2$, CONH-Alkyl, $CON(Alkyl)_2$, CN und $CF_3$;
$R^{27}$ ausgewählt ist aus OH, O-Alkyl, O-CO-Alkyl, $NH_2$, NH-Alkyl und $N(Alkyl)_2$,
Ar ausgewählt ist aus Thienyl und gegebenenfalls substituiertem Phenyl;
a 2 oder 3 ist, b 1, 2 oder 3 ist, c 1 oder 2 ist, d 1, 2 oder 3 ist, e 1 oder 2 ist, f 1, 2 oder 3 ist, g 4, 5 oder 6 ist, h 1, 2 oder 3 ist, i 1, 2, 3 oder 4 ist, j 4, 5 oder 6 ist, k 1, 2 oder 3 ist, l 1 oder 2 ist, m 1, 2 oder 3 ist und n 2, 3 oder 4 ist;

mit der Maßgabe, dass:

nicht mehr als eines von $A^8$, $A^9$ und $A^{10}$ NH, $N(CH_2)_mR^{26}$ oder S ist;
$A^7$ und $A^{11}$ nicht beide gleichzeitig N sind;
weder $A^7$ noch $A^{11}$ N ist, wenn eines von $A^8$, $A^9$ und $A^{10}$ NH, $N(CH_2)_mR^{26}$ oder S ist;
wenn $A^{10}$ -CH=CH- ist, dann $A^8$ N ist, $A^9$ CH ist und $A^7$ und $A^{11}$ beide C sind;
wenn $A^{10}$ nicht -CH=CH- ist, dann eines von $A^8$, $A^9$ und $A^{10}$ NH, $N(CH_2)_mR^{26}$ oder S ist, oder eines von $A^7$ und $A^{11}$ N ist;
nicht mehr als eines von $A^{14}$, $A^{15}$ und $A^{16}$ NH, $N-CH_3$ oder S ist;
$A^{12}$ und $A^{13}$ nicht beide gleichzeitig N sind;
wenn eines von $A^{14}$, $A^{15}$ und $A^{16}$ NH, $N-CH_3$ oder S ist, $A^{12}$ und $A^{13}$ beide C sind, und eines von $A^{14}$, $A^{15}$ und $A^{16}$ NH, $N-CH_3$ oder S ist, oder eines von $A^{12}$ und $A^{13}$ N ist.

**2.** Verbindung nach Anspruch 1, wobei mindestens eines von $R^1$, $R^2$ und $R^3$ H ist und eines nicht H ist.

**3.** Verbindung nach einem der vorangehenden Ansprüche, wobei eines von $R^1$, $R^2$ und $R^3$ ausgewählt ist aus Alkyl, F, Cl und Br und die anderen H sind.

**4.** Verbindung nach einem der vorangehenden Ansprüche, wobei $R^1$ Methyl oder Cl ist und $R^2$ und $R^3$ H sind.

**5.** Verbindung nach einem der vorangehenden Ansprüche, wobei eines von $R^4$ und $R^5$ H ist und das andere O-Alkyl ist.

**6.** Verbindung nach einem der vorangehenden Ansprüche, wobei eines von $R^4$ und $R^5$ H ist und das andere O-Methyl ist.

**7.** Verbindung nach einem der Ansprüche 1 bis 4, wobei $R^4$ und $R^5$ beide H sind.

**8.** Verbindung nach einem der Ansprüche 1 bis 4, wobei $R^4$ und $R^5$ beide F sind.

**9.** Verbindung nach einem der vorangehenden Ansprüche, wobei $X^1$ NH ist.

**10.** Verbindung nach einem der vorangehenden Ansprüche, wobei $G^1$ eine Gruppe der allgemeinen Formel 2 ist.

**11.** Verbindung nach einem der Ansprüche 1 bis 9, wobei $G^1$ eine Gruppe der allgemeinen Formel 3 ist.

**12.** Verbindung nach einem der vorangehenden Ansprüche, wobei $G^2$ eine Gruppe der allgemeinen Formel 4 ist.

**13.** Verbindung nach einem der Ansprüche 1 bis 11, wobei $G^2$ eine Gruppe der allgemeinen Formel 5, 6, 7, 8 oder 9 ist.

**14.** Verbindung nach einem der Ansprüche 1 bis 11, wobei $G^2$ eine Gruppe der allgemeinen Formel 5 ist, $A^1$ $CH_2$ ist und $A^2$ NH ist.

**15.** Verbindung nach einem der Ansprüche 1 bis 11, wobei $G^2$ eine Gruppe der allgemeinen Formel 5 ist, $A^1$ NH oder N-Alkyl ist und $A^2$ C(=O) ist.

**16.** Verbindung nach einem der Ansprüche 1 bis 11, wobei $G^2$ eine Gruppe der allgemeinen Formel 5, 6 oder 9 ist, $A^3$ S ist und $A^4$ und $A^5$ beide CH sind.

**17.** Verbindung nach einem der Ansprüche 1 bis 11, wobei $G^2$ eine Gruppe der allgemeinen Formel 5, 6 oder 9 ist $A^3$ -CH=CH- ist und $A^4$ und $A^5$ beide CH sind.

**18.** Verbindung nach Anspruch 1, wobei $R^1$ Methyl oder Cl ist, $R^2$ und $R^3$ beide H sind, $R^4$ H oder O-Methyl ist, $R^5$ H ist, $X^1$ NH ist und Y S ist.

**19.** Verbindung nach Anspruch 1, wobei $R^1$ Methyl oder Cl ist, $R^2$ und $R^3$ beide H sind, $R^4$ H oder O-Methyl ist, $R^5$ H ist, $X^1$ NH ist und Y O ist.

**20.** Verbindung nach Anspruch 1, wobei $R^2$ und $R^3$ beide H sind, $X^1$ NH ist und $G^1$

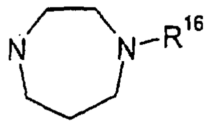

ist.

**21.** Verbindung nach Anspruch 1, wobei $R^1$ Methyl oder Cl ist, $R^2$ und $R^3$ beide H sind, $R^4$ H oder O-Methyl ist, $R^5$ H ist, $X^1$ NH ist und $G^2$

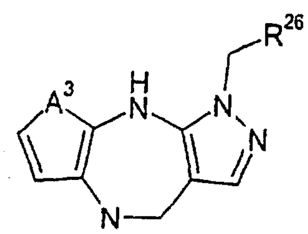

ist.

**22.** Verbindung nach Anspruch 1, wobei $R^1$ Methyl oder Cl ist, $R^2$ und $R^3$ beide H sind, $R^4$ H oder O-Methyl ist, $R^5$ H ist, $X^1$ NH ist, $G^1$

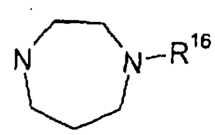

ist und $G^2$

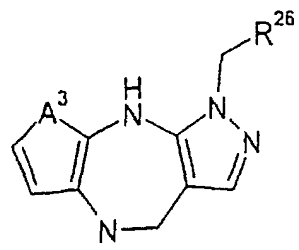

ist.

**23.** Verbindung nach Anspruch 1, ausgewählt aus

4-Methyl-1-(N-(2-methyl-4-(2,3,4,5-tetrahydro-1,5-benzodiazepin-4-on-1-yl-carbonyl)benzylcarbamoyl)-L-thioprolyl)perhydro-1,4-diazepin,

4-Methyl-1-(N-(2-methyl-4-(1-methyl-4,10-dihydropyrazolo[5,4-b][1,5]-benzodiazepin-5-ylcarbonyl)benzylcarbamoyl)-L-thioprolyl)perhydro-1,4-diazepin,

4,4-Dimethyl-1-(N-(2-methyl-4-(1-methyl-4,10-dihydropyrazolo[5,4-b][1,5]-benzodiazepin-5-yl-carbonyl)benzylcarbamoyl)-L-thioprolyl)perhydro-1,4-diazepinium-iodid,

4-Methyl-1-(N-(2-methyl-4-(5,6,7,8-tetrahydrothieno[3,2-b]azepin-4-yl-carbonyl)-benzylcarbamoyl)-L-thioprolyl)perhydro-1,4-diazepin,

4-Methyl-1-(N-(2-methyl-4-(5,6,7,8-tetrahydrothieno[3,2-b]azepin-4-yl-carbonyl)-benzyloxycarbonyl)-L-prolyl)perhydro-1,4-diazepin,

(4R)-$N^{\alpha}$-(2-Chlor-4-(5,6,7,8-tetrahydrothieno[3,2-b]azepin-4-yl-carbonyl)benzylcarbamoyl)-4-methoxy-L-prolin-N-methyl-N-(2-picolyl)amid und

1-((4R)-$N^{\alpha}$-(2-Chlor-4-(5,6,7,8-tetrahydrothieno[3,2-b]azepin-4-yl-carbonyl)benzylcarbamoyl)-4-methoxy-L-prolyl)-4-(1-pyrrolidinyl)piperidin.

**24.** Pharmazeutische Zusammensetzung, umfassend eine Verbindung nach einem der Ansprüche 1 bis 23 als Wirkstoff.

**25.** Pharmazeutische Zusammensetzung nach Anspruch 24, in Form einer Tablette oder Kapsel zur oralen Verabreichung.

**26.** Pharmazeutische Zusammensetzung nach Anspruch 24 oder 25 zur Behandlung von Erektionsstörungen bei Mann.

**27.** Verwendung einer Verbindung nach einem der Ansprüche 1 bis 23 zur Herstellung einer pharmazeutischen Zusammensetzung.

**28.** Verwendung nach Anspruch 27, wobei die pharmazeutische Zusammensetzung zur Behandlung von Erektionsstörungen bei Mann vorgesehen ist.

**29.** Verwendung einer Verbindung nach einem der Ansprüche 1 bis 23 zur Herstellung eines Arzneimittels zur Behandlung von Sexualstörungen beim Mann oder bei der Frau.

**30.** Ein oder mehrere optische Isomere einer Verbindung einer Verbindung nach einem der Ansprüche 1 bis 22.

**Revendications**

**1.** Composé selon la formule générale 1 ou un sel de celui-ci pharmaceutiquement acceptable

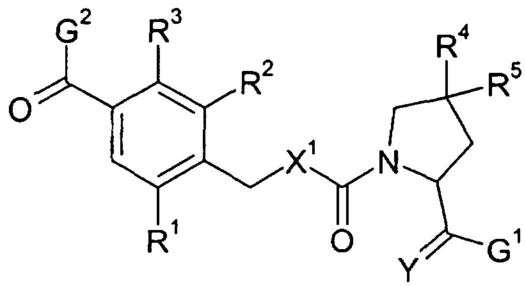

**1**

dans lequel:

$G^1$ est sélectionné dans un groupe selon la formule générale 2 et un groupe selon la formule générale 3;

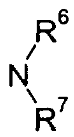

**2**

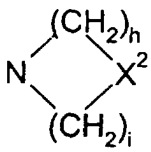

**3**

$G^2$ est sélectionné dans un groupe selon la formule générale 4, un groupe selon la formule générale 5, un groupe selon la formule générale 6, un groupe selon la formule générale 7, un groupe selon la formule générale 8 et un groupe selon la formule générale 9;

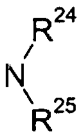

**4**

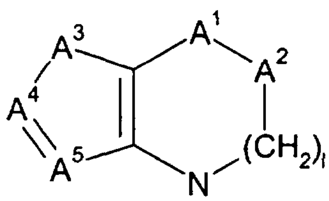

**5**

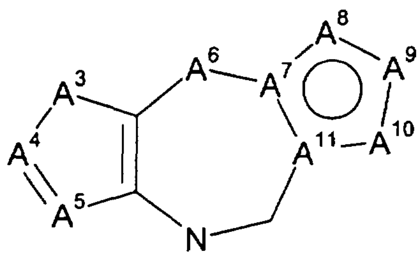

**6**

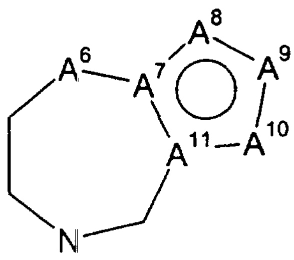
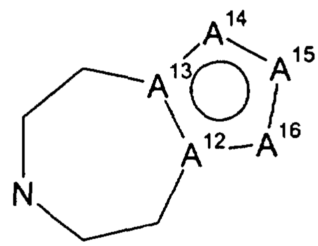
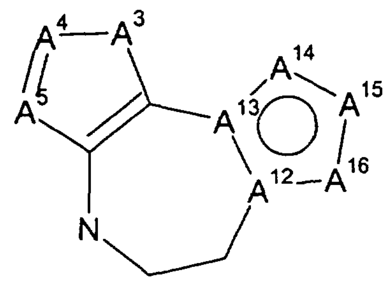

**7** **8** **9**

$A^1$ est sélectionné parmi $CH_2$, CH(OH), NH, N-alkyle, N-$(CH_2)_n$-$R^{27}$, O et S;

$A^2$ est sélectionné parmi $CH_2$, CH(OH), C(=O) et NH;

$A^3$ est sélectionné parmi S, NH, N-alkyle, -CH=CH- et -CH=N-;

$A^4$ et $A^5$ sont chacun sélectionnés parmi CH et N;

$A^6$ est sélectionné parmi $CH_2$, NH, N-alkyle et O;

$A^7$ et $A^{11}$ sont sélectionnés parmi C et N;

$A^8$ et $A^9$ sont sélectionnés parmi CH, N, NH, $N(CH_2)_mR^{26}$ et S;

$A^{10}$ est sélectionné parmi -CH=CH-, CH, N, NH, $N(CH_2)_mR^{26}$ et S;

$A^{12}$ et $A^{13}$ sont sélectionnés parmi N et C;

$A^{14}$, $A^{15}$ et $A^{16}$ sont sélectionnés parmi NH, N-$CH_3$, S, N et CH,

$X^1$ est sélectionné parmi O et NH;

$X^2$ est sélectionné parmi $NR^{16}$, CH-$NR^{17}R^{18}$, CH-$CH_2NR^{17}R^{18}$, $N^+R^{19}R^{21}$, CH-$N^+R^{21}R^{22}R^{23}$ et CH-$CH_2N^+R^{21}R^{22}R^{23}$;

Y est sélectionné parmi O et S;

$R^1$, $R^2$ et $R^3$ sont chacun sélectionnés parmi H, alkyle, O-alkyle, F, Cl et Br;

$R^4$ et $R^5$ sont soit chacun sélectionnés parmi H, O-alkyle, O-benzyl et F, soit $R^4$ et $R^5$ sont ensemble =O, -O$(CH_2)_a$O- ou -S$(CH_2)_a$S-;

$R^6$ est sélectionné dans un groupe selon la formule générale 10, un groupe selon la formule générale 11, un groupe selon la formule générale 12, un groupe selon la formule générale 13, un groupe selon la formule générale 14, un groupe selon la formule générale 15, un groupe selon la formule générale 16, un groupe selon la formule générale 17, un groupe selon la formule générale 18, un groupe selon la formule générale 19, un groupe selon la formule générale 20, un groupe selon la formule générale 21, un groupe selon la formule générale 22, un groupe selon la formule générale 23, un groupe selon la formule générale 24 et un groupe selon la formule générale 25;

$$-(CH_2)_b-NR^8R^9 \qquad \mathbf{10}$$

$$-(CH_2)_b-N^+R^{11}R^{12}R^{13} \qquad \mathbf{11}$$

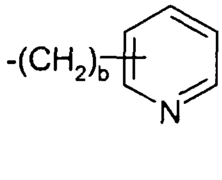

12

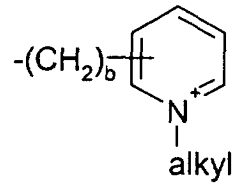

13

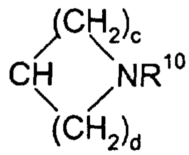

14

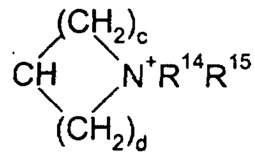

15

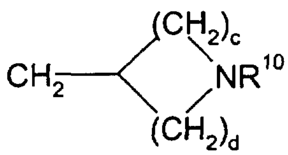

16

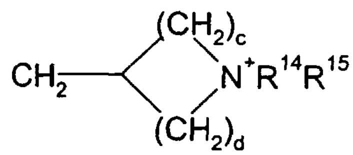

17

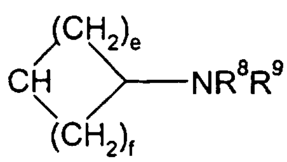

18

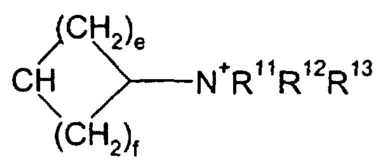

19

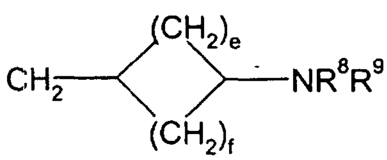

20

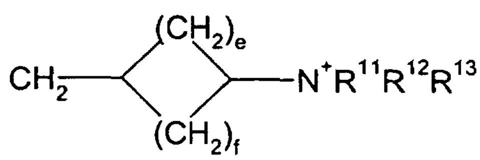

21

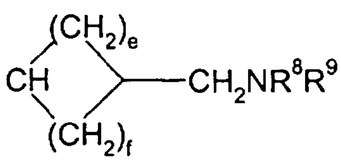

22

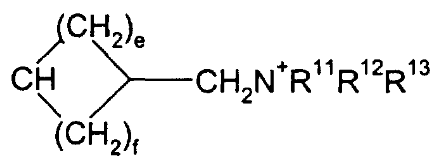

23

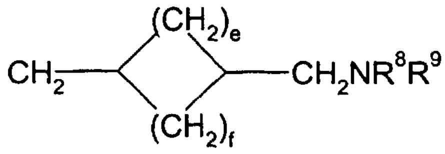

**24**

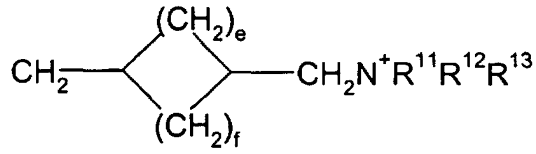

**25**

$R^7$ est sélectionné parmi H, alkyles et tout groupe tel que défini ci-dessus pour $R^6$;

$R^8$, $R^9$ et $R^{10}$ sont sélectionnés de façon indépendante parmi H et alkyle, ou $R^8$ et $R^9$ peuvent ensemble être $-(CH_2)_g-$;

$R^{11}$, $R^{12}$, $R^{13}$, $R^{14}$ et $R^{15}$ sont tous des alkyles, ou $R^{11}$ et $R^{12}$ ensemble ou $R^{14}$ et $R^{15}$ ensemble peuvent être $-(CH_2)_g-$ ;

$R^{16}$, $R^{17}$ et $R^{18}$ sont sélectionnés de façon indépendante parmi H et alkyle, ou $R^{17}$ et $R^{18}$ ensemble peuvent être $-(CH_2)_j-$;

$R^{19}$, $R^{20}$, $R^{21}$, $R^{22}$ et $R^{23}$ sont tous des alkyles, ou $R^{19}$ et $R^{20}$ ensemble ou $R^{21}$ et

$R^{22}$ ensemble peuvent être $-(CH_2)_j-$;

$R^{24}$ et $R^{25}$ sont sélectionnés de façon indépendante parmi alkyle, Ar et $-(CH_2)_k$-Ar;

$R^{26}$ est sélectionné parmi H, alkyle, phényle de substitution si désiré, pyridyl, thienyl, OH, O-alkyle, $NH_2$, NH-alkyle, N(alkyle)$_2$, $CO_2H$, $CO_2$-alkyle, $CONH_2$, CONH-alkyle, CON(alkyle)$_2$, CN et $CF_3$;

$R^{27}$ est sélectionné parmi OH, O-alkyle, O-CO-alkyle, $NH_2$, NH-alkyle et N(alkyle)$_2$;

Ar est sélectionné parmi thienyl et phényle de substitution si désiré;

a est 2 ou 3, b est 1, 2 ou 3; c est 1 ou 2, d est t, 2 ou 3; e est 1 ou 2; f est 1, 2 ou 3; g est 4, 5 ou 6; h est 1,2 ou 3; i est 1,2, 3 ou 4; j est 4, 5 ou 6; k est 1, 2 ou 3; l est 1 ou 2; m est 1, 2 ou 3; et n est 2, 3 ou 4;

à condition que:

pas plus d'un des $A^8$, $A^9$ et $A^{10}$ soit NH, $N(CH_2)_mR^{26}$ ou S;

$A^7$ et $A^{11}$ ne soient pas simultanément des N;

ni $A^7$ ni $A^{11}$ ne soient des N si l'un des $A^8$, $A^9$ et $A^{10}$ est NH, $N(CH_2)_mR^{26}$ ou S;

si $A^{10}$ est -CH=CH- alors $A^8$ soit N, $A^9$ soit CH et $A^7$ et $A^{11}$ soient tous deux C;

si $A^{10}$ n'est pas -CH=CH- alors un des $A^8$, $A^9$ et $A^{10}$ soit NH, $N(CH_2)_mR^{26}$ ou S ou un des $A^7$ et $A^{11}$ soit N;

pas plus d'un des $A^{14}$, $A^{15}$ et $A^{16}$ soit NH, $N-CH_3$ ou S;

$A^{12}$ et $A^{13}$ ne soient pas simultanément N;

si l'un d'entre $A^{14}$, $A^{15}$ et $A^{16}$ est NH, $N-CH_3$ ou S alors $A^{12}$ et $A^{13}$ soient tous deux C; et l'un d'entre $A^{14}$ $A^{15}$ et $A^{16}$ est NH, $N-CH_3$ ou S ou l'un d'entre $A^{12}$ et $A^{13}$ est N.

**2.** Composé selon la revendication 1 dans lequel au moins un de $R^1$, $R^2$ et $R^3$ est un H et un autre n'est pas un H.

**3.** Composé selon l'une quelconque des revendications précédentes dans lequel l'un d'entre $R^1$, $R^2$ et $R^3$ est sélec-

tionné parmi alkyle, F, Cl et Br et les autres sont des H.

4. Composé selon l'une quelconque des revendications précédentes dans lequel $R^1$ est du méthyle ou du Cl et $R^2$ et $R^3$ sont des H.

5. Composé selon l'une quelconque des revendications précédentes dans lequel un d'entre $R^4$ et $R^5$ est un H et l'autre un O-alkyle.

6. Composé selon l'une quelconque des revendications précédentes dans lequel un d'entre $R^4$ et $R^5$ est un H et l'autre est un O-méthyle.

7. Composé selon l'une quelconque des revendications 1 à 4 dans lequel $R^4$ et $R^5$ sont tous deux des H.

8. Composé selon l'une quelconque des revendications 1 à 4 dans lequel $R^4$ et $R^5$ sont tous deux des F.

9. Composé selon l'une quelconque des revendications précédentes dans lequel $X^1$ est NH.

10. Composé selon l'une quelconque des revendications précédentes dans lequel $G^1$ est un groupe selon la formule générale 2.

11. Composé selon l'une quelconque des revendications 1 à 9 dans lequel $G^1$ est un groupe selon la formule générale 3.

12. Composé selon l'une quelconque des revendications précédentes dans lequel $G^2$ est un groupe selon la formule générale 4.

13. Composé selon l'une quelconque des revendications 1 à 11 dans lequel $G^2$ est un groupe selon la formule générale 5, 6, 7, 8 ou 9.

14. Composé selon l'une quelconque des revendications 1 à 11 dans lequel $G^2$ est un groupe selon la formule générale 5, $A^1$ est du $CH_2$ et $A^2$ est du NH.

15. Composé selon l'une quelconque des revendications 1 à 11 dans lequel $G^2$ est un groupe selon la formule générale 5, $A^1$ est du NH ou N-alkyle et $A^2$ est du C(=O).

16. Composé selon l'une quelconque des revendications 1 à 11 dans lequel $G^2$ est un groupe selon la formule générale 5, 6 ou 9, $A^3$ est un S et $A^4$ et $A^5$ sont tous deux des CH.

17. Composé selon l'une quelconque des revendications 1 à 11 dans lequel $G^2$ est un groupe selon la formule générale 5, 6 ou 9, $A^3$ est -CH=CH- et $A^4$ et $A^5$ sont tous deux des CH.

18. Composé selon la revendication 1 dans lequel $R^1$ est du méthyle ou du Cl, $R^2$ et $R^3$ sont tous deux des H, $R^4$ est un H ou un O-méthyle, $R^5$ est un H, $X^1$ est un NH et Y est un S.

19. Composé selon la revendication 1 dans lequel $R^1$ est du méthyle ou du Cl, $R^2$ et $R^3$ sont tous deux du H, $R^4$ est du H ou du O-méthyle, $R^5$ est du H, $X^1$ est du NH et Y est du O.

20. Composé selon la revendication 1 dans lequel $R^2$ et $R^3$ sont tous deux du H, $X^1$ est du NH et $G^1$ est

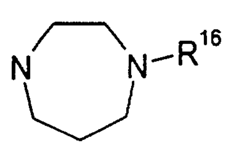

.

21. Composé selon la revendication 1 dans lequel $R^1$ est du méthyle ou du Cl, $R^2$ et $R^3$ sont tous deux du H, $R^4$ est

du H ou du O-méthyle, $R^5$ est du H, $X^1$ est du NH et $G^2$ est

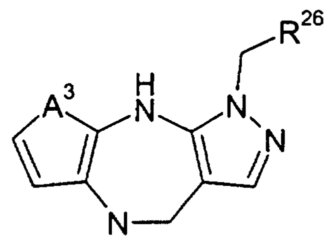

**22.** Composé selon la revendication 1 dans lequel $R^1$ est du méthyle ou du Cl, $R^2$ et R3 sont tous deux du H, $R^4$ est du H ou du O-méthyle, $R^5$ est du H, $X^1$ est du NH, $G^1$ est

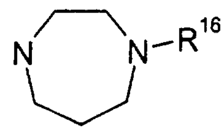

et $G^2$ est

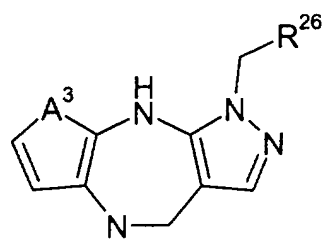

**23.** Composé selon la revendication 1 sélectionné parmi

4-methyl-1-(*N*-(2-methyl-4-(2,3,4,5-tetrahydro-1,5-benzodiazepin-4-on-1-yl-carbonyl)benzylcarbamoyl)-L-thioprolyl)perhydro-1,4-diazepine,

4-methyl-1-(*N*-(2-methyl-4-(1-methyl-4,10-dihydropyrazolo[5,4-*b*][1,5]-benzodiazepin-5-ylcarbonyl)benzyl-carbamoyl)-L-thioprolyl)perhydra-1,4-diazepine,

4,4-dimethyl-1-(*N*-(2-methyl-4-(1-methyl-4,10-dihydropyrazolo[5,4-*b*][1,5]-benzodiazepin-5-ylcarbonyl)benzylcarbamoyl)-L-thioprolyl)perhydro-1,4-diazepinium iodure,

4-methyl-1-(*N*-(2-methyl-4-(5,6,7,8-tetrahydrothieno[3,2-*b*]azepin-4-ylcarbonyl)-benzylcarbamoyl)-L-thioprolyl)perhydro-1,4-diazepine,

4-methyl-1-(*N*-(2-methyl-4-(5,6,7,8-tetrahydrothieno[3,2-*b*]azepin-4-ylcarbonyl)-benzyloxycarbonyl)-L-prolyl)perhydro-1,4-diazepine,

(4*R*)-$N^{\alpha}$-(2-chloro-4-(5,6,7,8-tetrahydrothieno[3,2-*b*]azepin-4- ylcarbonyl) benzyl-carbamoyl)-4-methoxy-L-proline-*N*-methyl-*N*-(2-picolyl)amide, et

1-((4*R*)-$N^{\alpha}$-(2-chloro-4-(5,6,7,8-tetrahydrothieno[3,2-*b*]azepin-4-ylcarbonyl) benzyl-carbamoyl)-4-methoxy-L-prolyl)-4-(1-pyrrolidinyl) pipéridine.

**24.** Composition pharmaceutique qui comprend un composé selon l'une quelconque des revendications 1 à 23 comme agent actif.

**25.** Composition pharmaceutique selon la revendication 24 qui est un cachet ou une gélule à administrer oralement.

**26.** Composition pharmaceutique selon la revendication 24 ou 25 utilisée pour le traitement de la dysérection chez l'homme.

**27.** Utilisation d'un composé selon l'une quelconque des revendications 1 à 23 dans la fabrication d'une composition pharmaceutique.

**28.** Utilisation selon la revendication 27 dans laquelle la composition pharmaceutique doit être utilisée dans le traitement de la dysérection chez l'homme.

**29.** Utilisation d'un composé selon l'une quelconque des revendications 1 à 23 dans la fabrication d'un médicament pour le traitement des problèmes sexuels chez l'homme ou chez la femme.

**30.** Un ou plusieurs isomères optiques d'un composé selon l'une quelconque des revendications 1 à 22.